# EUROPEAN PATENT SPECIFICATION

(11) **EP 2 742 148 B1**
(45) Date of publication and mention of the grant of the patent: **28.06.2017**
(21) Application number: 12741363.1
(22) Date of filing: 03.08.2012
(51) Int. Cl.: C12Q 1/68

(54) **COMPLEX SETS OF MIRNAS AS NON-INVASIVE BIOMARKERS FOR PSORIASIS**
KOMPLEXE SÄTZE AUS MIRNA ALS NICHTINVASIVE BIOMARKER FÜR PSORIASIS
ENSEMBLES COMPLEXES DE MI-ARN À TITRE DE BIOMARQUEURS NON INVASIFS POUR LE PSORIASIS

(30) Priority: 11.08.2011 EP 11177191
(43) Date of publication of application: 18.06.2014
(73) Proprietor: Hummingbird Diagnostics GmbH, 69120 Heidelberg (DE)
(72) Inventor: KELLER, Andreas, 66346 Püttlingen (DE); BEIER, Markus, 69469 Weinheim (DE); BOISGUERIN, Valesca, 55118 Mainz (DE); LEIDINGER, Petra, 66687 Wadern-Nunkirchen (DE); MEESE, Eckart, 66882 Hütschenhausen (DE)
(74) Representative: Geling, Andrea
(86) International application number: PCT/EP2012/065276
(87) International publication number: WO 2013/020926

(56) References cited:
- WO-A1-2011/026909
- WO-A1-2011/029903
- WO-A1-2011/029903
- WO-A1-2011/158191
- WO-A2-03/029459
- WO-A2-2008/142567
- WO-A2-2009/100342
- CHAUSSABEL DAMIEN ET AL: "Assessing the human immune system through blood transcriptomics", BMC BIOLOGY, BIOMED CENTRAL, LONDON, GB, GB, vol. 8, no. 1, 1 July 2010 (2010-07-01), page 84, XP021079430, ISSN: 1741-7007, DOI: 10.1186/1741-7007-8-84
- SUN B K ET AL: "Small RNAs in development and disease", JOURNAL OF THE AMERICAN ACADEMY OF DERMATOLOGY, MOSBY, INC, US, vol. 59, no. 5, 1 November 2008 (2008-11-01), pages 725-737, XP025518378, ISSN: 0190-9622, DOI: 10.1016/J.JAAD.2008.08.017 [retrieved on 2008-10-16]
- RIE OYAMA: "Circulating microRNA associated with TNF-alpha signaling pathway in patients with plaque psoriasis", JOURNAL OF DERMATOLOGICAL SCIENCE, vol. 61, 1 January 2010 (2010-01-01), pages 209-211, XP055039353,
- ZIBERT J R ET AL: "MicroRNAs and potential target interactions in psoriasis", JOURNAL OF DERMATOLOGICAL SCIENCE, ELSEVIER SCIENCE PUBLISHERS, SHANNON, IE, vol. 58, no. 3, 1 June 2010 (2010-06-01), pages 177-185, XP027068095, ISSN: 0923-1811, DOI: 10.1016/J.JDERMSCI.2010.03.004 [retrieved on 2010-03-17]
- CHAUSSABEL DAMIEN ET AL: "Assessing the human immune system through blood transcriptomics", BMC BIOLOGY, BIOMED CENTRAL, LONDON, GB, GB, vol. 8, no. 1, 1 July 2010 (2010-07-01), page 84, XP021079430, ISSN: 1741-7007, DOI: 10.1186/1741-7007-8-84
- SUN B K ET AL: "Small RNAs in development and disease", JOURNAL OF THE AMERICAN ACADEMY OF DERMATOLOGY, MOSBY, INC, US, vol. 59, no. 5, 1 November 2008 (2008-11-01), pages 725-737, XP025518378, ISSN: 0190-9622, DOI: 10.1016/J.JAAD.2008.08.017 [retrieved on 2008-10-16]

## Description

### TECHNICAL FIELD OF THE INVENTION

The present invention relates to a method for diagnosing and/or prognosing of psoriasis based on the determination of expression profiles of sets of miRNAs representative for psoriasis compared to a reference. Furthermore, the present invention relates to the use of sets of polynucleotides for detecting sets of miRNAs for diagnosing and/or prognosing of psoriasis in a blood cell sample from a subject. In addition, the present invention relates to the use of a kit for diagnosing and/or prognosing of psoriasis comprising means for determining expression profiles of sets of miRNAs representative for psoriasis and at least one reference. Further, the present invention relates to an *in vitro* use of a set of at least two miRNAs isolated from a blood cell sample from a subject for diagnosing and/or prognosing of psoriasis.

### BACKGROUND OF THE INVENTION

Today, biomarkers play a key role in early diagnosis, risk stratification, and therapeutic management of various diseases. While progess in biomarker research has accelerated over the last 5 years, the clinical translation of disease biomarkers as endpoints in disease management and as the foundation for diagnostic products still poses a challenge.

MicroRNAs (miRNAs) are a new class of biomarkers. They represent a group of small noncoding RNAs that regulate gene expression at the posttranslational level by degrading or blocking translation of messenger RNA (mRNA) targets. MiRNAs are important players when it comes to regulate cellular functions and in several diseases, including cancer.

So far, miRNAs have been extensively studied in tissue material. It has been found that miRNAs are expressed in a highly tissue-specific manner. Disease-specific expression of miRNAs have been reported in many human cancers employing primarily tissue material as the miRNA source. In this context miRNAs expression profiles were found to be useful in identifying the tissue of origin for cancers of unknown primary origin.

Since recently it is known that miRNAs are not only present in tissues but also in other body fluid samples, including human blood. Nevertheless, the mechanism why miRNAs are found in body fluids, especially in blood, or their function in these body fluids is not understood yet.

Various miRNA biomarkers found in tissue material have been proposed to be correlated with certain diseases, e.g. cancer. However, there is still a need for novel miRNAs as biomarkers for the detection and/or prediction of these and other types of diseases. Especially desirable are non-invasive biomarkers, that allow for quick, easy and cost-effective diagnosis/prognosis which cause only minimal stress for the patient eliminating the need for surgical intervention

Particularly, the potential role of miRNAs as non-invasive biomarkers for the diagnosis and/or prognosis of psoriasis has not been systematically evaluated yet. In addition, many of the miRNA biomarkers presently available for diagnosing and/or prognosing of diseases have shortcomings such as reduced sensitivity, not sufficient specificity or do not allow timely diagnosis or represent invasive biomarkers. Accordingly, there is still a need for novel and efficient miRNAs or sets of miRNAs as markers, effective methods and kits for the non-invasive diagnosis and/or prognosis of diseases such as psoriasis.

WO 2008/142567 A2, for example, relates to the finding that inflammatory skin disorders, such as psoriasis and atopic eczema, are characterized by changes in the expression of specific microRNA molecules (miRNAs). These miRNAs may, therefore, be useful as biomarkers for inflammatory skin disorders as well as therapeutic targets.

RIE OYAMA (Journal of Dermatological Science, Vol. 61, 2010, pages 209-211) describes circulating miRNAs associated with TNF-alpha signaling pathway in patients with plaque psoriasis.

WO 03/029459 A2 also refers to novel small expressed miRNA molecules associated with physiological regulator mechanisms, particularly in developmental control.

WO 2011/158191 A1 relates to the monitoring of the immune system using peripheral blood micro-RNA expression profile analysis and uses thereof.

WO 2011/029903 A1 describes a method for the preparation of miRNA and its therapeutic application.

The inventors of the present invention assessed for the first time the expression of miRNAs on a whole-genome level in subjects with psoriasis as non-invasive biomarkers from body fluids, preferably in blood. They surprisingly found that miRNAs are significantly dysregulated in blood of psoriasis subjects in comparison to healthy controls and thus, miRNAs are appropriated non-invasive biomarkers for diagnosing and/or prognosing of psoriasis. This finding is surprising, since there is nearly no overlap of the miRNA biomarkers found in blood and the miRNA biomarkers found in tissue material representing the origin of the disease. The inventors of the present invention surprisingly found miRNA biomarkers in body fluids, especially in blood, that have not been found to be correlated to psoriasis when tissues material was used for this kind of analysis. Therefore, the inventors of the invention identified for the first time miRNAs as non-invasive surrogate biomarkers for diagnosis and/or prognosis of psoriasis. The inventors of the present invention identified single miRNAs which predict psoriasis with high specificity, sensitivity and accuracy. The inventors of the present invention also pursued a multiple biomarker strategy, thus implementing sets of miRNA biomarkers for diagnosing and/or prognosing of psoriasis leading to added specificity, sensitivity, accuracy and predictive power, thereby circumventing some limitations of single biomarkers. In detail, by using a machine learning algorithms, they identified unique sets of miRNAs (miRNA signatures) that allow for non-invasive diagnosis of psoriasis with even higher power, indicating that sets of miRNAs (miRNA signatures) derived from a body fluid sample, such as blood from a subject (e.g. human) can be used as novel non-invasive biomarkers.

### SUMMARY OF THE INVENTION

In a first aspect, the invention provides a method for diagnosing and/or prognosing of psoriasis comprising the steps of:
(i) determining an expression profile of a set comprising at least two miRNAs representative for psoriasis in a blood sample from a subject, and
(ii) comparing said expression profile to a reference, wherein the comparison of said expression profile to said reference allows for the diagnosis and/or prognosis of psoriasis,
wherein the nucleotide sequences of the miRNAs comprised in the set are selected from the group consisting of SEQ ID NO: 1 to 111, a fragment thereof, and a sequence having at least 80% sequence identity thereto, and wherein the nucleotide sequence of a first of said at least two miRNAs is SEQ ID NO: 2, and wherein the blood sample is a blood cell sample.

In a second aspect, the invention provides the use of a set comprising polynucleotides for detecting a set comprising at least two miRNAs for diagnosing and/or prognosing of psoriasis in a blood sample from a subject,
wherein the nucleotide sequences of the miRNAs comprised in the set are selected from the group consisting of SEQ ID NO: 1 to 111, and wherein the nucleotide sequence of a first of said at least two miRNAs is SEQ ID NO: 2, and wherein the blood sample is a blood cell sample.

In a third aspect, the invention provides the use of a kit for diagnosing and/or prognosing of psoriasis comprising
(i) means for determining an expression profile of a set comprising at least two miRNAs representative for psoriasis in a blood sample from a subject, and
(ii) at least one reference,
wherein the nucleotide sequences of said miRNAs are selected from the group consisting of SEQ ID NO: 1 to 111, a fragment thereof, and a sequence having at least 80% sequence identity thereto, and
wherein the nucleotide sequence of a first of said at least two miRNAs is SEQ ID NO: 2, and
wherein the blood sample is a blood cell sample, and
wherein the reference is generated from reference expression profiles of at least two control subjects of at least two clinical conditions from which at least one is psoriasis determined in the same type of blood sample as the subject to be diagnosed and/or prognosed.

In a fourth aspect, the invention provides the *in vitro* use of a set of at least two miRNAs isolated from a blood sample from a subject for diagnosing and/or prognosing of psoriasis, wherein the miRNAs are selected from the group consisting of SEQ ID NO: 1 to 111, and wherein the nucleotide sequence of a first of said at least two miRNA's is SEQ ID NO: 2, and wherein the blood sample is a blood cell sample.

This summary of the invention does not necessarily describe all features of the invention.

### DETAILED DESCRIPTION OF THE INVENTION

Before the present invention is described in detail below, it is to be understood that this invention is not limited to the particular methodology, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art.

In the following, the elements of the present invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine the explicitly described embodiments with any number of the disclosed and/or preferred elements. Furthermore, any permutations and combinations of all described elements in this application should be considered disclosed by the description of the present application unless the context indicates otherwise.

Preferably, the terms used herein are defined as described in "A multilingual glossary of biotechnological terms: (IUPAC Recommendations)", H.G.W. Leuenberger, B. Nagel, and H. Kölbl, Eds., Helvetica Chimica Acta, CH-4010 Basel, Switzerland, (1995).

To practice the present invention, unless otherwise indicated, conventional methods of chemistry, biochemistry, and recombinant DNA techniques are employed which are explained in the literature in the field (cf., e.g., Molecular Cloning: A Laboratory Manual, 2nd Edition, J. Sambrook et al. eds., Cold Spring Harbor Laboratory Press, Cold Spring Harbor 1989).

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integers or steps.

As used in this specification and in the appended claims, the singular forms "a", "an", and "the" include plural referents, unless the content clearly dictates otherwise. For example, the term "a test compound" also includes "test compounds".

The terms "microRNA" or "miRNA" refer to single-stranded RNA molecules of at least 10 nucleotides and of not more than 35 nucleotides covalently linked together. Preferably, the polynucleotides of the present invention are molecules of 10 to 33 nucleotides or 15 to 30 nucleotides in length, more preferably of 17 to 27 nucleotides or 18 to 26 nucleotides in length, i.e. 10, 11, 12, 13, 14, 15, 16, 17, 18, 19, 20, 21, 22, 23, 24, 25, 26, 27, 28, 29, 30, 31, 32, 33, 34, or 35 nucleotides in length, not including optionally labels and/or elongated sequences (e.g. biotin stretches). The miRNAs regulate gene expression and are encoded by genes from whose DNA they are transcribed but miRNAs are not translated into protein (i.e. miRNAs are non-coding RNAs). The genes encoding miRNAs are longer than the processed mature miRNA molecules. The miRNAs are first transcribed as primary transcripts or pri-miRNAs with a cap and poly-A tail and processed to short, 70 nucleotide stem-loop structures known as pre-miRNAs in the cell nucleus. This processing is performed in animals by a protein complex known as the Microprocessor complex consisting of the nuclease Drosha and the double-stranded RNA binding protein Pasha. These pre-miRNAs are then processed to mature miRNAs in the cytoplasm by interaction with the endonuclease Dicer, which also initiates the formation of the RNA-induced silencing complex (RISC). When Dicer cleaves the pre-miRNA stem-loop, two complementary short RNA molecules are formed, but only one is integrated into the RISC. This strand is known as the guide strand and is selected by the argonaute protein, the catalytically active RNase in the RISC, on the basis of the stability of the 5' end. The remaining strand, known as the miRNA*, anti-guide (anti-strand), or passenger strand, is degraded as a RISC substrate. Therefore, the miRNA*s are derived from the same hairpin structure like the "normal" miRNAs. So if the "normal" miRNA is then later called the "mature miRNA" or "guide strand", the miRNA* is the "anti-guide strand" or "passenger strand".

The terms "microRNA*" or "miRNA*" refer to single-stranded RNA molecules of at least 10 nucleotides and of not more than 35 nucleotides covalently linked together. Preferably, the polynucleotides of the present invention are molecules of 10 to 33 nucleotides or 15 to 30 nucleotides in length, more preferably of 17 to 27 nucleotides or 18 to 26 nucleotides in length, i.e. 10, 11, 12, 13, 14, 15, 16, 17, 18, 19, 20, 21, 22, 23, 24, 25, 26, 27, 28, 29, 30, 31, 32, 33, 34, or 35 nucleotides in length, not including optionally labels and/or elongated sequences (e.g. biotin stretches). The "miRNA*s", also known as the "anti-guide strands" or "passenger strands", are mostly complementary to the "mature miRNAs" or "guide strands", but have usually single-stranded overhangs on each end. There are usually one or more mispairs and there are sometimes extra or missing bases causing single-stranded "bubbles". The miRNA*s are likely to act in a regulatory fashion as the miRNAs (see also above). In the context of the present invention, the terms "miRNA" and "miRNA*" are interchangeable used. Described herein are (target) miRNAs which are dysregulated in biological samples such as blood or tissue of psoriasis patients in comparison to healthy controls. Said (target) miRNAs are preferably selected from the group consisting of SEQ ID NO: 1 to 111 or SEQ ID NO: 1 to 283.

The term "miRBase" refers to a well established repository of validated miRNAs. The miRBase (www.mirbase.org) is a searchable database of published miRNA sequences and annotation. Each entry in the miRBase Sequence database represents a predicted hairpin portion of a miRNA transcript (termed mir in the database), with information on the location and sequence of the mature miRNA sequence (termed miR). Both hairpin and mature sequences are available for searching and browsing, and entries can also be retrieved by name, keyword, references and annotation. All sequence and annotation data are also available for download.

As used herein, the term "nucleotides" refers to structural components, or building blocks, of DNA and RNA. Nucleotides consist of a base (one of four chemicals: adenine, thymine, guanine, and cytosine) plus a molecule of sugar and one of phosphoric acid. The term "nucleosides" refers to glycosylamine consisting of a nucleobase (often referred to simply base) bound to a ribose or deoxyribose sugar. Examples of nucleosides include cytidine, uridine, adenosine, guanosine, thymidine and inosine. Nucleosides can be phosphorylated by specific kinases in the cell on the sugar's primary alcohol group (-CH2-OH), producing nucleotides, which are the molecular building blocks of DNA and RNA.

The term "polynucleotide", as used herein, means a molecule of at least 10 nucleotides and of not more than 35 nucleotides covalently linked together. Preferably, the polynucleotides used herein are molecules of 10 to 33 nucleotides or 15 to 30 nucleotides in length, more preferably of 17 to 27 nucleotides or 18 to 26 nucleotides in length, i.e. 10, 11, 12, 13, 14, 15, 16, 17, 18, 19, 20, 21, 22, 23, 24, 25, 26, 27, 28, 29, 30, 31, 32, 33, 34, or 35 nucleotides in length, not including optionally spacer elements and/or elongation elements described below. The depiction of a single strand of a polynucleotide also defines the sequence of the complementary strand. Polynucleotides may be single stranded or double stranded, or may contain portions of both double stranded and single stranded sequences. The term "polynucleotide" means a polymer of deoxyribonucleotide or ribonucleotide bases and includes DNA and RNA molecules, both sense and anti-sense strands. In detail, the polynucleotide may be DNA, both cDNA and genomic DNA, RNA, cRNA or a hybrid, where the polynucleotide sequence may contain combinations of deoxyribonucleotide or ribonucleotide bases, and combinations of bases including uracil, adenine, thymine, cytosine, guanine, inosine, xanthine, hypoxanthine, isocytosine and isoguanine. Polynucleotides may be obtained by chemical synthesis methods or by recombinant methods.

In the context of the present invention, a polynucleotide as a single polynucleotide strand provides a probe (e.g. miRNA capture probe) that is capable of binding to, hybridizing with, or detecting a target of complementary sequence, such as a nucleotide sequence of a miRNA or miRNA*, through one or more types of chemical bonds, usually through complementary base pairing, usually through hydrogen bond formation. Polynucleotides in their function as probes may bind target sequences, such as nucleotide sequences of miRNAs or miRNAs*, lacking complete complementarity with the polynucleotide sequences depending upon the stringency of the hybridization condition. There may be any number of base pair mismatches which will interfere with hybridization between the target sequence, such as a nucleotide sequence of a miRNA or miRNA*, and the single stranded polynucleotide described herein. However, if the number of mutations is so great that no hybridization can occur under even the least stringent hybridization conditions, the sequences are no complementary sequences. Polynucleotides in form of single polynucleotide strands as probes for binding to, hybridizing with or detecting complementary sequences of (target) miRNAs for diagnosing and/or prognosing of psoriasis are described herein. Said (target) miRNAs are preferably selected from the group consisting of SEQ ID NO: 1 to 111 or SEQ ID NO: 1 to 283.

Because of the conservation of miRNAs among species, for example between humans and other mammals, e.g. animals such as mice, monkey or rat, the polynucleotide(s) used herein may not only be suitable for detecting a miRNA(s) of a specific species, e.g. a human miRNA, but may also be suitable for detecting the respective miRNA orthologue(s) in another species, e.g. in another mammal, e.g. animal such as mouse or rat.

The term "antisense", as used herein, refers to nucleotide sequences which are complementary to a specific DNA or RNA sequence. The term "antisense strand" is used in reference to a nucleic acid strand that is complementary to the "sense" strand.

The term "label", as used herein, means a composition detectable by spectroscopic, photochemical, biochemical, immunochemical, chemical, or other physical means. For example, useful labels include 32P, fluorescent dyes, electron-dense reagents, enzymes (e.g., as commonly used in an ELISA), biotin, digoxigenin, or haptens and other entities which can be made detectable. A label may be incorporated into nucleic acids at any position, e.g. at the 3' or 5' end or internally. The polynucleotide for detecting a miRNA (polynucleotide probe) and/or the miRNA itself may be labeled. For detection purposes, the miRNA(s) or miRNA*(s) may be employed unlabeled, directly labeled, or indirectly labeled, such as with biotin to which a streptavidin complex may later bind.

The term "stringent hybridization conditions", as used herein, means conditions under which a first nucleotide sequence (e.g. polynucleotide in its function as a probe for detecting a miRNA or miRNA*) will hybridize to a second nucleotide sequence (e.g. target sequence such as nucleotide sequence of a miRNA or miRNA*), such as in a complex mixture of nucleotide sequences. Stringent conditions are sequence-dependent and will be different in different circumstances. Stringent conditions may be selected to be about 5 to 10°C lower than the thermal melting point (Tm) for the specific sequence at a defined ionic strength, pH. The Tm may be the temperature (under defined ionic strength, pH, and nucleic acid concentration) at which 50% of the probes complementary to the target hybridize to the target sequence at equilibrium (as the target sequences are present in excess, at Tm, 50% of the probes are occupied at equilibrium). Stringent conditions may be those in which the salt concentration is less than about 1.0 M sodium ion, such as about 0.01 to 1.0 M sodium ion concentration (or other salts) at pH 7.0 to 8.3 and the temperature is at least about 20°C for short probes (e.g. about 10-35 nucleotides) and up to 60°C for long probes (e.g. greater than about 50 nucleotides). Stringent conditions may also be achieved with the addition of destabilizing agents such as formamide. For selective or specific hybridization, a positive signal may be at least 2 to 10 times background hybridization. Exemplary stringent hybridization conditions include the following: 50% formamide, 5x SSC, and 1% SDS, incubating at 42°C, or, 5x SSC, 1% SDS, incubating at 65°C, with wash in 0.2x SSC, and 0.1% SDS at 65°C; or 6x SSPE, 10 % formamide, 0.01 %, Tween 20, 0.1 x TE buffer, 0.5 mg/ml BSA, 0.1 mg/ml herring sperm DNA, incubating at 42°C with wash in 05x SSPE and 6x SSPE at 45°C.

The term "sensitivity", as used herein, means a statistical measure of how well a binary classification test correctly identifies a condition, for example how frequently it correctly classifies a heart and cardiovascular system disease into the correct type out of two or more possible types (e.g. heart and cardiovascular system disease type and healthy type). The sensitivity for class A is the proportion of cases that are determined to belong to class "A" by the test out of the cases that are in class "A". A theoretical, optimal prediction can achieve 100% sensitivity (i.e. predict all patients from the sick group as sick).

The term "specificity", as used herein, means a statistical measure of how well a binary classification test correctly identifies a condition, for example how frequently it correctly classifies a heart and cardiovascular system disease into the correct type out of two or more possible types.

The specificity for class A is the proportion of cases that are determined to belong to class "not A" by the test out of the cases that are in class "not A". A theoretical, optimal prediction can achieve 100% specificity (i.e. not predict anyone from the healthy group as sick).

The term "accuracy", as used herein, means a statistical measure for the correctness of classification or identification of sample types. The accuracy is the proportion of true results (both true positives and true negatives).

The term "Receiver operating characteristic (ROC) curves" means a graphical measure of sensitivity (y-axis) vs. 1 - specificity (x-axis) for a clinical test. An important measure of the accuracy of the clinical test is the area under the ROC curve value (AUC value). If this area is equal to 1.0 then this test is 100% accurate because both the sensitivity and specificity are 1.0 so there are no false positives and no false negatives. On the other hand a test that cannot discriminate that is the diagonal line from 0,0 to 1,1. The ROC area for this line is 0.5. ROC curve areas (AUC-values) are typically between 0.5 and 1.0, but also ROC values below 0.5 can - according to information theory- be as good, if the result is interpreted inversely. Therefore, according to the present invention an AUC-value close to 1 (e.g. 0.95) represents the same good measure for a clinical test as an AUC-value close to 0 (e.g. 0.05).

The term "biological sample", as used herein, refers to any biological sample containing miRNA(s). Said biological sample may be a biological fluid, tissue, cell(s) or mixtures thereof. For example, biological samples are body fluids, tissue (e.g. section or explant) samples, cell culture samples, cell colony samples, single cell samples, collection of single cell samples, blood samples (e.g. whole blood or a blood fraction such as serum or plasma), urine samples, or samples from other peripheral sources. Said biological samples may be mixed or pooled, e.g. a biological sample may be a mixture of blood and urine samples. A "biological sample" may be provided by removing cell(s), cell colonies, an explant, or a section from a subject suspected to be affected by psoriasis, but may also be provided by using a previously isolated sample. For example, a tissue sample may be removed from a subject suspected to be affected by psoriasiss by conventional biopsy techniques or a blood sample may be taken from a subject suspected to be affected by psoriasis by conventional blood collection techniques. The biological sample, e.g. tissue or blood sample, may be obtained from a subject suspected to be affected by psoriasis prior to initiation of the therapeutic treatment, during the therapeutic treatment and/or after the therapeutic treatment.

The term "body fluid sample", as used herein, refers to liquids originating from the body of a subject. Said body fluid samples include, but are not limited to, blood, urine, sputum, breast milk, cerebrospinal fluid, amniotic fluid, bronchial lavage, colostrum, seminal fluid, cerumen (earwax), endolymph, perilymph, gastric juice, mucus, peritoneal fluid, pleural fluid, saliva, sebum (skin oil), semen, sweat, tears, vaginal secretion, vomit including components or fractions thereof Said body fluid samples may be mixed or pooled, e.g. a body fluid sample may be a mixture of blood and urine samples or blood and tissue material. A "body fluid sample" may be provided by removing a body liquid from a subject, but may also be provided by using previously isolated sample material.

Preferably, the body fluid sample from a subject (e.g. human or animal) has a volume of between 0.1 and 20 ml, more preferably of between 0.5 and 10 ml, more preferably between 1 and 8 ml and most preferably between 2 and 5 ml, i.e. 0.1, 0.2, 0.3, 0.4, 0.5, 0.6, 0.7, 0.8, 0.9, 1, 2, 2.5, 3, 4, 5, 6, 7, 8, 9, 10, 11, 12, 13, 14, 15, 16, 17, 18, 19, or 20 ml.

Said "body fluid sample" allows for a non-invasive diagnosis/and or prognosis of a subject.

The term "blood sample", as used herein, refers to a blood sample originating from a subject. The "blood sample" may be derived by removing blood from a subject by conventional blood collecting techniques, but may also be provided by using previously isolated and/or stored blood samples. For example a blood sample may be whole blood, plasma, serum, blood cells, PBMC (peripheral blood mononuclear cells), blood cellular fractions including or comprising red blood cells (erythrocytes), white blood cells (leukocytes), platelets (thrombocytes), or blood collected in blood collection tubes (e.g. EDTA-, heparin-, citrate-, PAXgene- , Tempus-tubes) including components or fractions thereof. For example, a blood sample may be taken from a subject suspected to be affected or to be suspected to be affected by psoriasis, prior to initiation of a therapeutic treatment, during the therapeutic treatment and/or after the therapeutic treatment. Preferably, the blood sample from a subject (e.g. human or animal) has a volume of between 0.1 and 20 ml, more preferably of between 0.5 and 10 ml, more preferably between 1 and 8 ml and most preferably between 2 and 5 ml, i.e. 0.1, 0.2, 0.3, 0.4, 0.5, 0.6, 0.7, 0.8, 0.9, 1, 2, 2.5, 3, 4, 5, 6, 7, 8, 9, 10, 11, 12, 13, 14, 15, 16, 17, 18, 19, or 20 ml.

Said "body fluid sample" or "blood sample" allows for a non-invasive diagnosis/and or prognosis of a subject.

In the context of the present invention, the blood sample is a blood cell sample.

Preferably, when the blood sample is collected from the subject the RNA-fraction, especially the the miRNA fraction, is guarded against degradation. For this purpose special collection tubes (e.g. PAXgene RNA tubes from Preanalytix, Tempus Blood RNA tubes from Applied Biosystems) or additives (e.g. RNAlater from Ambion, RNAsin from Promega) that stabilize the RNA fraction and/or the miRNA fraction are employed.

The biological sample, preferably the body fluid sample may be from a subject (e.g. human or mammal) that has been therapeutically treated or that has not been therapeutically treated. In one embodiment, the therapeutical treatment is monitored on the basis of the detection of the miRNA or set of miRNAs by the polynucleotide or set of polynucleotides described herein. It is also preferred that total RNA or a subtraction thereof, isolated (e.g. extracted) from a biological sample of a subject (e.g. human or animal), is used for detecting the miRNA or set of miRNAs by the polynucleotide or set of polynucleotides or primer pairs described herein.

The term "non-invasive", as used in the context of the present invention, refers to methods for obtaining a biological sample, particularly a body fluid sample, without the need for an invasive surgical intervention or invasive medical procedure. In the context of the present invention, a blood drawn represents a non-invasive procedure, therefore a blood-based test (utilizing blood or fractions thereof) is a non-invasive test. Other body fluid samples for non-invasive tests are e.g. urine, sputum, tears, mothers mild, cerumen, sweat, saliva, vaginal secretion, vomit, etc..

The term "minimal invasive", as used in the context of the present invention, refers to methods for obtaining a biological sample, particularly a body fluid sample, with a minimal need for an invasive surgical intervention or invasive medical procedure.

The term "biomarker", as used in the context of the present invention, represents a characteristic that can be objectively measured and evaluated as an indicator of normal and disease processes or pharmacological responses. A biomarker is a parameter that can be used to measure the onset or the progress of disease or the effects of treatment. The parameter can be chemical, physical or biological.

The term "surrogate biomarker", as used in the context of the present invention, represents biomarker intended to substitute for a clinical endpoint. It is a measure of a clinical condition or a measure of effect of a certain treatment that may correlate with the real clinical condition (e.g. healthy, diseased) but doesn't necessarily have a guaranteed relationship. An ideal surrogate biomarker is a laboratory substitute for a clinically meaningful result, and should lie directly in the causal pathway linking disease to outcome. Surrogate biomarkers are used when the primary endpoint is undesired (e.g. death). A commonly used example is cholesterol : while elevated cholesterol levels increase the likelihood for heart disease, the relationship is not linear - many people with normal cholesterol develop heart disease, and many with high cholesterol do not. "Death from heart disease" is the endpoint of interest, but "cholesterol" is the surrogate biomarker.

The term "diagnosis" as used in the context of the present invention refers to the process of determining a possible disease or disorder and therefore is a process attempting to define the (clinical) condition of a subject. The determination of the expression level of a set of miRNAs according to the present invention correlates with the (clinical) condition of a subject. Preferably, the diagnosis comprises (i) determining the occurrence/presence of psoriasis, (ii) monitoring the course of psoriasis, (iii) staging of psoriasis, (iv) measuring the response of a patient with psoriasis to therapeutic intervention, and/or (v) segmentation of a subject suffering from psoriasis.

The term "prognosis" as used in the context of the present invention refers to describing the likelihood of the outcome or course of a disease or a disorder. Preferably, the prognosis comprises (i) identifying of a subject who has a risk to develop psoriasis, (ii) predicting/estimating the occurrence, preferably the severity of occurrence of psoriasis, and/or(iii) predicting the response of a subject with psoriasis to therapeutic intervention.

The term "(clinical) condition" (biological state or health state), as used herein, means a status of a subject that can be described by physical, mental or social criteria. It includes so-called "healthy" and "diseased" conditions. For the definition of "healthy" and "diseased" conditions it is referred to the international classification of diseases (ICD) of the WHO (http://www.int/classifications/icd/en/index.html). When one condition is compared according to a preferred embodiment of the method of the present invention, it is understood that said condition is psoriasis or a specific form of psoriasis. When two or more conditions are compared according to another preferred embodiment of the method of the present invention, it is understood that this is possible for all conditions that can be defined and is not limited to a comparison of a diseased versus healthy comparison and extends to multiway comparison, under the proviso that at least one condition is psoriasiss, preferably a specific form of psoriasis.

The term "miRNA expression profile" as used in the context of the present invention, represents the determination of the miRNA expression level or a measure that correlates with the miRNA expression level in a biological sample. The miRNA expression profile may be generated by any convenient means, e.g. nucleic acid hybridization (e.g. to a microarray, bead-based methods), nucleic acid amplification (PCR, RT-PCR, qRT-PCR, high-throughput RT-PCR), ELISA for quantitation, next generation sequencing (e.g. ABI SOLID, Illumina Genome Analyzer, Roche/454 GS FLX), flow cytometry (e.g. LUMINEX, Firefly Bioworks) and the like, that allow the analysis of differential miRNA expression levels between samples of a subject (e.g. diseased) and a control subject (e.g. healthy, reference sample). The sample material measure by the aforementioned means may be total RNA, labeled total RNA, amplified total RNA, cDNA, labeled cDNA, amplified cDNA, miRNA, labeled miRNA, amplified miRNA or any derivatives that may be generated from the aforementioned RNA/DNA species. By determining the miRNA expression profile, each miRNA is represented by a numerical value. The higher the value of an individual miRNA, the higher is the expression level of said miRNA, or the lower the value of an individual miRNA, the lower is the expression level of said miRNA.

The "miRNA expression profile", as used herein, represents the expression level/expression data of a single miRNA or a collection of expression levels of at least two miRNAs, preferably of least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35 or more, or up to all known miRNAs.

The term "differential expression" of miRNAs as used herein, means qualitative and/or quantitative differences in the temporal and/or local miRNA expression patterns, e.g. within and/or among biological samples, body fluid samples, cells, or within blood. Thus, a differentially expressed miRNA may qualitatively have its expression altered, including an activation or inactivation in, for example, blood from a diseases subject versus blood from a healthy subject. The difference in miRNA expression may also be quantitative, e.g. in that expression is modulated, i.e. either up-regulated, resulting in an increased amount of miRNA, or down-regulated, resulting in a decreased amount of miRNA. The degree to which miRNA expression differs need only be large enough to be quantified via standard expression characterization techniques, e.g. by quantitative hybridization (e.g. to a microarray, to beads), amplification (PCR, RT-PCR, qRT-PCR, high-throughput RT-PCR), ELISA for quantitation, next generation sequencing (e.g. ABI SOLID, Illumina Genome Analyzer, Roche 454 GS FL), flow cytometry (e.g. LUMINEX, Firefly Bioworks) and the like.

Nucleic acid hybridization may be performed using a microarray/biochip or *in situ* hybridization. *In situ* hybridization is preferred for the analysis of a single miRNA or a set comprising a low number of miRNAs (e.g. a set of at least 2 to 50 miRNAs such as a set of 2, 5, 10, 20, 30, or 40 miRNAs). The microarray/biochip, however, allows the analysis of a single miRNA as well as a complex set of miRNAs (e.g. a all known miRNAs or subsets therof).

For nucleic acid hybridization, for example, the polynucleotides (probes) with complementarity to the corresponding miRNAs to be detected are attached to a solid phase to generate a microarray/biochip (e.g. 111 polynucleotides (probes) which are complementary to the 111 miRNAs having SEQ ID NO: 1 to 111 or 283 polynucleotides (probes) which are complementary to the 283 miRNAs having SEQ ID NO: 1 to 283. Said microarray/biochip is then incubated with a biological sample containing miRNAs, isolated (e.g. extracted) from the body fluid sample such as blood sample from a subject such as a human or an animal, which may be labelled, e.g. fluorescently labelled, or unlabelled. Quantification of the expression level of the miRNAs may then be carried out e.g. by direct read out of a label or by additional manipulations, e.g. by use of a polymerase reaction (e.g. template directed primer extension, MPEA-Assay, RAKE-assay) or a ligation reaction to incorporate or add labels to the captured miRNAs.

Alternatively, the polynucleotides which are at least partially complementary (e.g.a set of chimeric polynucleotides with each a first stretch being complementary to a set of miRNA sequences and a second stretch complementary to capture probes bound to a solid surface (e.g. beads, Luminex or Firefly Bioworks beads)) to miRNAs having SEQ ID NO: 1 to 111 or SEQ ID NO: 1 to 283. are contacted with the biological sample containing miRNAs (e.g a body fluid sample, preferably a blood sample) in solution to hybridize. Afterwards, the hybridized duplexes are pulled down to the surface (e.g a plurality of beads) and successfully captured miRNAs are quantitatively determined (e.g. FlexmiR-assay, FlexmiR v2 detection assays from Luminex or Firefly Bioworks Assay).

Nucleic acid amplification may be performed using real time polymerase chain reaction (RT-PCR) such as real time quantitative polymerase chain reaction (RT qPCR). The standard real time polymerase chain reaction (RT-PCR) is preferred for the analysis of a single miRNA or a set comprising a low number of miRNAs (e.g. a set of at least 2 to 50 miRNAs such as a set of 2, 5, 10, 20, 30, or 40 miRNAs), whereas high-throughput RT-PCR technologies (e.g. OpenArray from Applied Biosystems, SmartPCR from Wafergen, Biomark System from Fluidigm) are also able to measure large sets (e.g a set of 10, 20, 30, 50, 80, 100, 200 or more) to all known miRNAs in a high parallel fashion. RT-PCR is particularly suitable for detecting low abandoned miRNAs.

The aforesaid real time polymerase chain reaction (RT-PCR) may include the following steps: (i) extracting the total RNA from a biological sample or body fluid sample such as a blood sample (e.g. whole blood, serum, or plasma) of a subjects such as human or animal, and obtaining cDNA samples by RNA reverse transcription (RT) reaction using universal or miRNA-specific primers; or collecting a body fluid sample such as urine or blood sample (e.g. whole blood, serum, or plasma) of a patient such as human or animal, and conducting reverse transcriptase reaction using universal or miRNA-specific primers (e.g. looped RT-primers) within the body fluid sample such as urine or blood sample (e.g. whole blood, serum, or plasma) being a buffer so as to prepare directly cDNA samples, (ii) designing miRNA-specific cDNA forward primers and providing universal reverse primers to amplify the cDNA via polymerase chain reaction (PCR), (iii) adding a fluorescent dye (e.g. SYBR Green) or a fluorescent probe (e.g. Taqman probe) probe to conduct PCR, and (iv) detecting the miRNA(s) level in the body fluid sample such as urine or blood sample (e.g. whole blood, serum, or plasma).

A variety of kits and protocols to determine an expression profile by real time polymerase chain reaction (RT-PCR) such as real time quantitative polymerase chain reaction (RT qPCR) are available. For example, reverse transcription of miRNAs may be performed using the TaqMan MicroRNA Reverse Transcription Kit (Applied Biosystems) according to manufacturer's recommendations. Briefly, miRNA may be combined with dNTPs, MultiScribe reverse transcriptase and the primer specific for the target miRNA. The resulting cDNA may be diluted and may be used for PCR reaction. The PCR may be performed according to the manufacturer's recommendation (Applied Biosystems). Briefly, cDNA may be combined with the TaqMan assay specific for the target miRNA and PCR reaction may be performed using ABI7300. Alternative kits are available from Ambion, Roche, Qiagen, Invitrogen, SABiosciences, Exiqon etc.

The term "subject", as used in the context of the present invention, means a patient or individual or mammal suspected to be affected by psoriasis. The patient may be diagnosed to be affected by psoriasis, i.e. diseased, or may be diagnosed to be not affected by psoriasis, i.e. healthy. The subject may also be diagnosed to be affected by a specific form of psoriasis. The subject may further be diagnosed to develop psoriasis or a specific form of psoriasis as the inventors of the present invention surprisingly found that miRNAs representative for psoriasis are already present in the biological sample, e.g. blood sample, before psoriasis occurs or during the early stage of psoriasis. It should be noted that a subject that is diagnosed as being healthy, i.e. not suffering from psoriasis or from a specific form of psoriasis, may possibly suffer from another disease not tested/known. The subject may be any mammal, including both a human and another mammal, e.g. an animal such as a rabbit, mouse, rat, or monkey. Human subjects are particularly preferred. Therefore, the miRNA from a subject may be a human miRNA or a miRNA from another mammal, e.g. an animal miRNA such as a mouse, monkey or rat miRNA, or the miRNAs comprised in a set may be human miRNAs or miRNAs from another mammal, e.g. animal miRNAs such as mouse, monkey or rat miRNAs.

The term "control subject", as used in the context of the present invention, may refer to a subject known to be affected with psoriasis (positive control), i.e. diseased, or to a subject known to be not affected with psoriasis (negative control), i.e. healthy. It may also refer to a subject known to be effected by another disease/condition (see definition "(clinical) condition"). It should be noted that a control subject that is known to be healthy, i.e. not suffering from psoriasis, may possibly suffer from another disease not tested/known. The control subject may be any mammal, including both a human and another mammal, e.g. an animal such as a rabbit, mouse, rat, or monkey. Human "control subjects" are particularly preferred.

The term "set comprising at least two miRNAs representative for psoriasis", as used herein, refers to refers to at least two fixed defined miRNAs comprised in a set which are known to be differential (differentially expressed) between subjects (e.g. humans or other mammals such as animals) suffering from psoriasis (diseased state) and control subjects (e.g. humans or other mammals such as animals and are, thus, representative for psoriasis. Said "set comprising at least two miRNAs representative for psoriasis" are preferably selected from the group consisting of SEQ ID NO: 1 to 111 or SEQ ID NO: 1 to 283, a fragment thereof, and a sequence having at least 80% sequence identity thereto.

The term "psoriasis", as used herein refers to a chronic immune-mediated disease that appears on the skin. It occurs when the immune system sends out faulty signals that speed up the growth cycle of skin cells. There are five types of psoriasis: plaque, guttate, inverse, pustular and erythrodermic. The disorder is a chronic recurring condition that varies in severity from minor localized patches to complete body coverage. Psoriasis can also cause inflammation of the joints, which is known as psoriatic arthritis. Between 10% and 40% of all people with psoriasis have psoriatic arthritis. Psoriasis is typically a lifelong condition. There is currently no cure, but various treatments can help to control the symptoms. Many of the most effective agents used to treat severe psoriasis carry an increased risk of significant morbidity including skin cancers, lymphoma and liver disease. Psoriasis can get worse over time, but it is not possible to predict who will go on to develop extensive psoriasis or those in whom the disease may appear to vanish.

The inventors of the present invention surprisingly found that miRNAs are significantly dysregulated in body fluid samples such as blood of psoriasis subjects in comparison to a cohort of controls (healthy subjects) and thus, miRNAs are appropriated biomarkers for diagnosing and/or prognosing of psoriasis in a non-invasive fashion or minimal-invasive fashion. Furthermore, the sets of miRNAs of the present invention lead to high performance in diagnosing and/or prognosing of psoriasis, thus expose very high specificity, sensitivity and accuracy. They succeeded in determining the miRNAs that are differentially regulated in body fluid samples from patients having psoriasis compared to a cohort of controls (healthy subjects) (see experimental section for experimental details). Additionally, the inventors of the present invention performed hypothesis tests (e.g. t-test, limma-test) or other measurements (e.g. AUC, mutual information) on the expression level of the found miRNAs, in all controls (healthy subjects) and subjects suffering from psoriasis. These tests resulted in a significance value (p-value) for each miRNA. This p-value is a measure for the diagnostic power of each of these single miRNAs to discriminate, for example, between the two clinical conditions: controls (healthy subjects), i.e. not suffering from psoriasis, or diseased, i.e. suffering from psoriasis. Since a manifold of tests are carried out, one for each miRNA, the p-values may be too optimistic and, thus, over-estimate the actual discriminatory power. Hence, the p-values are corrected for multiple testing by the Benjamini Hochberg approach.

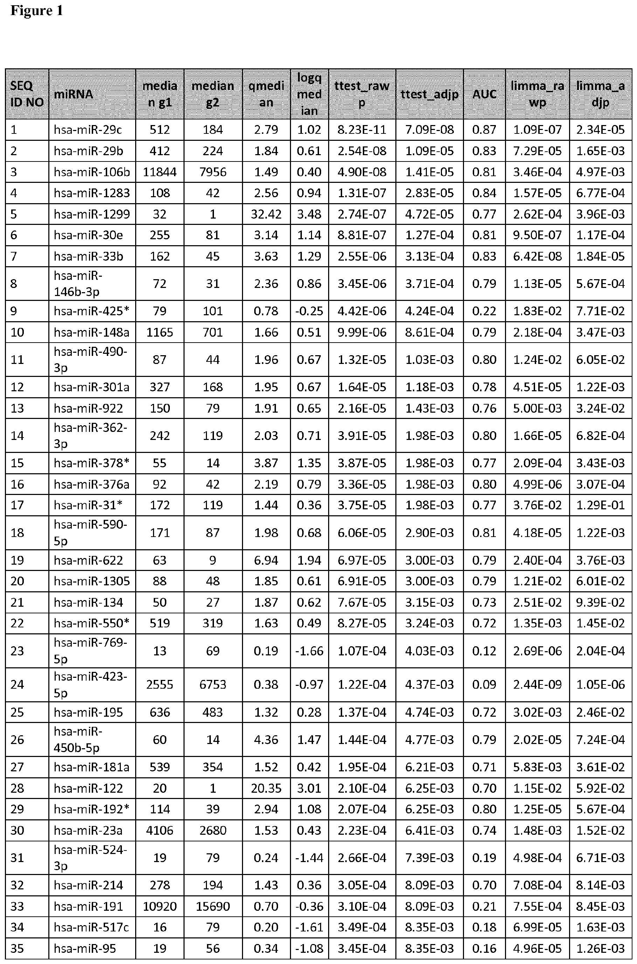

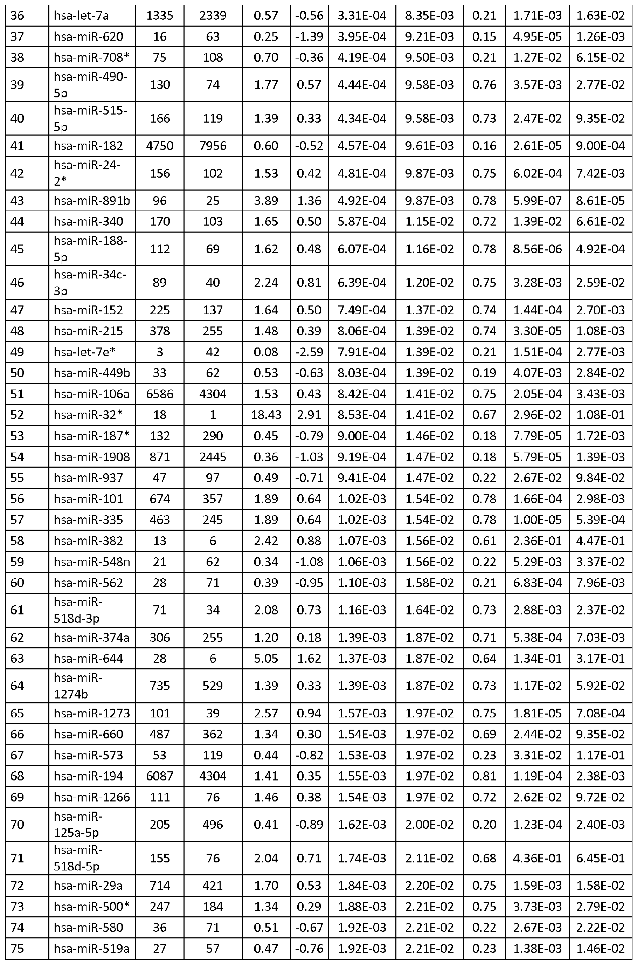

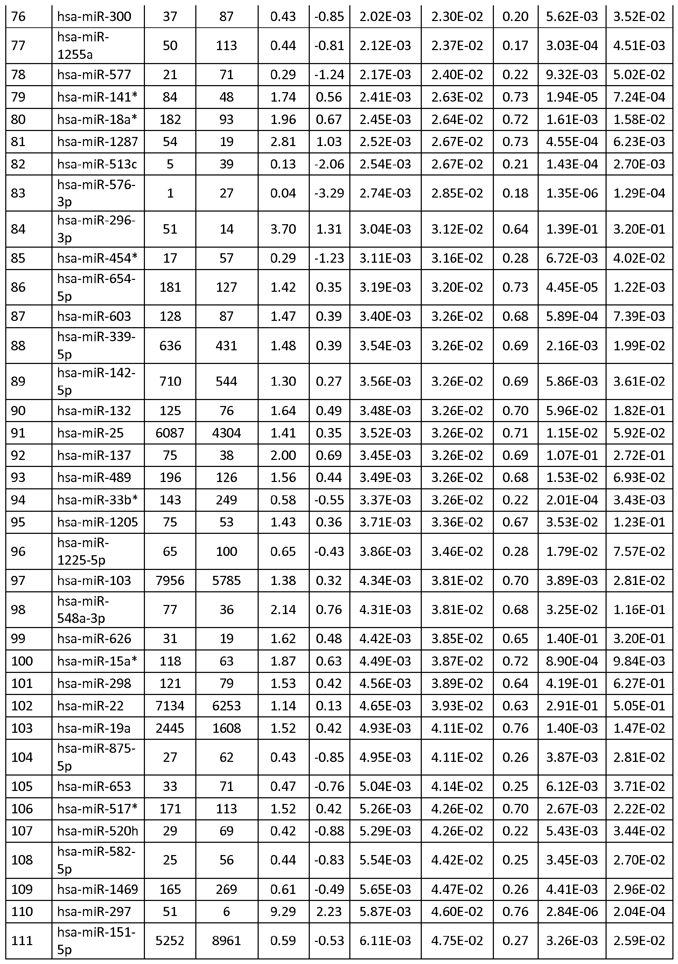

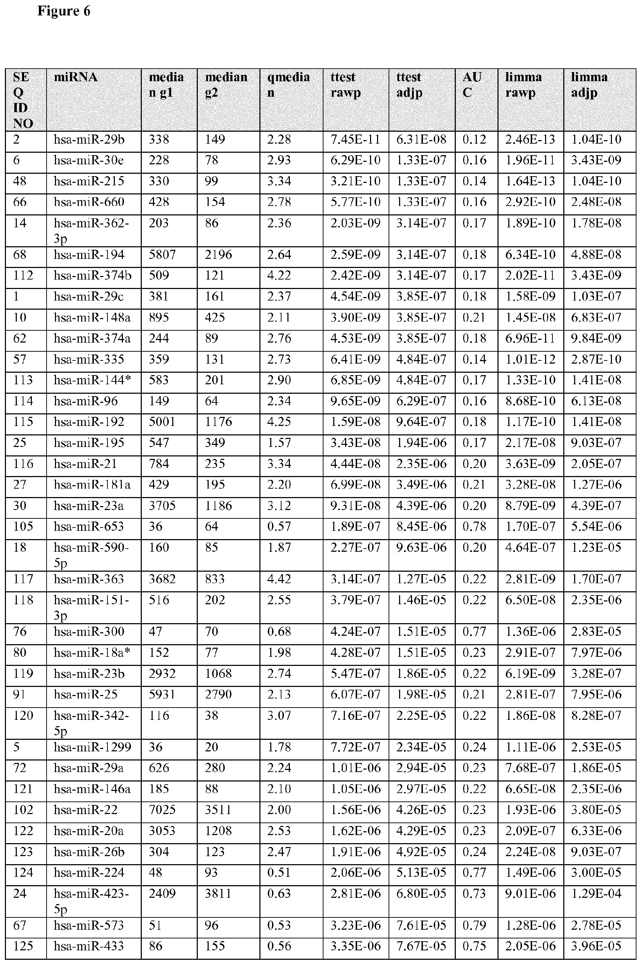

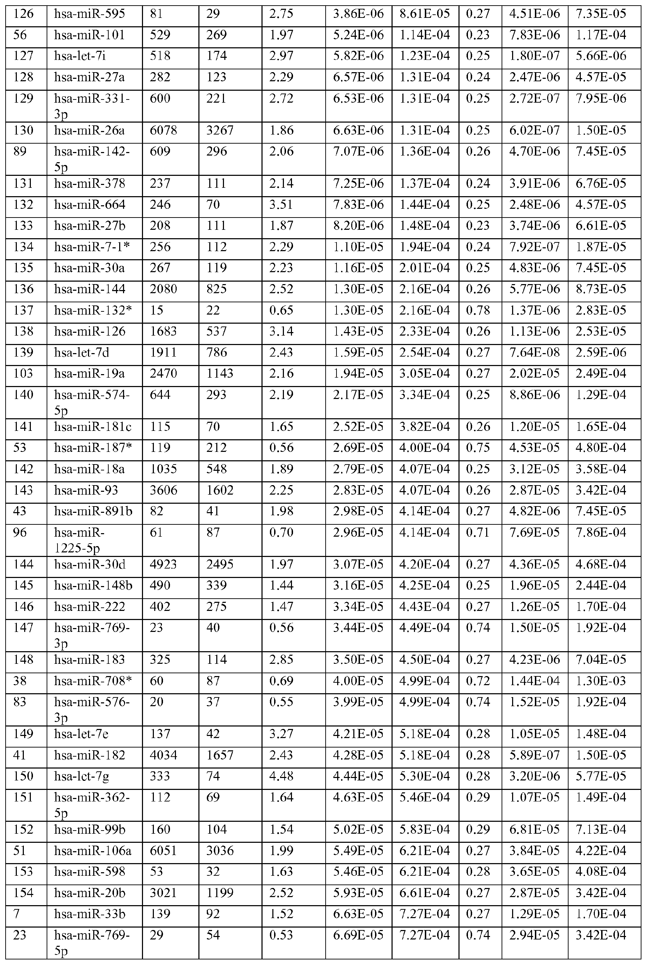

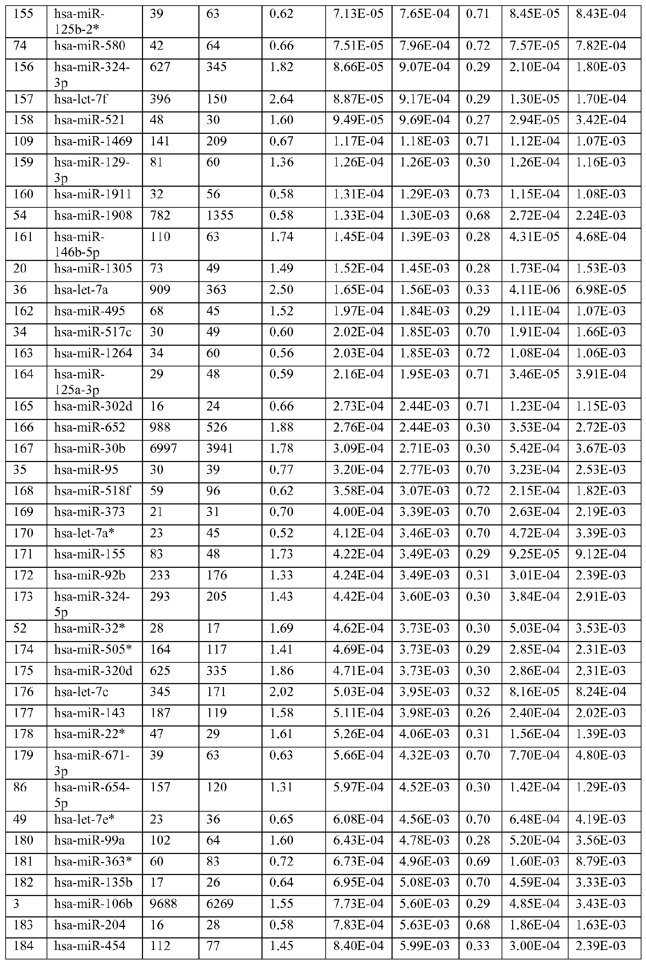

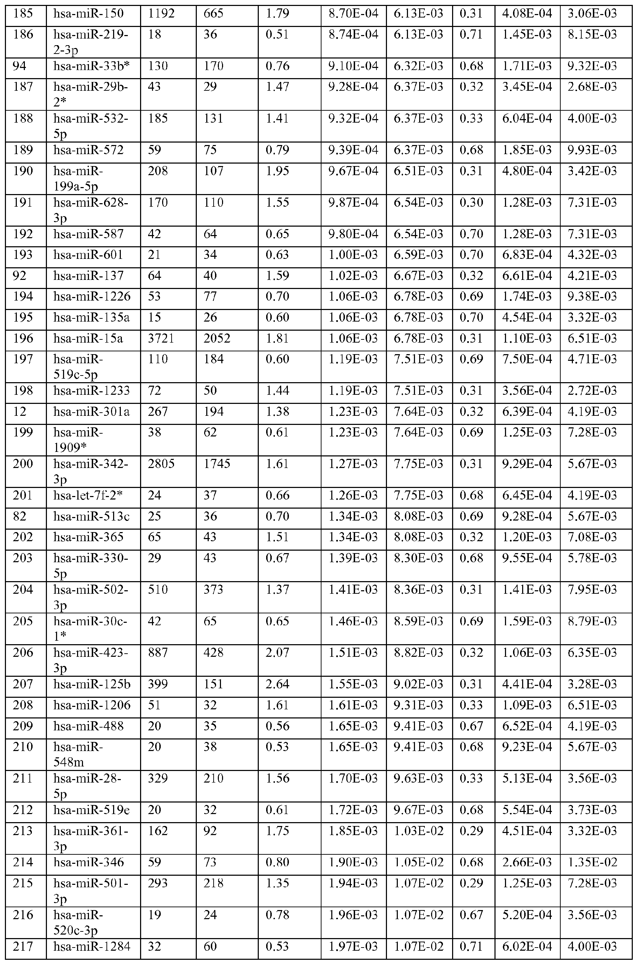

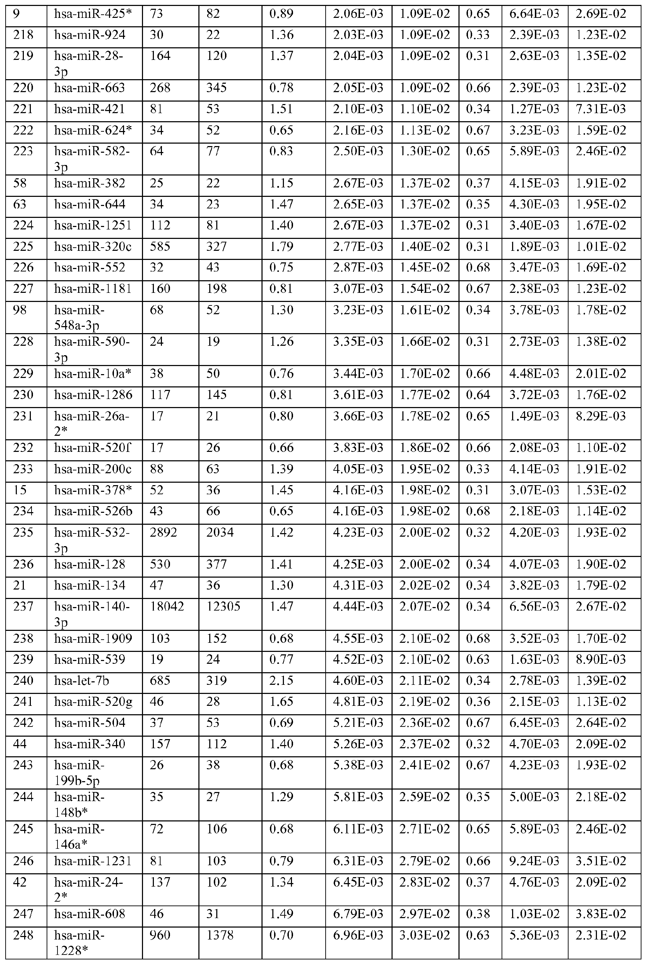

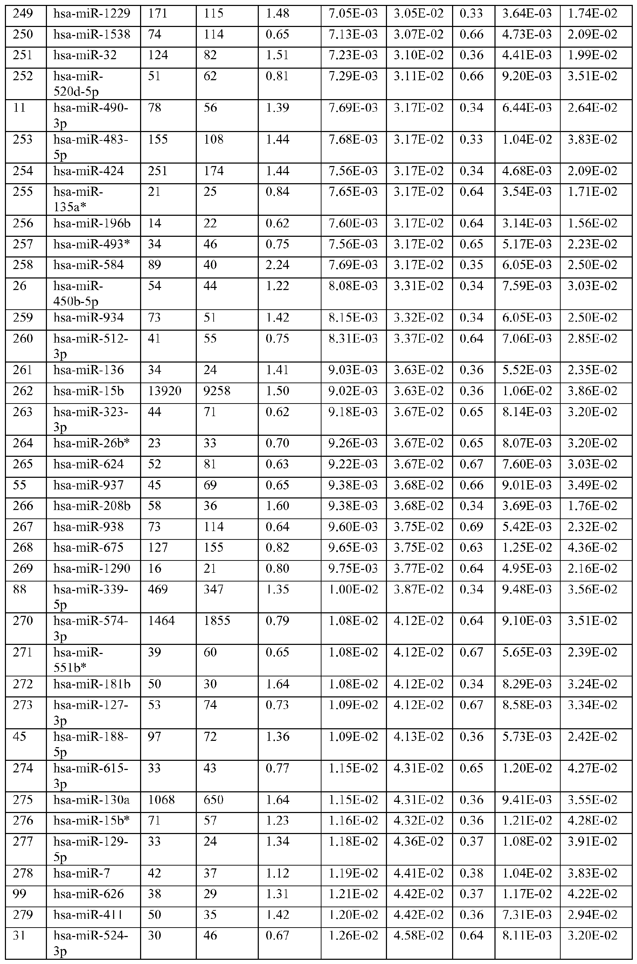

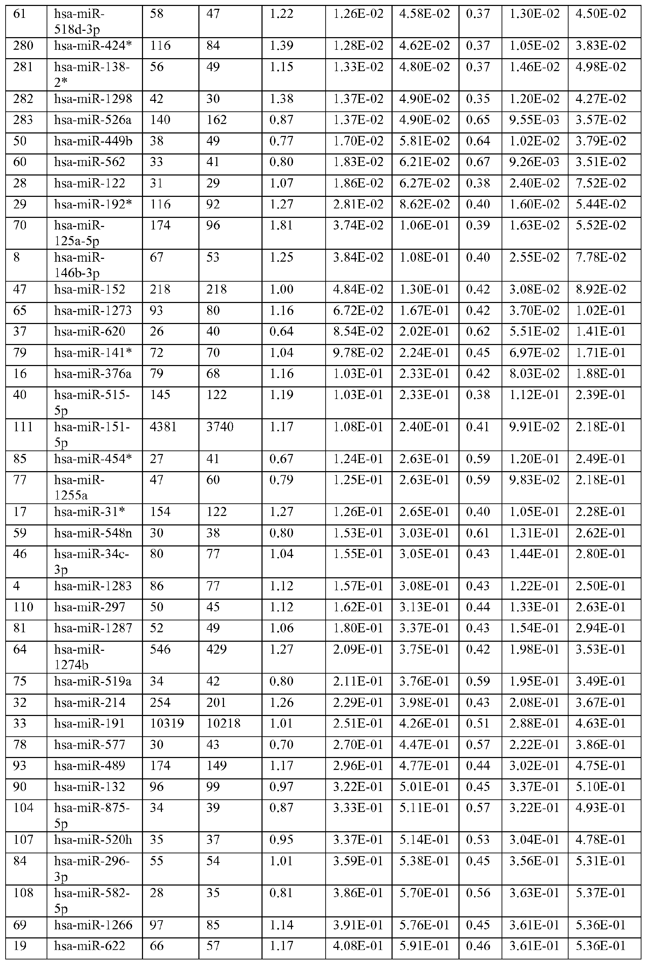

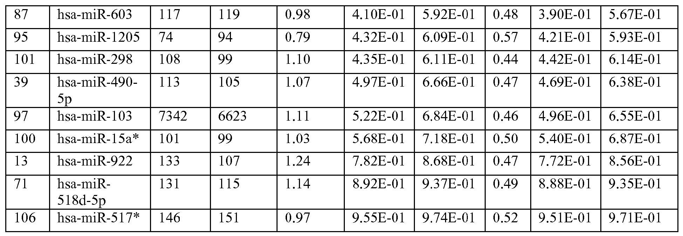

An overview of the miRNAs that are found to be significantly differentially regulated in biological samples of psoriasis and that performed best according to t-test, limma-test or AUC is provided in Figure 1 or Figure 6 (Experimental details: SEQ ID NO: sequence identification number, miRNA: identifier of the miRNA according to miRBase, median g1: median intensity obtained from microarray analysis for healthy controls, median g2: median intensity obtained from microarray analysis for individuals with psoriasis, qmedian: ratio of median g1/median g2, logqmedian: log of qmedian, ttest_rawp: p-value obtained when applying t-test, ttest_adjp: adjusted p-value in order to reduce false discovery rate by Benjamini-Hochberg adjustment, AUC: Area under the curve, limma_rawp: p-value obtained when applying limma-test, limma_adjp: adjusted p-value in order to reduce false discovery rate by Benjamini-Hochberg adjustment.). The miRNAs, i.e. miRNAs according to SEQ ID NO: 1 to 111 or SEQ ID NO: 1 to 283, are sorted in order of their t-test significance as described in more detail in the experimental section (see ttest_adjp= adjusted p-value calculated according to ttest). It should be noted that the lower the ttest_adjp value of a single miRNA, the higher is the diagnostic power of said miRNA for diagnosing and/or prognosing of psoriasis.

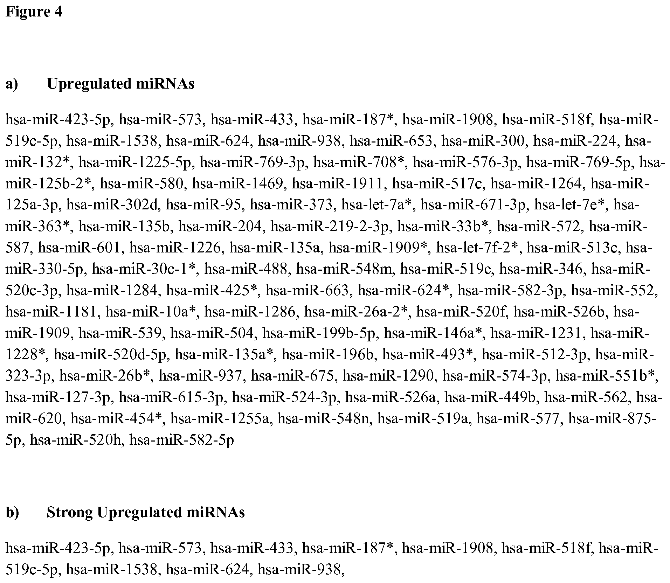

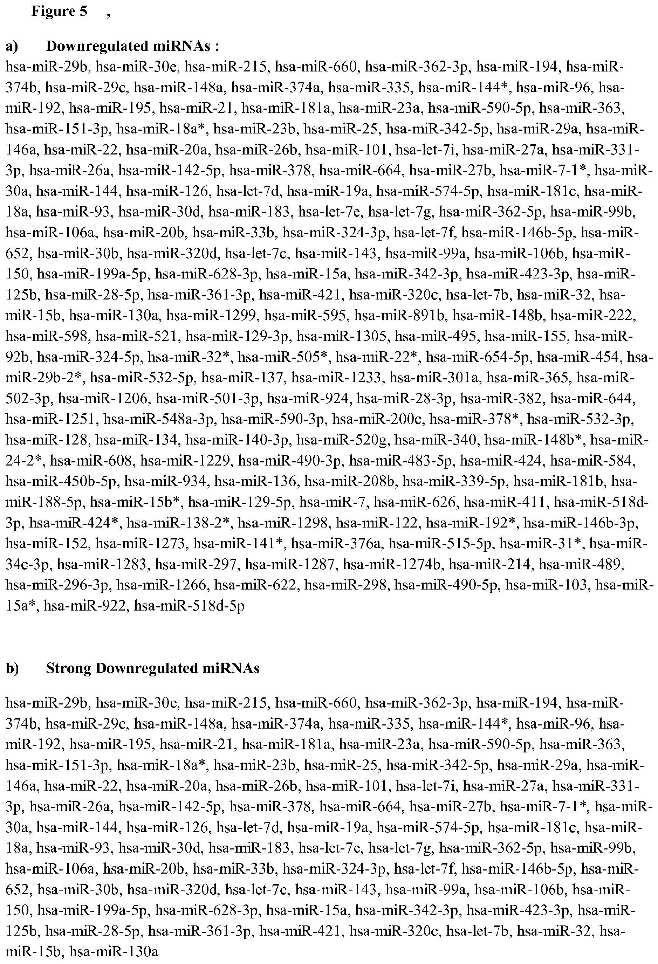

The significantly differentially regulated miRNAs are either up-regulated (see Figure 4a) or even strong up-regulated (see Figure 4b) or alternatively down-regulated (see Figure 5a) or even strong down-regulated (see Figure 5b) in biological samples of psoriasis as compared to healthy controls. Usually the diagnostic power of a single miRNA biomarker is not sufficient to reach high accuracy, specificity and sensitivity for discrimination between healthy subjects (controls) and subjects suffering from psoriasis, hence no simple threshold method can be used for diagnosis and/or prognosis.

Therefore, the inventors of the present invention employed more than one miRNA biomarker, i.e. sets of miRNA biomarkers (signatures), to further increase and/or improve the performance for diagnosing and/or prognosing of subjects suffering from psoriasis. This leads to a significant increase in sensitivity, specificity and accuracy when compared to the prior art.

In order to be able to discriminate, for example, between two or more clinical conditions, e.g. healthy and suffering from psoriasis, for a defined set of miRNA biomarkers, the inventors of the present invention applied a machine learning approach (e.g. t-test, AUC, support vector machine, hierarchical clustering, or k-means) which leads to an algorithm that is trained by reference data (i.e. data of reference miRNA expression profiles from the two clinical conditions, e.g. healthy and suffering from psoriasis, for the defined set of miRNA markers) to discriminate between the two statistical classes (i.e. two clinical conditions, e.g. healthy or suffering from psoriasis).

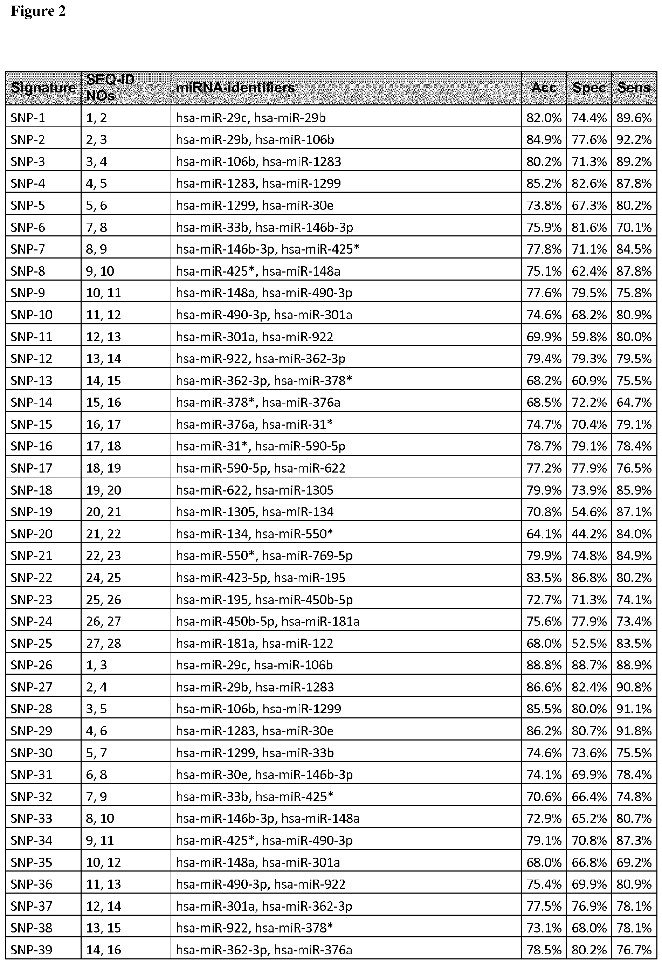

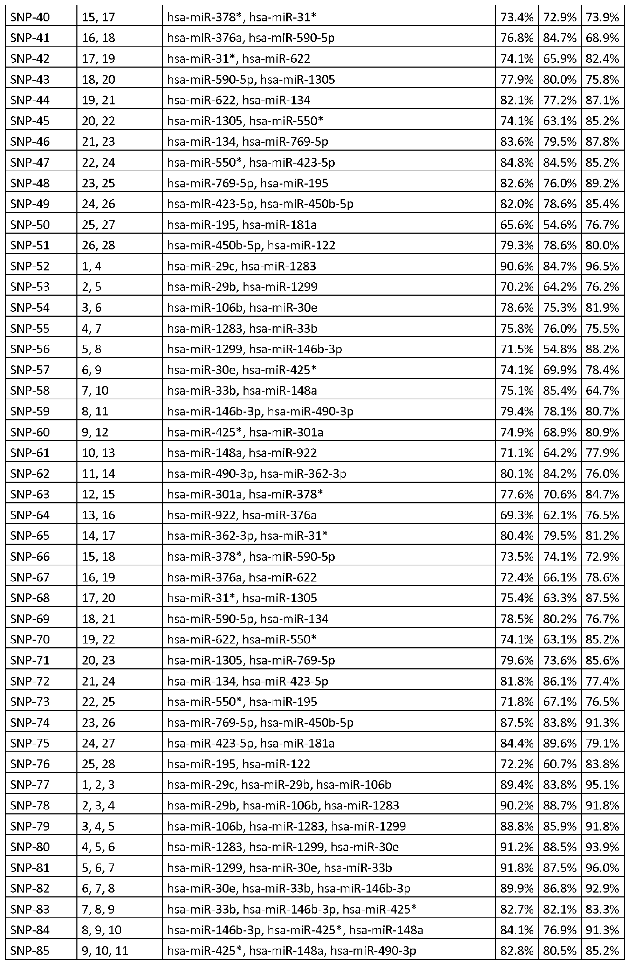

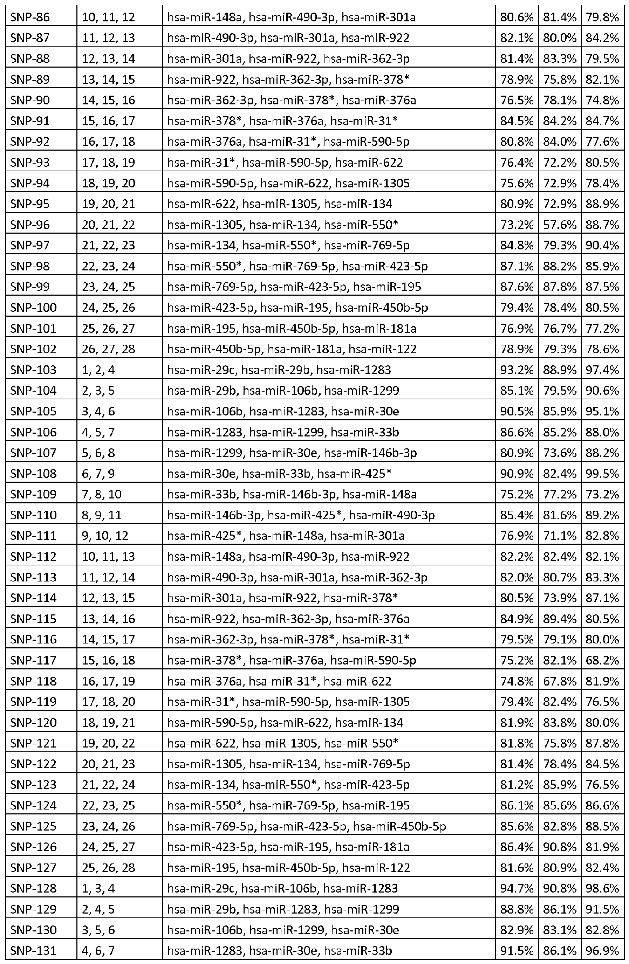

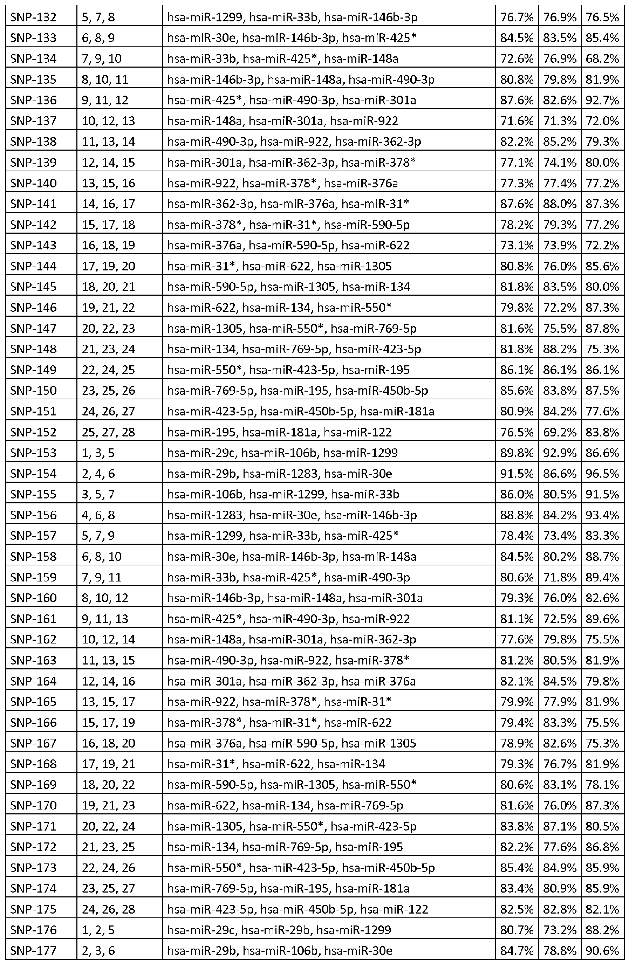

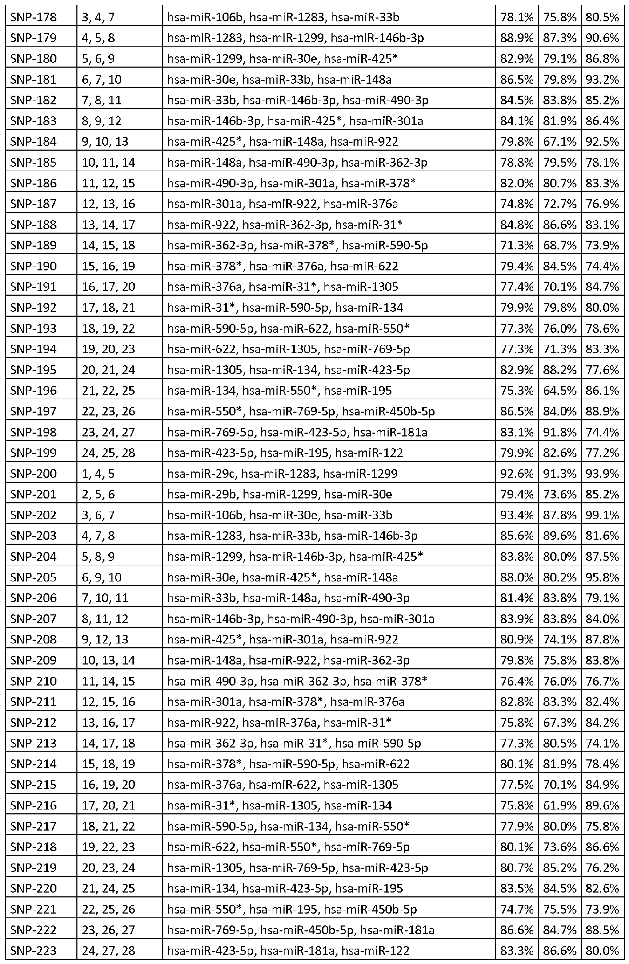

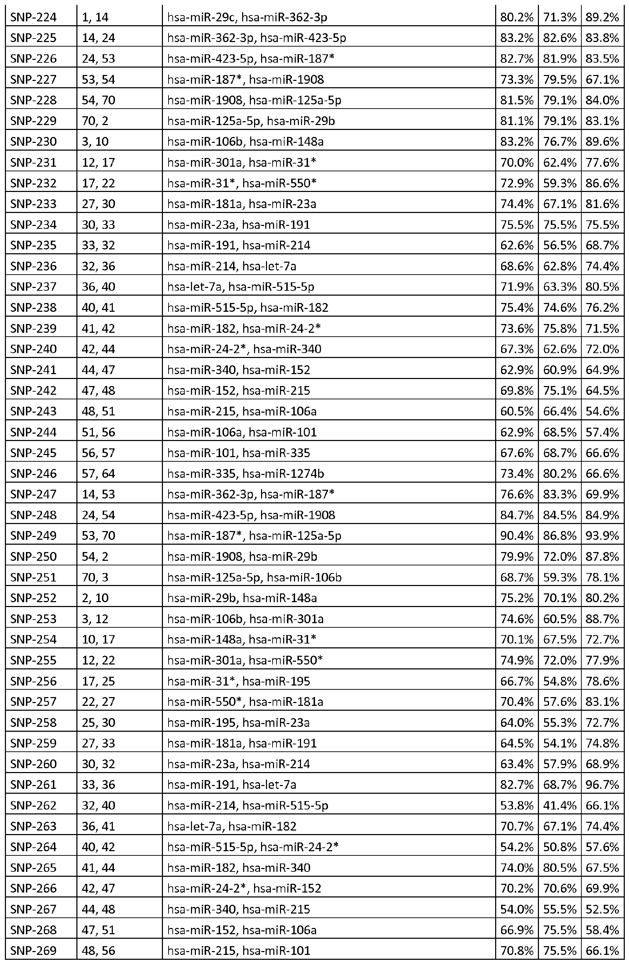

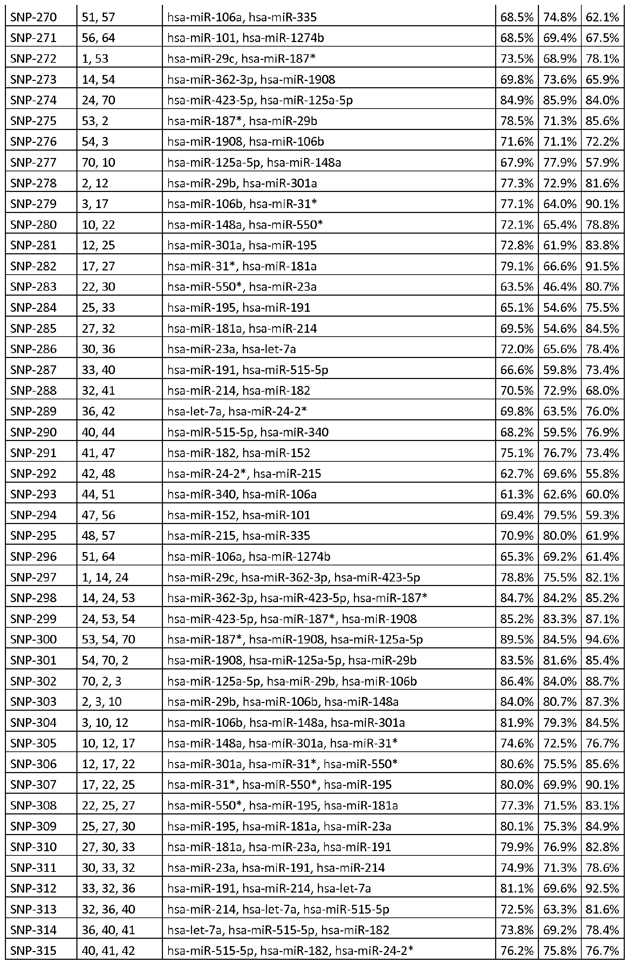

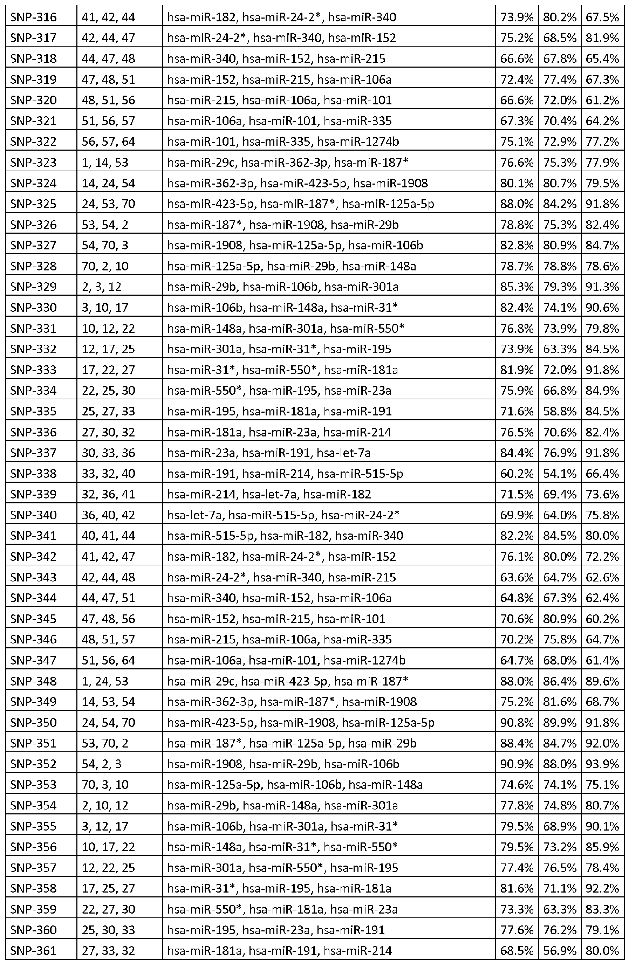

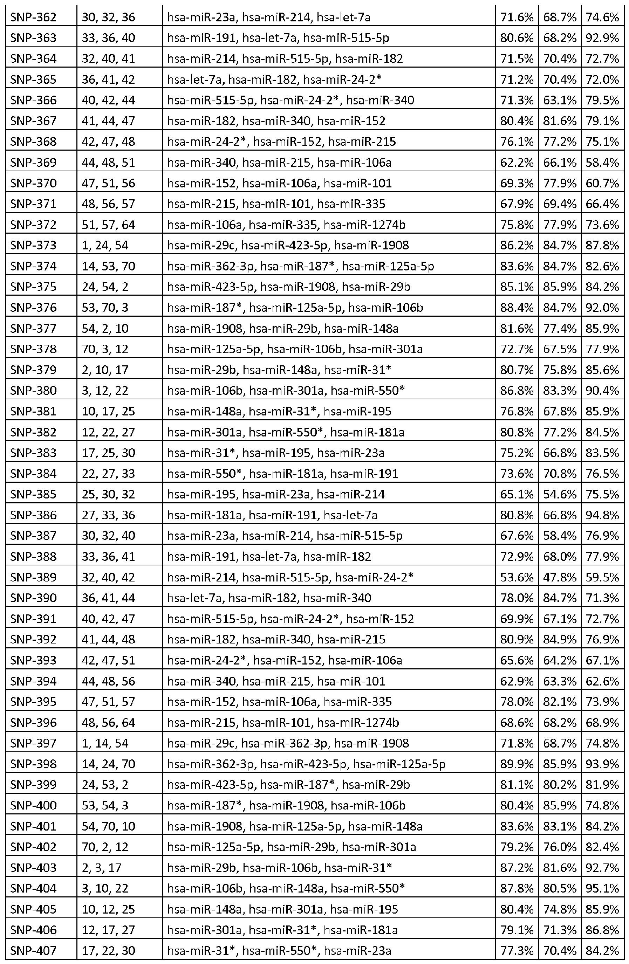

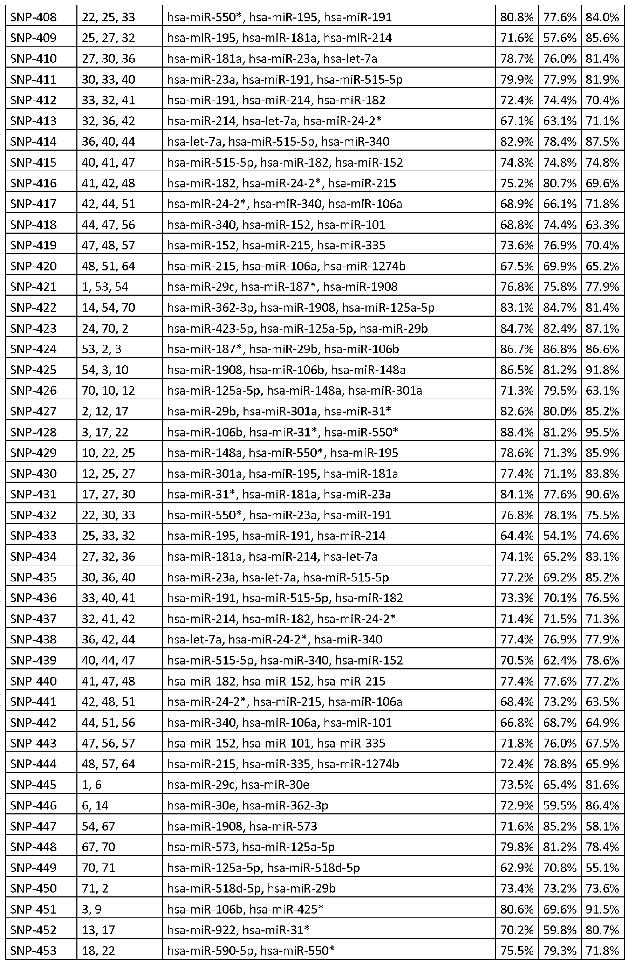

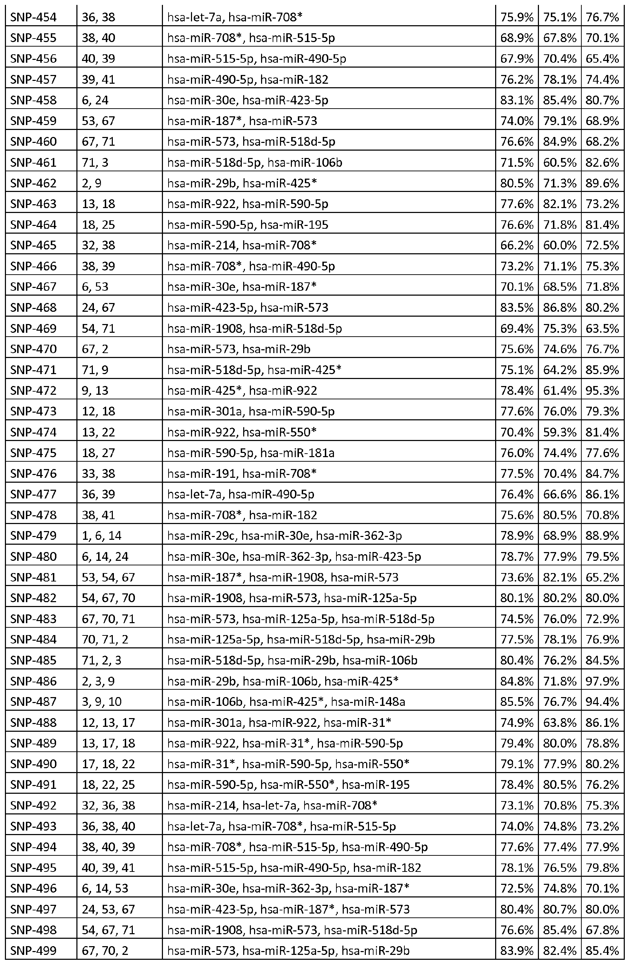

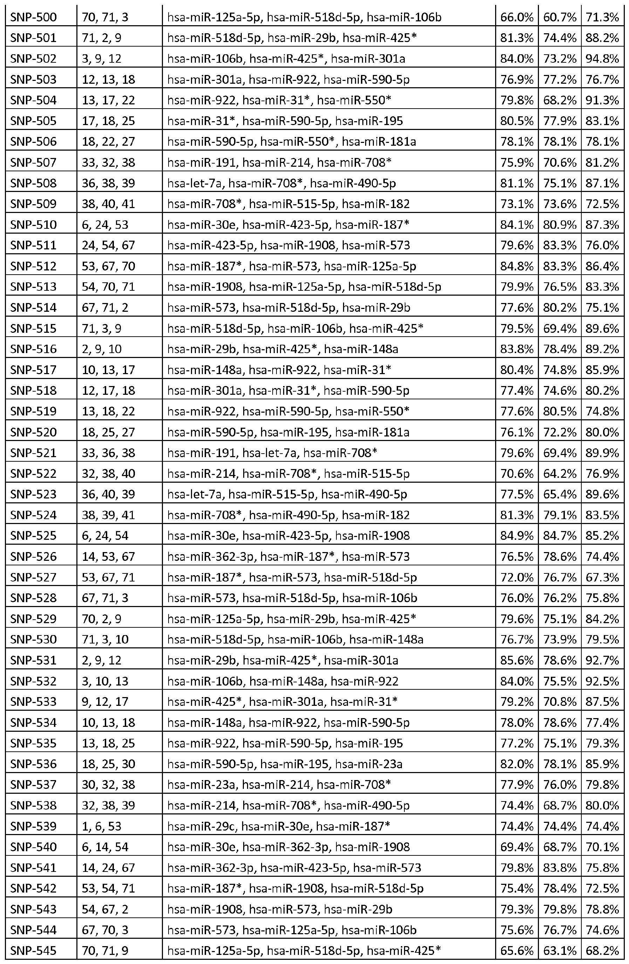

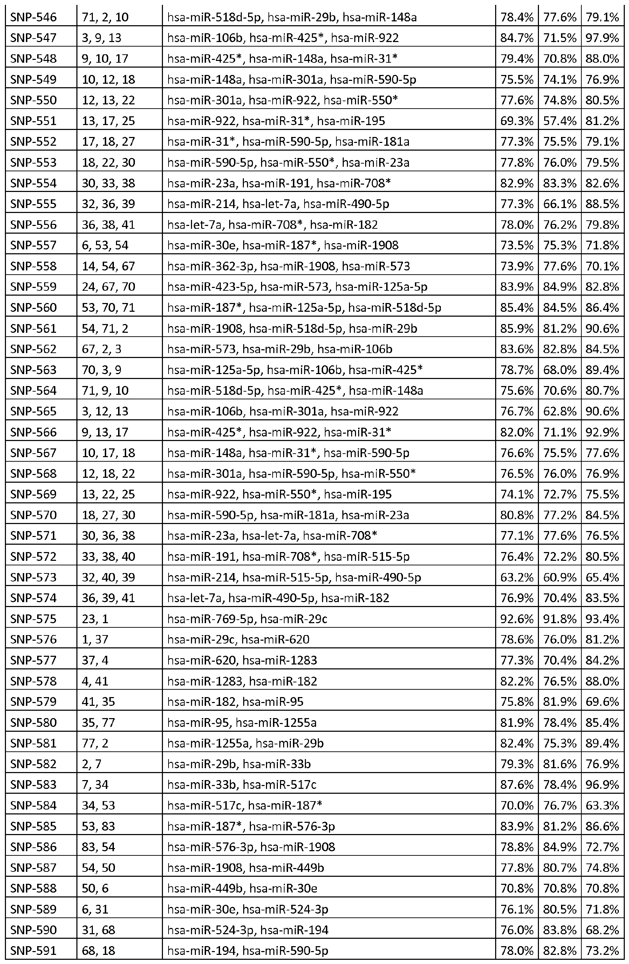

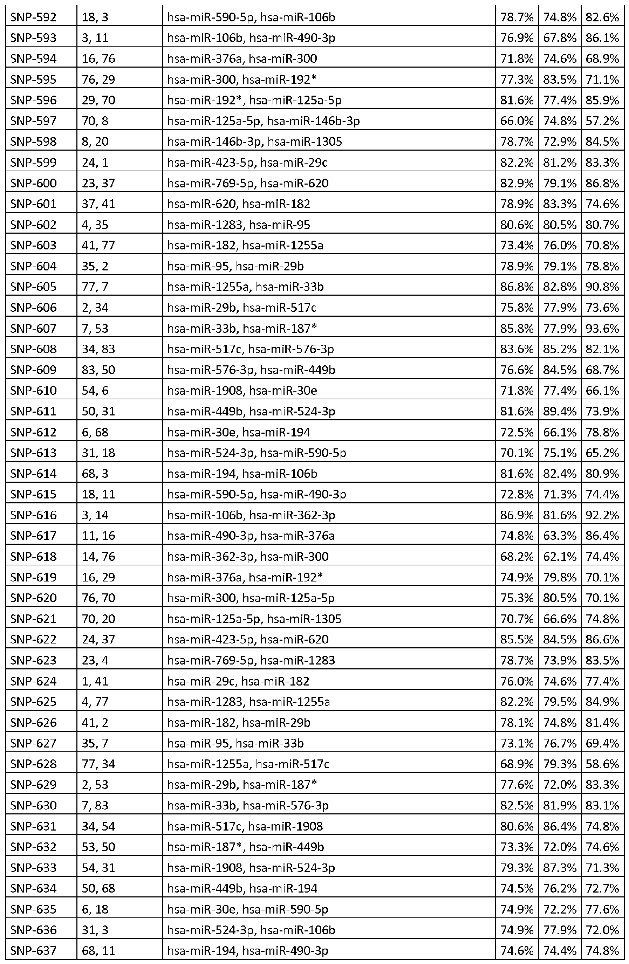

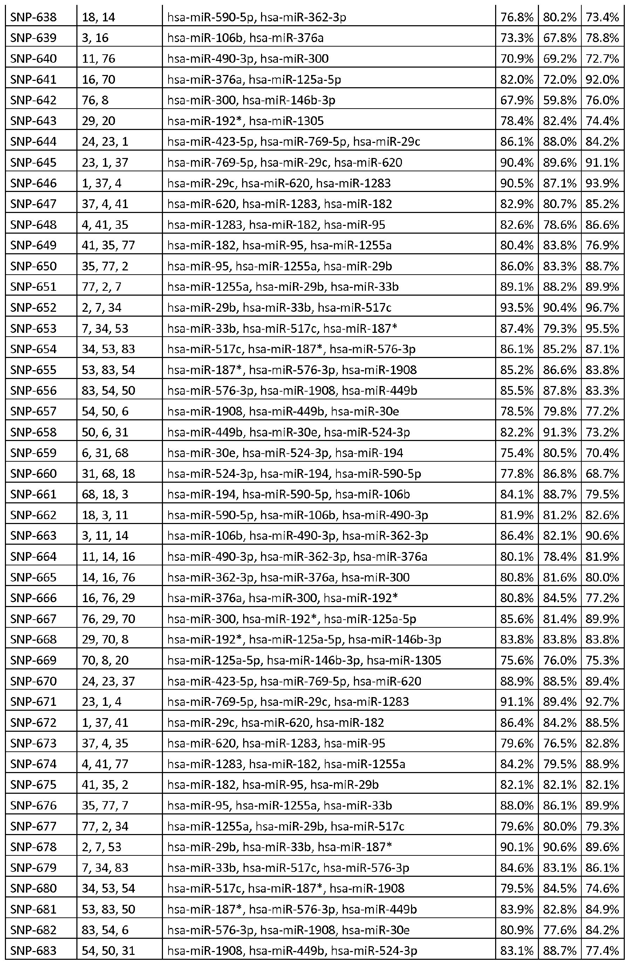

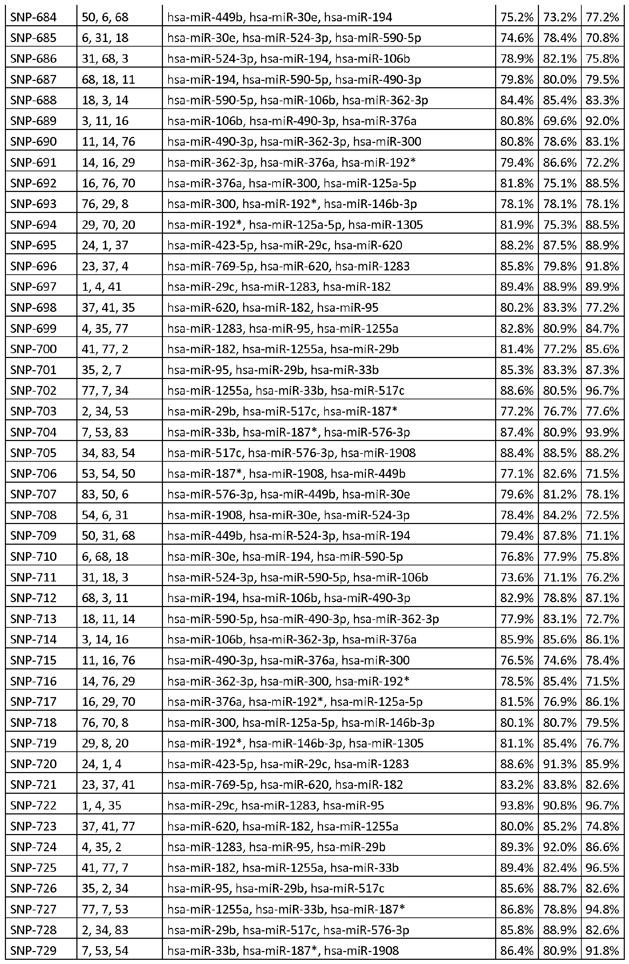

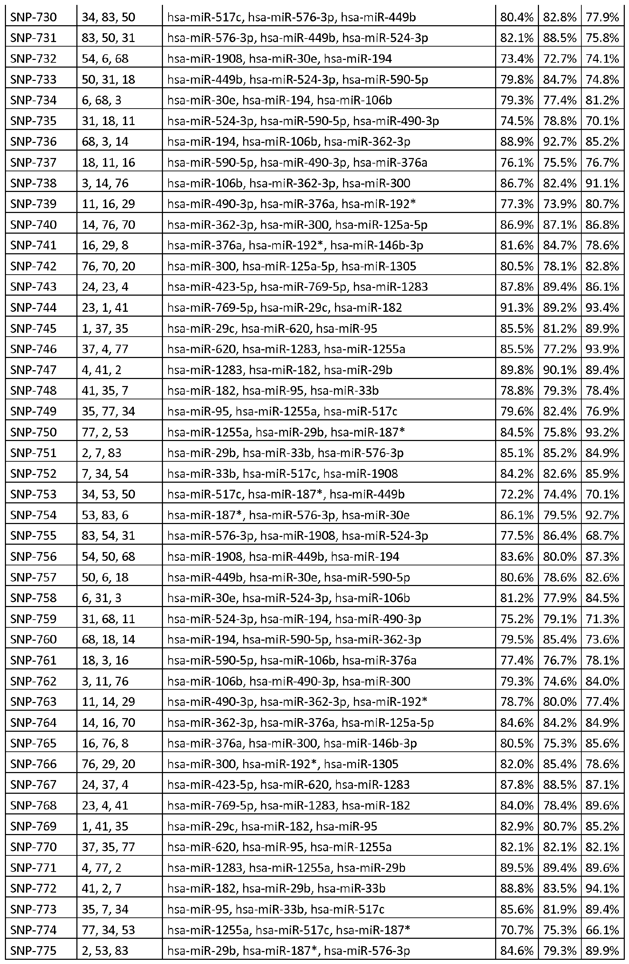

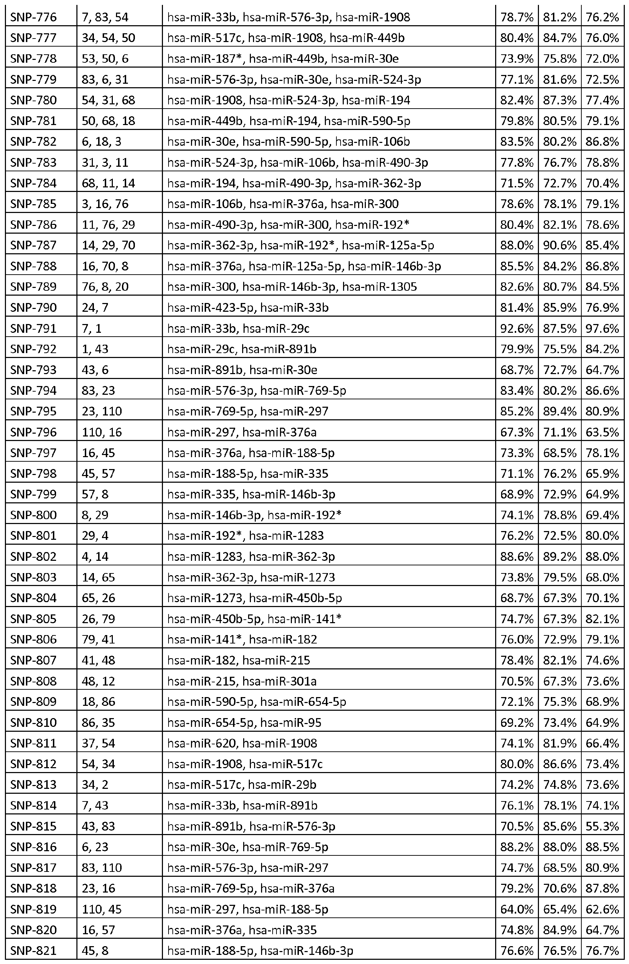

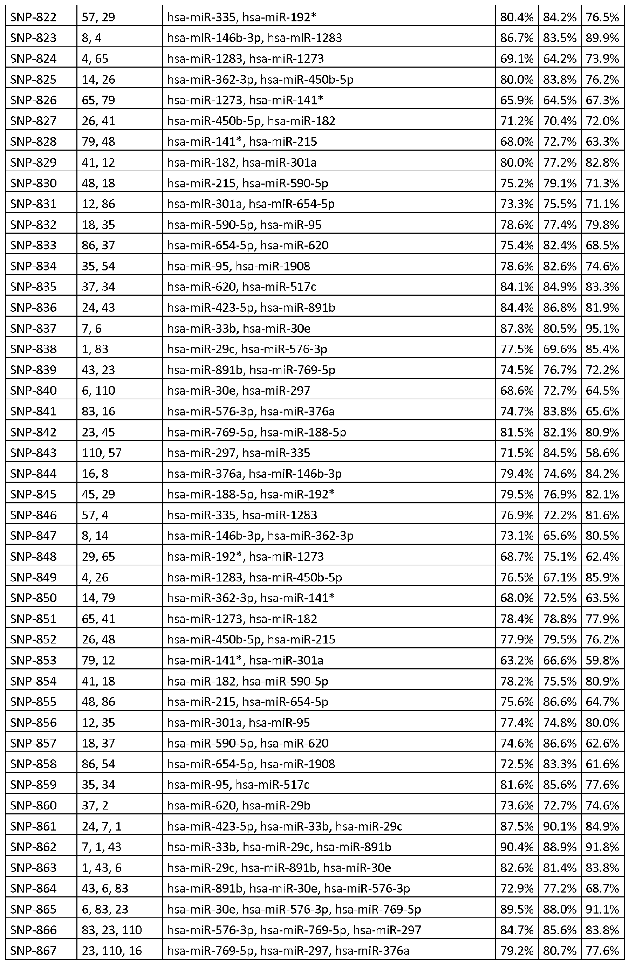

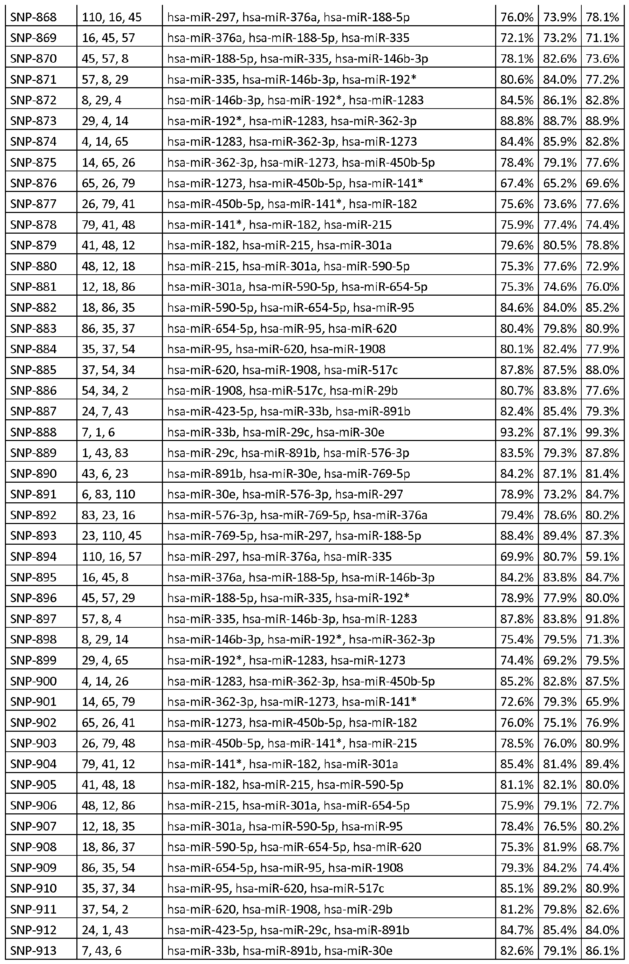

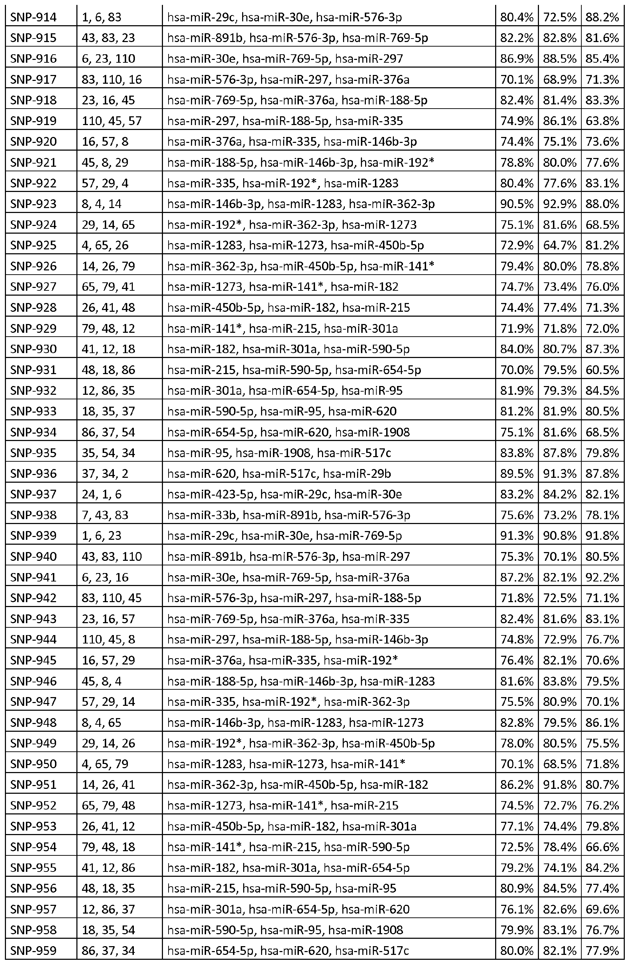

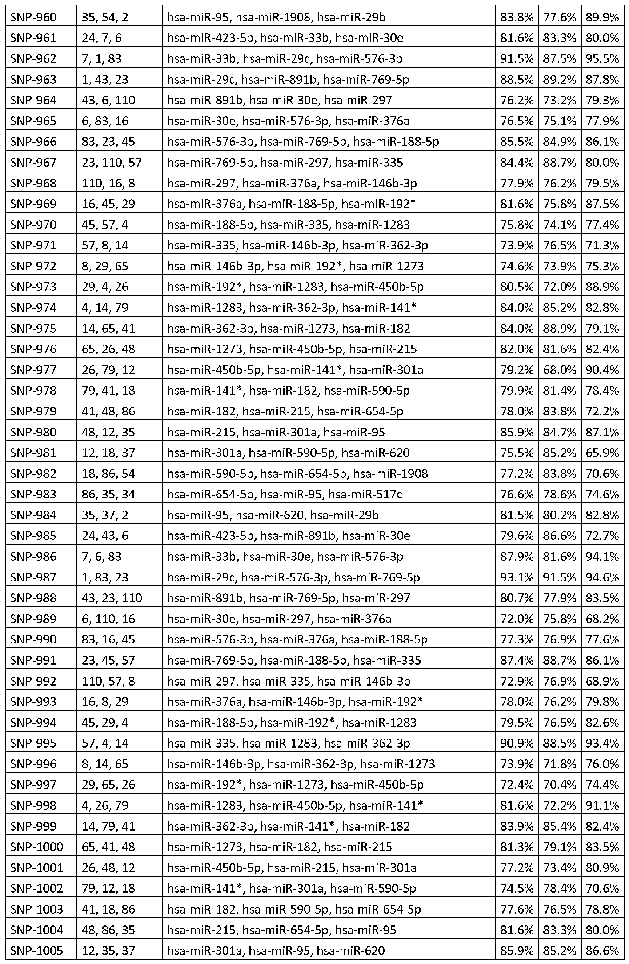

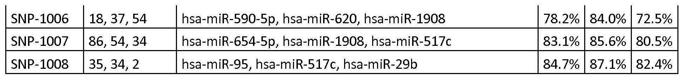

The inventors of the present invention surprisingly found that this approach yields in miRNA sets (signatures) that provide high diagnostic accuracy, specificity and sensitivity in the determination of psoriasis in patients (see Figure 2). Said miRNA sets (signatures) comprise at least two miRNAs, wherein the nucleotide sequences of said miRNAs are preferably selected from the group consisting of SEQ ID NO: 1 to 111 or SEQ ID NO: 1 to 283 (see Figure 1 or Figure 6).

An exemplarily approach to arrive at miRNA sets/signatures that correlate with psoriasis is summarized below :
Step 1: Total RNA (or subfractions thereof) is extracted from the biological sample, e.g. a body fluid sample, preferably a blood sample (including, but not limited to plasma, serum, PBMC or other blood fractions), using suitable kits and/or purification methods.
Step 2: From the respective samples the quantity (expression level) of one miRNA or sets of at least two miRNAs, e.g. selected from the group consisting of SEQ ID NO: 1 to SEQ ID NO: 111 or SEQ ID NO: 1 to 283, is measured using experimental techniques. These techniques include but are not restricted to array based approaches, amplification methods (PCR, RT-PCR, qPCR), sequencing, next generation sequencing, flow cytometry and/or mass spectroscopy.
Step 3: In order to gather information on the diagnostic/prognostic value and the redundancy of each of the single miRNA biomarkers, mathematical methods are applied. These methods include, but are not restricted to, basic mathematic approaches (e.g. Fold Quotients, Signal to Noise ratios, Correlation), statistical methods as hypothesis tests (e.g. t-test, Wilcoxon-Mann-Whitney test), the Area under the Receiver operator Characteristics Curve, information theory approaches, (e.g. the Mutual Information, Cross-entropy), probability theory (e.g. joint and conditional probabilities) or combinations and modifications of the previously mentioned methods.
Step 4: The information gathered in step 3) is used to estimate for each miRNA biomarker the diagnostic content or value. Usually, however, this diagnostic value is too small to get a highly accurate diagnosis with accuracy rates, specificities and sensitivities beyond the 90% barrier.
   The diagnostic content of the miRNAs suitable for diagnosing/prognosing psoriasis is exemplarily listed in Figure 1 or Figure 6 (Experimental details: SEQ ID NO: sequence identification number, miRNA: identifier of the miRNA according to miRBase, median g1: median intensity obtained from microarray analysis for healthy controls, median g2: median intensity obtained from microarray analysis for subjects with psoriasis, qmedian: ratio of median g1/median g2, logqmedian: log of qmedian, ttest_rawp: p-value obtained when applying t-test, ttest_adjp: adjusted p-value in order to reduce false discovery rate by Benjamini-Hochberg adjustment, AUC: Area under the curve, limma_rawp: p-value obtained when applying limma-test, limma_adjp: adjusted p-value in order to reduce false discovery rate by Benjamini-Hochberg adjustment.). These Figures include the miRNAs according to SEQ ID NO: 1 to 111 or SEQ ID NO: 1 to 283.
Step 5: In order to increase the performance for diagnosing/prognosing of subjects suffering from psoriasis, more than one miRNA biomarker needs to be employed. Thus statistical learning / machine learning / bioinformatics / computational approaches are applied for set selection in order to select/define sets of miRNA biomarkers (e.g. comprising miRNAs SEQ ID NO: 1 to 111 or SEQ ID NO: 1 to 283) that are tailored for the detection of psoriasis. These techniques include, but are not restricted to, Wrapper subset selection techniques (e.g. forward step-wise, backward step-wise, combinatorial approaches, optimization approaches), filter subset selection methods (e.g. the methods mentioned in Step 3), principal component analysis, or combinations and modifications of such methods (e.g. hybrid approaches).
Step 6: The subsets, selected/defined in Step 5, which may range from only a small number (at least two for the set) to all measured biomarkers is then used to carry out a diagnosis/prognosis of psoriasis. To this end, statistical learning / machine learning / bioinformatics / computational approaches are applied that include but are not restricted to any type of supervised or unsupervised analysis: classification techniques (e.g. naive Bayes, Linear Discriminant Analysis, Quadratic Discriminant Analysis Neural Nets, Tree based approaches, Support Vector Machines, Nearest Neighbour Approaches), Regression techniques (e.g. linear Regression, Multiple Regression, logistic regression, probit regression, ordinal logistic regression ordinal Probit-Regression, Poisson Regression, negative binomial Regression, multinomial logistic Regression, truncated regression), Clustering techniques (e.g. k-means clustering, hierarchical clustering, PCA), Adaptations, extensions, and combinations of the previously mentioned approaches.
Step 7: By combination of subset selection (Step 5) and machine learning (Step 6) an algorithm or mathematical function for diagnosing/prognosing psoriasis is obtained. This algorithm or mathematical function is applied to a miRNA expression profile of a subject to be diagnosed for psoriasis.

In a first aspect, the present invention relates to a method for diagnosing and/or prognosing of psoriasis comprising the steps of:
(i) determining an expression profile of a set comprising at least two miRNAs representative for psoriasis in a blood sample from a subject, and
(ii) comparing said expression profile to a reference, wherein the comparison of said expression profile to said reference allows for the diagnosis and/or prognosis of psoriasis,
wherein the nucleotide sequences of the miRNAs comprised in the set are selected from the group consisting of SEQ ID NO: 1 to 111, a fragment thereof, and a sequence having at least 80% sequence identity thereto, and wherein the nucleotide sequence of a first of said at least two miRNAs is SEQ ID NO: 2, and wherein the blood sample is a blood cell sample.

Preferably, the blood cell sample is a leukocyte- , erythrocyte- and/or platelet-containing sample.

It is further preferred that the blood sample has been collected under conditions where the RNA-fraction is guarded against degradation, preferably the blood sample is collected in a PAXgene (RNA) Tube.

It is preferred that the subject is a mammal including both a human and another mammal, e.g. an animal such as a mouse, a rat, a rabbit, or a monkey. It is particularly preferred that the subject is a human.

Preferably, the set comprising at least two miRNAs comprises at least one up-regulated miRNA listed in Figure 4a or comprises at least one down-regulated miRNAs listed in Figure 5a. More preferably, the set comprising at least two miRNAs comprises at least one up-regulated miRNA listed in Figure 4b or comprises at least one down-regulated miRNAs listed in Figure 5b. It is further preferred, that the set comprising at least two miRNAs comprises at least one up-regulated miRNA listed in Figure 4a and further comprises at least one down-regulated miRNAs listed in Figure 5a.

It is preferred that the set comprising at least two miRNAs is selected from the set of miRNAs listed in Figure 2, which comprise SEQ ID NO: 2.

It is preferred that the set comprising at least two miRNAs comprises at least one set of miRNAs listed in Figure 2, which comprise SEQ ID NO: 2.

Thus, it is preferred that the method for diagnosing and/or prognosing of psoriasis comprises the steps of:
(i) determining an expression profile (expression profile data) of a set comprising, essentially consisting of, or consisting of at least two miRNAs representative for psoriasis in a blood sample from a subject (e.g. a human or another mammal such as an animal), and
(ii) comparing said expression profile (expression profile data) to a reference, wherein the comparison of said expression profile (expression profile data) to said reference allows for the diagnosis and/or prognosis of psoriasis,
wherein the nucleotide sequences of the miRNAs comprised in the set are selected from the group consisting of SEQ ID NO: 1 to 111, a fragment thereof, and a sequence having at least 80% sequence identity thereto, and wherein the nucleotide sequence of a first of said at least two miRNAs is SEQ ID NO: 2, and wherein the blood sample is a blood cell sample.

Thus, for analysis of a blood cell sample) in step (i) of the method of the present invention, an expression profile of a set comprising at least two miRNAs which are known to be differential between subjects (e.g. humans or other mammals such as animals) having or being suspected to have psoriasis or a special form of psoriasis (diseased state) and subjects (e.g. humans or other mammals such as animals) not having psoriasis or a special form of psoriasis (healthy/control state) and are, thus, representative for psoriasis, is determined, wherein the nucleotide sequences of said miRNAs are selected from the group consisting of SEQ ID NO: 1 to 111, a fragment thereof, and a sequence having at least 80% sequence identity thereto, wherein the nucleotide sequence of a first of said at least two miRNAs is SEQ ID NO: 2.

It is more particularly preferred that an expression profile of a set comprising, essentially consisting of, or consisting of at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40 or more, or comprising/consisting of 111 miRNAs, representative for psoriasis in a blood cell sample from a subject (e.g. a human or another mammal such as an animal) is determined in the step (i) of the method of the present invention, wherein the nucleotide sequences of said miRNAs are selected from the group consisting of
(i) a nucleotide sequence according to SEQ ID NO: 1 to SEQ ID NO: 111,
(ii) a nucleotide sequence that is a fragment of the nucleotide sequence according to (i), preferably, a nucleotide sequence that is a fragment which is between 1 and 12, more preferably between 1 and 8, and most preferably between 1 and 5 or 1 and 3, i.e. 1, 2, 3, 4, 5, 6, 7, 8, 9, 10, 11, or 12, nucleotides shorter than the nucleotide sequence according to (i), and
(iii) a nucleotide sequence that has at least 80%, preferably at least 85%, more preferably at least 90%, and most preferably at least 95% or 99%, i.e. 80, 81, 82, 83, 84, 85, 86, 87, 88, 89, 90, 91, 92, 93, 94, 95, 96, 97, 98, or 99%, sequence identity to the nucleotide sequence according to (i) or nucleotide sequence fragment according to (ii).

Additionally, it is more particularly preferred that an expression profile of a set comprising, essentially consisting of, or consisting of at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40 or more, or comprising/consisting of 111 miRNAs, representative for psoriasis in a blood cell sample from a subject (e.g. a human or another mammal such as an animal) is determined in the step (i) of the method of the present invention, wherein the set comprising at least two miRNAs is selected from the group consisting of
(i) a set of miRNAs listed in Figure 2
(ii) a combination of at least 2 sets of miRNAs listed in Figure 2
(iii) nucleotide sequences that are fragments of the nucleotide sequence according to (i) or (ii), preferably, nucleotide sequences that are fragments which are between 1 and 12, more preferably between 1 and 8, and most preferably between 1 and 5 or 1 and 3, i.e. 1, 2, 3, 4, 5, 6, 7, 8, 9, 10, 11, or 12, nucleotides shorter than the nucleotide sequences according to (i) or (ii), and
(iv) nucleotide sequences that have at least 80%, preferably at least 85%, more preferably at least 90%, and most preferably at least 95% or 99%, i.e. 80, 81, 82, 83, 84, 85, 86, 87, 88, 89, 90, 91, 92, 93, 94, 95, 96, 97, 98, or 99%, sequence identity to the nucleotide sequences according to (i) or (ii) or nucleotide sequence fragments according to (iii).

A first of said at least two miRNAs comprised in the set mentioned above is SEQ ID NO: 2. In addition, the set of miRNAs mentioned above comprises at least one of the sets of miRNAs listed in Figure 2, which comprise SEQ ID NO: 2.

It is particularly preferred that the nucleotide sequences as defined in (iv) have at least 80%, preferably at least 85%, more preferably at least 90%, and most preferably at least 95% or 99%, i.e. 80, 81, 82, 83, 84, 85, 86, 87, 88, 89, 90, 91, 92, 93, 94, 95, 96, 97, 98, or 99%, sequence identity over a continuous stretch of at least 10, 11, 12, 13, 14, 15, 16, 17, 18, 19, 20 or more nucleotides, preferably over the whole length, to the nucleotide sequences of the nucleotides according to (i) or (ii) or nucleotide fragments according to (iii).

Furthermore, according to the present invention, a first diagnosis and/or prognosis of psoriasis can be performed employing, as disclosed, miRNA-detection in a blood cell sample followed by a second diagnosis and/or prognosis that is based on other methods (e.g. other biomarkers and/or imaging methods).

Furthermore, according to the present invention, the set comprising at least two miRNAs for diagnosing and/or prognosing psoriasis in a blood cell sample from a patient, e.g. human or animal, may be established on one experimental platform (e.g. microarray/biochip), while for routine diagnosis/prognosis another experimental platform (e.g. qPCR) may be chosen.

Subsequent to the determination of an expression profile (of expression profile data) of a set comprising at least two miRNAs representative for psoriasis as defined above in a blood cell sample from a patient (e.g. human or animal) in step (i) of the method for diagnosing and/or prognosing of psoriasis of the present invention, said method further comprises the step (ii) of comparing said expression profile (expression profile data) to a reference, wherein the comparison of said expression profile (expression profile data) to said reference allows for the diagnosis and/or prognosis of psoriasis.

The subject (e.g. human or another mammal (e.g. animal)) to be diagnosed with the method of the present invention may suffer, may be suspected to suffer or may not suffer from psoriasis. The subject (e.g. human or another mammal (e.g. animal)) to be diagnosed with the method of the present invention may suffer from a specific type of psoriasis. It is also possible to determine, whether the subject (e.g. human or another mammal (e.g. animal) to be diagnosed will develop psoriasis as the inventors of the present invention surprisingly found that miRNAs representative for psoriasis are already present in the blood cell sample before psoriasis occurs or during the early stage of psoriasis.

The reference may be the reference (e.g. reference expression profile (data)) of a healthy condition (i.e. not psoriasis), may be the reference (e.g. reference expression profile (data)) of a diseased condition (i.e. psoriasis) or may be the reference (e.g. reference expression profiles (data)) of at least two conditions from which at least one condition is a diseased condition (i.e. psoriasis). For example, (i) one condition may be a healthy condition (i.e. not psoriasis) and one condition may be a diseased condition (i.e. psoriasis), or (ii) one condition may be a diseased condition (e.g. a specific form of psoriasis) and one condition may be another diseased condition (i.e. specific form of psoriasis, or other timepoint oftreatement, other therapeutic treatment).

Further, the reference may be the reference expression profiles (data) of essentially the same, preferably the same, set of miRNAs of step (i), preferably in a body fluid sample originated from the same source as the blood cell sample from the subject (e.g. human or animal) to be tested, but obtained from subjects (e.g. human or animal) known to not suffer from psoriasis and from subjects (e.g. human or animal) known to suffer from psoriasis (e.g.psoriasis, e.g. psoriasis that has been therapeutically treated).

Preferably, both the reference expression profile and the expression profile of step (i) are determined in a blood cell sample (e.g. erythrocytes, leukocytes and/or thrombocytes). It is understood that the reference expression profile is not necessarily obtained from a single subject known to be affected by psoriasis or known to be not affected by psoriasis (e.g. healthy subject, such as healthy human or animal, or diseased subject, such as diseased human or animal) but may be an average reference expression profile of a plurality of subjects known to be affected by psoriasis or known to be not affected by psoriasis (e.g. healthy subjects, such as healthy humans or animals, or diseased subjects, such as diseased humans or animals), e.g. at least 2 to 200 subjects, more preferably at least 10 to 150 subjects, and most preferably at least 20 to 100 subjects, (e.g. healthy subject, such as healthy human or animal, or diseased subject, such as diseased human or animal). The expression profile and the reference expression profile may be obtained from a subject/patient of the same species (e.g. human or animal), or may be obtained from a subject/patient of a different species (e.g. human or animal). Preferably, the expression profile is obtained from a subject known to be affected by psoriasis or known to be not affected by psoriasis of the same species (e.g. human or animal), of the same gender (e.g. female or male) and/or of a similar age/phase of life (e.g. infant, young child, juvenile, adult) as the subject (e.g. human or animal) to be tested or diagnosed.

Thus, in a preferred embodiment of the method of the present invention, the reference is a reference expression profile (data) of at least one subject, preferably the reference is an average expression profile (data) of at least 2 to 200 subjects, more preferably of at least 10 to 150 subjects, and most preferably of at least 20 to 100 subjects, , with one known clinical condition which is psoriasis or a specific form of psoriasis, or which is not psoriasis or a specific form of psoriasis (i.e. healthy/healthiness), wherein the reference expression profile is the the profile of a set comprising at least two miRNAs that have nucleotide sequences that essentially correspond (are essentially identical), preferably that correspond (are identical), to the nucleotide sequences of the miRNAs of step (i). Preferably, the reference expression profile is the profile of a set comprising at least two miRNAs that have nucleotide sequences that essentially correspond (are essentially identical), preferably that correspond (are identical), to the nucleotide sequences of the miRNAs selected from the group consisting of SEQ ID NO: 1 to 111, a fragment thereof, and a sequence having at least 80% sequence identity thereto of step (i).

The comparison of the expression profile of the patient to be diagnosed (e.g. human or animal) to the (average) reference expression profile may then allow for diagnosing and/or prognosing of psoriasis or a specific form of psoriasis (step (ii)), either the subject/patient (e.g. human or animal) to be diagnosed is healthy, i.e. not suffering from psoriasis, or diseased, i.e. suffering from psoriasis or a specific form of psoriasis.

The comparison of the expression profile of the subject (e.g. human or animal) to be diagnosed to said reference expression profile(s) may then allow for the diagnosis and/or prognosis of psoriasis (step (ii)), either the subject(e.g. human or animal) to be diagnosed is healthy, i.e. not suffering from psoriasis, or the subject (e.g. human or animal) is diseased, i.e. suffering from psoriasis.

The comparison of the expression profile of the patient (e.g. human or animal) to be diagnosed to said reference expression profiles may then allow for the diagnosis/prognosis of a specific form of psoriasis (step (ii)), e.g. whether the patient to be diagnosed suffers from psoriasis.

In a particularly preferred embodiment of the method of the present invention, the reference is an algorithm or mathematical function. Preferably, the algorithm or mathematical function is obtained on the basis of reference expression profiles (data) of at least 2 to 200 subjects, more preferably of at least 10 to 150 subjects, and most preferably of at least 20 to 100 subjects, with two known clinical conditions from which one is psoriasis, wherein the reference expression profiles is the profile of a set comprising at least two miRNAs that have nucleotide sequences that essentially correspond (are essentially identical), preferably that correspond (are identical), to the nucleotide sequences of the miRNAs of step (i). Preferably, is the profile of a set comprising at least two miRNAs that have nucleotide sequences that essentially correspond (are essentially identical), preferably that correspond (are identical), to the nucleotide sequences of the miRNAs selected from the group consisting of SEQ ID NO: 1 to 111, a fragment thereof, and a sequence having at least 80% sequence identity thereto of step (i).

It is preferred that the algorithm or mathematical function is obtained using a machine learning approach.

Machine learning approaches may include but are not limited to supervised or unsupervised analysis: classification techniques (e.g. naive Bayes, Linear Discriminant Analysis, Quadratic Discriminant Analysis Neural Nets, Tree based approaches, Support Vector Machines, Nearest Neighbour Approaches), Regression techniques (e.g. linear Regression, Multiple Regression, logistic regression, probit regression, ordinal logistic regression ordinal Probit-Regression, Poisson Regression, negative binomial Regression, multinomial logistic Regression, truncated regression), Clustering techniques (e.g. k-means clustering, hierarchical clustering, PCA), Adaptations, extensions, and combinations of the previously mentioned approaches.

The inventors of the present invention surprisingly found that the application/use of a machine learning approach (e.g. t-test, AUC, support vector machine, hierarchical clustering, or k-means) leads to the obtainment of an algorithm or mathematical function that is trained by the reference expression profile(s) or reference expression profile data mentioned above (e.g. trained by the miRNA reference expression profile (data) of a diseased condition (i.e. psoriasis or a specific form of psoriasis), for example, obtained from subjects (e.g. humans or animals) known to suffer from psoriasis or from a specific form of psoriasis (i.e. being diseased) and/or a trained by the miRNA reference expression profile (data) of a healthy condition (i.e. not psoriasis or a specific form of psoriasis), for example, obtained from subjects (e.g. humans or animals) known to not suffer from psoriasis or from a specific form of psoriasis and that this allows a better (i) discrimination between the at least two (e.g. 2 or 3) clinical conditions (the at least two statistical classes, e.g. the two conditions healthy or suffering from psoriasis or the two clinical conditions suffering from a specific form of psoriasis or suffering from another specific form of psoriasis or at least three clinical conditions, e.g. the three clinical conditions healthy, suffering from a specific form of psoriasis or suffering from another specific form of psoriasis or (ii) decision whether the at least one clinical condition (the one condition healthy or suffering from psoriasis is present. In this way, the performance for diagnosing/prognosing of individuals suffering from psoriasis can be increased (see also experimental section for details).

Thus, in a preferred embodiment of the method of the present invention, the algorithm or mathematical function is obtained using a machine learning approach, wherein said algorithm or mathematical function is trained by a reference expression profile (data) of at least 2 to 200 subjects, more preferably of at least 10 to 150 subjects, and most preferably of at least 20 to 100 subjects with two known clinical condition for which one is psoriasis or a specific form of psoriasis, wherein the reference expression profile is the profile of a set comprising at least two miRNAs that have nucleotide sequences that essentially correspond (are essentially identical), preferably that correspond (are identical), to the nucleotide sequences of the miRNAs of step (i), preferably to decide whether the at least one clinical condition which is psoriasis or a specific form of psoriasis.

Further, for instance, the machine learning approach may be applied to the reference expression profiles (data) of a set comprising at least 2 miRNAs (e.g. 10 miRNAs such as miRNAs according to SEQ ID NO: 1 to 10) of at least one subject (e.g. human or animal) known to suffer from psoriasis and of at least one subject (e.g. human or animal) known to be healthy and may led to the obtainment of an algorithm or mathematical function. This algorithm or mathematical function may then be applied to a miRNA expression profile of the same at least 2 miRNAs as mentioned above (e.g. 10 miRNAs such as miRNAs according to SEQ ID NO: 1 to 10) of a subject (e.g. human or animal) to be diagnosed for psoriasis and, thus, may then allow to discriminate whether the subject (e.g. human or animal) tested is healthy, i.e. not suffering from psoriasis, or diseased, i.e. suffering from psoriasis.

Additionally the algorithm may be trained to discriminate between more than 2 (e.g. 3, 4, 5 or more) clinical conditions from which at least one is psoriasis.

Preferably, the reference and optionally the expression profile (data) of the miRNA(s) representative for psoriasis is (are) stored in a database, e.g. an internet database, a centralized, and/or a decentralized database. It is preferred that the reference, e.g. mathematical function or algorithm, is comprised in a computer program, e.g. saved on a data carrier.

The above mentioned method is for diagnosing psoriasis in a subject, e.g. a human or another mammal such as an animal. Preferably, the diagnosis comprises (i) determining the occurrence/presence of psoriasis, (ii) monitoring the course of psoriasis, (iii) staging of psoriasis, (iv) measuring the response of a patient with psoriasis to therapeutic intervention, and/or (v) segmentation of a subject suffering from psoriasis.

Further, the above mentioned method is for prognosis of psoriasis in a subject, a human or another mammal such as an animal. Preferably, the prognosis comprises (i) identifying of a subject who has a risk to develop psoriasis, (ii) predicting/estimating the occurrence, preferably the severity of occurrence of psoriasis, and/or(iii) predicting the response of a subject with psoriasis to therapeutic intervention.

Further, in a preferred embodiment of the method of the present invention, for determining an expression profile of the set comprising at least two miRNAs representative for psoriasis in a blood cell sample from a subject comprises a set of miRNAs listed in Figure 2, which comprises SEQ ID NO: 2.

For example, said set comprising 30 miRNAs representative for psoriasis in a blood cell sample from a subject comprises a set of miRNAs listed in Figure 2, which comprises SEQ ID NO: 2. Alternatively, said set comprising 29, 28, 27 ,26, 25, 24, 23, 22, 21, 20, 19, 18, 17, 16, 15, 14, 13, 12, 11, 10, 9, 8, 7, 6, 5, 4 or 3 miRNAs comprises a set of miRNAs listed in Figure 2, which comprises SEQ ID NO: 2.

For example, said set comprising 30 miRNAs representative for psoriasis in a blood cell sample from a subject comprises a set of miRNAs listed in Figure 2, which comprises SEQ ID NO: 2. For example, said set comprising 25 miRNAs representative for psoriasis in a blood cell sample from a subject comprises a set of miRNAs listed in Figure 2, which comprises SEQ ID NO: 2. For example, said set comprising 20 miRNAs representative for psoriasis in a blood cell sample from a subject comprises a set of miRNAs listed in Figure 2, which comprises SEQ ID NO: 2. For example, said set comprising 15 miRNAs representative for psoriasis in a blood cell sample from a subject comprises a set of miRNAs listed in Figure 2, which comprises SEQ ID NO: 2. For example, said set comprising 10 miRNAs representative for psoriasis in a blood cell sample from a subject comprises a set of miRNAs listed in Figure 2, which comprises SEQ ID NO: 2. For example, said set comprising 5 miRNAs representative for psoriasis in a blood cell sample from a subject comprises a set of miRNAs listed in Figure 2, which comprises SEQ ID NO: 2.

Further, in another preferred embodiment of the method of the present invention, for determining an expression profile of the set comprising at least two miRNAs representative for psoriasis in a blood cell sample from a subject comprises combinations of sets of miRNAs listed in Figure 2. For example, said set comprising 30 miRNAs representative for psoriasis in a blood cell sample from a subject comprises at least 2 sets of miRNAs listed in Figure 2. Alternatively, said set comprising 29, 28, 27 ,26, 25, 24, 23, 22, 21, 20, 19, 18, 17, 16, 15, 14, 13, 12, 11, 10, 9, 8, 7, 6, 5 or 4 miRNAs comprises a least 2 sets of miRNAs listed in Figure 2.

For example, said set comprising 30 miRNAs representative for psoriasis in a blood cell sample from a subject comprises a least 2 sets of miRNAs listed in Figure 2. For example, said set comprising 25 miRNAs representative for psoriasis in a blood cell sample from a subject comprises a least 2 sets of miRNAs listed in Figure 2. For example, said set comprising 20 miRNAs representative for psoriasis in a blood cell sample from a subject comprises a least 2 sets of miRNAs listed in Figure 2. For example, said set comprising 15 miRNAs representative for psoriasis in a blood cell sample from a subject comprises a least 2 sets of miRNAs listed in Figure 2. For example, said set comprising 10 miRNAs representative for psoriasis in a blood cell sample from a subject comprises a least 2 sets of miRNAs listed in Figure 2. For example, said set comprising 5 miRNAs representative for psoriasis in a blood cell sample from a subject comprises a least 2 sets of miRNAs listed in Figure 2.

Said set of miRNAs comprises at least one of the sets listed in Figure 2, which comprise SEQ ID NO: 2.

In a second aspect, the invention relates to the use of a set comprising polynucleotides for detecting a set comprising at least two miRNAs for diagnosing and/or prognosing of psoriasis in a blood sample from a subject, wherein the nucleotide sequences of the miRNAs comprised in the set are selected from the group consisting of SEQ ID NO: 1 to 111, and wherein the nucleotide sequence of a first of said at least two miRNAs is SEQ ID NO: 2, and wherein the blood sample is a blood cell sample.

Preferably, the blood cell sample is a leukocyte-, erythrocyte- and/or platelet-containing sample.

It is further preferred that the blood sample has been collected under conditions where the RNA-fraction is guarded against degradation, preferably the blood sample is collected in a PAXgene (RNA) Tube.

It is preferred that the subject is a mammal including both a human and another mammal, e.g. an animal such as a mouse, a rat, a rabbit, or a monkey. It is particularly preferred that the subject is a human.

Preferably, the set comprising at least two miRNAs comprises at least one up-regulated miRNA listed in Figure 4a or comprises at least one down-regulated miRNAs listed in Figure 5a. More preferably, the set comprising at least two miRNAs comprises at least one up-regulated miRNA listed in Figure 4b or comprises at least one down-regulated miRNAs listed in Figure 5b. It is further preferred, that the set comprising at least two miRNAs comprises at least one up-regulated miRNA listed in Figure 4a and further comprises at least one down-regulated miRNAs listed in Figure 5a.

It is preferred that the set comprising at least two miRNAs is selected from the sets of miRNAs listed in Figure 2, which comprise SEQ ID NO: 2 or that the set comprising at least two miRNAs comprises the sets of miRNAs listed in Figure 2. The set of miRNAs comprises at least one of the sets of miRNAs listed in Figure 2, which comprise SEQ ID NO: 2.

It is preferred that
(i) the polynucleotides comprised in the set used in the present invention are complementary to the miRNAs comprised in the set, wherein the nucleotide sequences of said miRNAs are selected from the group consisting of SEQ ID NO: 1 to 111,
(ii) the polynucleotides comprised in the set are fragments of the polynucleotides comprised in the set according to (i), preferably the polynucleotides comprised in the set are fragments which are between 1 and 12, more preferably between 1 and 8, and most preferably between 1 and 5 or 1 and 3, i.e. 1, 2, 3, 4, 5, 6, 7, 8, 9, 10, 11, or 12, nucleotides shorter than the polynucleotides comprised in the set according to (i), or
(iii) the polynucleotides comprised in the set have at least 80%, preferably at least 85%, more preferably at least 90%, and most preferably at least 95% or 99%, i.e. 80, 81, 82, 83, 84, 85, 86, 87, 88, 89, 90, 91, 92, 93, 94, 95, 96, 97, 98, or 99%, sequence identity to the polynucleotide sequences of the polynucleotides comprised in the set according to (i) or polynucleotide fragments comprised in the set according to (ii).

It is preferred that the polynucleotides used in the present invention are for detecting a set comprising, essentially consisting of, or consisting of at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 35, 40 or more miRNAs, or comprising/consisting of 111 miRNAs and wherein the nucleotide sequences of said miRNAs are selected from the group consisting of SEQ ID NO: 1 to 111.

It is preferred that the polynucleotides used in the present invention are for detecting a set comprising, essentially consisting of, or consisting of at least 2 miRNAs, wherein the set comprising, miRNAs is selected from the set listed in Figure 2.

It is preferred that the polynucleotides used in the present invention are for detecting a set comprising, essentially consisting of, or consisting of at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 35, 40 or more miRNAs, or comprising/consisting of 111 miRNAs and wherein the set of miRNAs comprises at least one of the sets listed in Figure 2.

At least one of the miRNA sets described above comprises SEQ ID NO: 2.

For the blood cell sample analysis, it may be required that a set of polynucleotides (probes) capable of detecting a fixed defined set of miRNAs are attached to a solid support, bead, substrate, surface, platform, or matrix, e.g. biochip, which may be used for body fluid sample (e.g. blood sample) analysis. For example, if the fixed defined set of miRNAs for diagnosing psoriasis comprises or consists of 20 miRNAs, polynucleotides capable of detecting these 20 miRNAs are attached to a solid support, substrate, surface, platform or matrix, e.g. biochip, in order to perform the diagnostic sample analysis.

Alternatively, it may be required that a set of chimeric polynucleotides (probes) capable of detecting a fixed defined set of miRNAs it contacted in solution with a sample containing miRNAs derived from a blood cell sample. The chimeric polynucleotide may comprise of a first sequence stretch that is complementary to a miRNA and a second sequence stretch that allows to pull down the chimeric polynucleotide-miRNA-duplexes to one or more solid supports (e.g. a set of beads for determining the set of miRNAs). For example, a set of 20 chimeric polynucleotides capable of detecting 20 miRNAs are contacted with sample containing miRNAs derived from a blood cell sample in order to form duplexes that can be pulled down to 20 different species of beads and detected theron.

For example, the polynucleotides used in the present invention are for detecting a set of 40 or 39 or 38 or 37 or 36 or 35 or 34 or 33 or 32 or 31 or 30 or 29 or 28 or 27 or 26 or 25 or 24 or 23 or 22 or 21 or 20 or 19 or 18 or 17 or 16 or 15 or 14 or 13 or 12 or 11 or 10 or 9 or 8 or 7 or 6 or 5 or 4 or 3 miRNAs wherein the set of miRNAs comprises at least one of the sets of miRNAs listed in Figure 2. The set of miRNAs comprises at least one of the sets of miRNAs listed in Figure 2, which comprise SEQ ID NO: 2.

For example, the polynucleotides used in present invention are for detecting a set of 30 miRNAs wherein the set of miRNAs comprises at least one of the sets of miRNAs listed in Figure 2, which comprise SEQ ID NO: 2.

For example, the polynucleotides used in present invention are for detecting a set of 25 miRNAs wherein the set of miRNAs comprises at least one of the sets of miRNAs listed in Figure 2, which comprise SEQ ID NO: 2.

For example, the polynucleotides used in present invention are for detecting a set of 20 miRNAs wherein the set of miRNAs comprises at least one of the sets of miRNAs listed in Figure 2, which comprise SEQ ID NO: 2.

For example, the polynucleotides used in present invention are for detecting a set of 15 miRNAs wherein the set of miRNAs comprises at least one of the sets of miRNAs listed in Figure 2, which comprise SEQ ID NO: 2.

For example, the polynucleotides used in present invention are for detecting a set of 10 miRNAs wherein the set of miRNAs comprises at least one of the sets of miRNAs listed in Figure 2, which comprise SEQ ID NO: 2.

For example, the polynucleotides used in present invention are for detecting a set of 5 miRNAs wherein the set of miRNAs comprises at least one of the sets of miRNAs listed in Figure 2, which comprise SEQ ID NO: 2.

Also described herein but not encompassed by the present invention is a set of at least two primer pairs for determining the expression level of a set of miRNAs in a body fluid sample of a subject suffering or suspected of suffering from psoriasis.

It is preferred that the body fluid sample is a blood sample, particularly preferred it is a whole blood, PBMC, serum, plasma or leukocyte sample, more particularly preferred it is a blood cell sample, preferably a leukocyte- , erythrocyte and/or platelet-containing sample..

It is further preferred that the body fluid sample is a blood sample that has been collected under conditions where the RNA-fraction is guarded against degradation, preferably the blood sample is collected in a PAXgene (RNA) Tube.

It is preferred that the subject is a mammal including both a human and another mammal, e.g. an animal such as a mouse, a rat, a rabbit, or a monkey. It is particularly preferred that the subject is a human.

Preferably, that the set comprising at least two miRNAs for diagnosing and/or prognosing of psoriasis in a body fluid sample, e.g. blood sample, from a subject, e.g. patient, human or animal, are selected from the group consisting of SEQ ID NO: 1 to 111 or SEQ ID NO: 1 to 283.

Preferably, the set comprising at least two miRNAs comprises at least one up-regulated miRNA listed in Figure 4a or comprises at least one down-regulated miRNAs listed in Figure 5a. More preferably, the set comprising at least two miRNAs comprises at least one up-regulated miRNA listed in Figure 4b or comprises at least one down-regulated miRNAs listed in Figure 5b. It is further preferred, that the set comprising at least two miRNAs comprises at least one up-regulated miRNA listed in Figure 4a and further comprises at least one down-regulated miRNAs listed in Figure 5a.

It is preferred that the set comprising at least two miRNAs is selected from or comprises the sets of miRNAs listed in Figure 2.

It is preferred that the set of at least two primer pairs for determining the expression level of a set of miRNAs in a body fluid sample of a subject suffering or suspected of suffering from psoriasis are primer pairs that are specific for at least one miRNA selected from the group consisting of SEQD ID NO:1 to 111 or SEQD ID NO: 1 to 283.

It is preferred that the set of at least two primer pairs for determining the expression level of a set of miRNAs in a body fluid sample of a subject suffering or suspected of suffering from psoriasis are primer pairs that are specific for at least one set of miRNAs listed in Figure 2.

It is preferred that the set of at least two primer pairs are for detecting a set comprising, essentially consisting of, or consisting of at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 35, 36, 37, 38, 39, 40 or more miRNAs, or comprising/consisting of 111 miRNAs and wherein the nucleotide sequences of said miRNAs are selected from the group consisting of SEQ ID NO: 1 to 111 or SEQ ID NO: 1 to 283.

It is preferred that the set of at least two primer pairs are for detecting a set comprising, essentially consisting of, or consisting of at least 2 miRNAs, wherein the set comprising, miRNAs is selected from the set listed in Figure 2.

It is preferred that the set of at least two primer pairs are for detecting a set comprising, essentially consisting of, or consisting of at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 35, 36, 37, 38, 39, 40 or more miRNAs, or comprising/consisting of 111 miRNAs and wherein the set of miRNAs comprises at least one of the sets listed in Figure 2.

For example, the set of at least two primer pairs are for detecting a set of 40 or 39 or 38 or 37 or 36 or 35 or 34 or 33 or 32 or 31 or 30 or 29 or 28 or 27 or 26 or 25 or 24 or 23 or 22 or 21 or 20 or 19 or 18 or 17 or 16 or 15 or 14 or 13 or 12 or 11 or 10 or 9 or 8 or 7 or 6 or 5 or 4 or 3 or 2 miRNAs wherein the set of miRNAs comprises at least one of the set of miRNAs listed in Figure 2.

For example, the set of primer pairs are for detecting a set of 30 miRNAs wherein the set of miRNAs comprises at least one of the sets of miRNAs listed in Figure 2. For example, the set of primer pairs are for detecting a set of 25 miRNAs wherein the set of miRNAs comprises at least one of the sets of miRNAs listed in Figure 2. For example, the set of primer pairs are for detecting a set of 20 miRNAs wherein the set of miRNAs comprises at least one of the sets of miRNAs listed in Figure 2. For example, the set of primer pairs are for detecting a set of 15 miRNAs wherein the set of miRNAs comprises at least one of the sets of miRNAs listed in Figure 2. For example, the set of primer pairs are for detecting a set of 10 miRNAs wherein the set of miRNAs comprises at least one of the sets of miRNAs listed in Figure 2.

Preferably, the said primer pairs may be used for amplifying cDNA transcripts of the set of miRNAs selected from the group consisting of SEQ ID 1 to 111 or SEQ ID NO: 1 to 283. Furthermore, the said primer pairs may be used for amplifying cDNA transcripts of the set of miRNAs listed in Figure 2

It is understood that the primer pairs for detecting a set of miRNAs may consist of specific and or non-specific primers. Additionally, the set of primer pairs may be complemented by other substances or reagents (e.g. buffers, enzymes, dye, labelled probes) known to the skilled in the art for conducting real time polymerase chain reaction (RT-PCR)

Also described herein but not encompassed by the present invention is the use of a set of primer pairs as described above for diagnosing and/or prognosing psoriasis in a subject.

Also described herein but not encompassed by the present invention are means for diagnosing and/or prognosing of psoriasis in a body fluid sample of a subject.

Preferably, the means for diagnosing and/or prognosing of psoriasis in a body fluid sample of a subject comprises
(i) a set of at least two polynucleotides used according to the second aspect of the invention or
(ii) a set of at least two primer pairs as described above.

It is preferred that the body fluid sample is a blood sample, particularly preferred it is a whole blood, PBMC, serum, plasma or leukocyte sample, more particularly preferred it is a blood cell sample, preferably a leukocyte- , erythrocyte and/or platelet-containing sample..

It is further preferred that the body fluid sample is a blood sample that has been collected under conditions where the RNA-fraction is guarded against degradation, preferably the blood sample is collected in a PAXgene (RNA) Tube.

It is preferred that the subject is a mammal including both a human and another mammal, e.g. an animal such as a mouse, a rat, a rabbit, or a monkey. It is particularly preferred that the subject is a human.

Preferably, that the set of at least two polynucleotides or the set of at least 2 primer pairs are for detecting a set comprising at least two miRNAs for diagnosing and/or prognosing of psoriasis in a body fluid sample, e.g. blood sample, from a subject, e.g. patient, human or animal, wherein the set of miRNAs is selected from the group consisting of SEQ ID NO: 1 to 111 or SEQ ID NO: 1 to 283.

Preferably, the set comprising at least two miRNAs comprises at least one up-regulated miRNA listed in Figure 4a or comprises at least one down-regulated miRNAs listed in Figure 5a. More preferably, the set comprising at least two miRNAs comprises at least one up-regulated miRNA listed in Figure 4b or comprises at least one down-regulated miRNAs listed in Figure 5b. It is further preferred, that the set comprising at least two miRNAs comprises at least one up-regulated miRNA listed in Figure 4a and further comprises at least one down-regulated miRNAs listed in Figure 5a.

It is preferred that the set of at least two polynucleotides or the set of at least 2 primer pairs are for detecting a set comprising at least two miRNAs for diagnosing and/or prognosing of psoriasis in a body fluid sample, e.g. blood sample, from a subject, e.g. patient, human or animal, wherein the set of miRNAs is selected from or comprises the sets of miRNAs listed in Figure 2.

It is preferred that the set of at least two primer pairs for determining the expression level of a set of miRNAs in a body fluid sample of a subject suffering or suspected of suffering from psoriasis are primer pairs that are specific for at least two miRNAs selected from the group consisting of SEQ ID NO: 1 to 111 or SEQ ID NO: 1 to 283..

It is preferred that the set of at least two primer pairs for determining the expression level of a set of miRNAs in a body fluid sample of a subject suffering or suspected of suffering from psoriasis are primer pairs that are specific for at least one set of miRNAs listed in Figure 2.

It is preferred that the subject is a mammal including both a human and another mammal, e.g. an animal such as a mouse, a rat, a rabbit, or a monkey. It is particularly preferred that the subject is a human.

The means for diagnosing and/or prognosing of psoriasis comprising a set comprising, essentially consisting of, or consisting of at least two polynucleotides (probes) used according to the second aspect of the present invention, e.g. a polynucleotide for detecting a set comprising, essentially consisting of, or consisting of at least 2 polynucleotides, preferably comprising, essentially consisting of, or consisting of at least 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40 or up to 111 or more polynucleotides for detecting a set comprising, essentially consisting of, or consisting of at least 2 miRNAs, preferably comprising, essentially consisting of, or consisting of at least 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39 or 40 or 111 miRNAs or all known miRNAs, wherein the nucleotide sequence of said miRNAs are preferably selected from the group consisting of SEQ ID NO: 1 to 111 or SEQ ID NO: 1 to 283 , a fragment thereof, and a sequence having at least 80% sequence identity thereto.

The means for diagnosing and/or prognosing of psoriasis comprises, essentially consists of, or consists of a solid support, substrate, surface, platform or matrix comprising a set comprising, essentially consisting of, or consisting of at least two polynucleotides (probes) used according to the second aspect of the present invention, e.g. a solid support, substrate, surface, platform or matrix comprising at least 2 polynucleotides, preferably comprising, essentially consisting of, or consisting of at least 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40 or more polynucleotides, or comprising/consisting of 111 polynucleotides for detecting a set comprising, essentially consisting of, or consisting of at least 2 miRNAs, preferably comprising, essentially consisting of, or consisting of at least 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40 or more miRNAs, or comprising/consisting of 111 miRNAs, wherein the nucleotide sequence said miRNAs are preferably selected from the group consisting of SEQ ID NO: 1 to 111 or SEQ ID NO: 1 to 283, a fragment thereof, and a sequence having at least 80% sequence identity thereto. Preferably, the above mentioned polynucleotide(s) is (are) attached or immobilized to the solid support, substrate, surface, platform or matrix. It is possible to include appropriate controls for non-specific hybridization on the solid support, substrate, surface, platform or matrix.

Additionally, the means for diagnosing and/or prognosing of psoriasis comprises, essentially consists of, or consists of a solid support, substrate, surface, platform or matrix comprising a set comprising, essentially consisting of, or consisting of at least two polynucleotides (probes) used according to the second aspect of the present invention, e.g. a solid support, substrate, surface, platform or matrix comprising at least 2 polynucleotides, preferably comprising, essentially consisting of, or consisting of at least 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40 or more polynucleotides, or comprising/consisting of 111 polynucleotides for detecting a set comprising, essentially consisting of, or consisting of at least 2 miRNAs, preferably comprising, essentially consisting of, or consisting of at least 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40 or more miRNAs, or comprising/consisting of 111 miRNAs, wherein the set of miRNAs comprises at least one set of miRNAs listed in Figure 2. Preferably, the above mentioned polynucleotides are attached or immobilized to the solid support, substrate, surface, platform or matrix. It is possible to include appropriate controls for non-specific hybridization on the solid support, substrate, surface, platform or matrix.

It is particularly preferred that said means for diagnosing and/or prognosing of psoriasis comprise, essentially consists of, or consists of a microarray/biochip comprising at least two polynucleotides used according to the second aspect of the present invention.

It is also preferred that said means for diagnosing and/or prognosing of psoriasis comprise, essentially consists of, or consists of a set of beads comprising a at least two polynucleotides used according to the second aspect of the present invention. It is especially preferred that the beads are employed within a flow cytometer setup or a setup for analysing magnetic beads for diagnosing and/or prognosing of psoriasis, e.g. in a LUMINEX system (*www.luminexcorp.com)*

Additionally, the means for diagnosing and/or prognosing of psoriasis comprises a set comprising, essentially consisting of, or consisting of at least two primer pairs as described above, e.g. of at least 2 primer pairs, preferably comprising, essentially consisting of, or consisting of at least 3, 4, 5, 6, 7, 8, 9, 10, 1l, 12, 13, 14, 15, 16, 17, 18, 19, 20, 21, 22, 23, 24, 25, 26, 27, 28, 29, 30, 31, 32, 33, 34, 35, 36 ,37 ,38 ,39, 40 or up to 111 or up to 283 or more primer pairs for detecting a set comprising, essentially consisting of, or consisting of at least 2 miRNAs, preferably comprising, essentially consisting of, or consisting of at least 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40 or 111 or 283 miRNAs or all known miRNAs, wherein the nucleotide sequence of said miRNA or the nucleotide sequences of said miRNAs is (are) preferably selected from the group consisting of SEQ ID NO: 1 to 111 or SEQ ID NO: 1 to 283 , a fragment thereof, and a sequence having at least 80% sequence identity thereto.

Also, the means for diagnosing and/or prognosing of psoriasis comprises a set comprising, essentially consisting of, or consisting of at least two primer pairs as described above, e.g. of at least 2 primer pairs, preferably comprising, essentially consisting of, or consisting of at least 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36 ,37 ,38 ,39, 40 or up to 111 or up to 283 or more primer pairs for detecting a set comprising, essentially consisting of, or consisting of at least 2 miRNAs, preferably comprising, essentially consisting of, or consisting of at least 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40 or 111 or 283 miRNAs or all known miRNAs, wherein the set of miRNAs comprises at least one set of miRNAs listed in Figure 2.

In a third aspect, the invention relates to the use of a kit for diagnosing and/or prognosing of psoriasis comprising
(i) means for determining an expression profile of a set comprising at least two miRNAs representative for psoriasis in a blood sample from a subject, and
(ii) at least one reference
wherein the nucleotide sequences of said miRNAs are selected from the group consisting of SEQ ID NO: 1 to 111, a fragment thereof, and a sequence having at least 80% sequence identity thereto, and
wherein the nucleotide sequence of a first of said at least two miRNAs is SEQ ID NO: 2, and
wherein the blood sample is a blood cell sample, and
wherein the reference is generated from reference expression profiles of at least two control subjects of at least two clinical conditions from which at least one is psoriasis determined in the same type of blood sample as the subject to be diagnosed and/or prognosed.

Preferably, the blood cell sample is a leukocyte- , erythrocyte- and/or platelet-containing sample.

It is further preferred that the blood sample has been collected under conditions where the RNA-fraction is guarded against degradation, preferably the blood sample is collected in a PAXgene (RNA) Tube.

It is preferred that the subject is a mammal including both a human and another mammal, e.g. an animal such as a mouse, a rat, a rabbit, or a monkey. It is particularly preferred that the subject is a human.

Also described herein is a kit for diagnosing and/or prognosing of psoriasis comprising
(i) means for determining an expression profile of a a set comprising, essentially consisting of, or consisting of at least two miRNAs (e.g. human miRNAs or miRNAs from another mammal such as an animal (e.g. mouse miRNA or rat miRNAs)), preferably comprising, essentially consisting of, or consisting of at least 2 or up to 111 or up to 283 or more polynucleotides or alternatively a set of at least 2 or up to 111 or up to 283 or more primer pairs for detecting a set comprising, essentially consisting of, or consisting of at least 2 miRNAs, preferably comprising, essentially consisting of, or consisting of at least 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36 ,37, 38 ,39, 40 or more or 111 or 283 miRNAs or all known miRNAs, representative for psoriasis in a biological sample (e.g. a body fluid samples or a blood sample) from a subject (e.g. human or animal), wherein the nucleotide sequence of said miRNA or the nucleotide sequences of said miRNAs is (are) preferably selected from the group consisting of SEQ ID NO: 1 to 111 or SEQ ID NO: 1 to 283, a fragment thereof, and a sequence having at least 80% sequence identity thereto; and
(ii) at least one reference.

Also described herein is a kit for diagnosing and/or prognosing of psoriasis comprising
(i) means for determining an expression profile of a a set comprising, essentially consisting of, or consisting of at least two miRNAs (e.g. human miRNAs or miRNAs from another mammal such as an animal (e.g. mouse miRNA or rat miRNAs)), preferably comprising, essentially consisting of, or consisting of at least 2 or up to 111 or up to 283 or more polynucleotides or alternatively a set of at least 2 or up to 111 or up to 283 or more primer pairs for detecting a set comprising, essentially consisting of, or consisting of at least 2 miRNAs, preferably comprising, essentially consisting of, or consisting of at least 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36 ,37, 38 ,39, 40 or more or 111 or 283 miRNAs or all known miRNAs, representative for psoriasis in a biological sample (e.g. a body fluid samples or a blood sample) from a subject (e.g. human or animal), wherein the set of miRNAs comprises at least one of the set of miRNAs listed in Figure 2.
(ii) at least one reference.

Said means may comprise a set comprising, essentially consisting of, or consisting of at least two polynucleotides used according to the second aspect of the present invention, a set of at least 2 primer pairs as described above; means as described above; primers suitable to perform reverse transcriptase reaction and/or real time polymerase chain reaction such as quantitative polymerase chain reaction; and/or means for conducting next generation sequencing.

It is particularly preferred that the kit used comprises
(ia) a set comprising, essentially consisting of, or consisting of at least two polynucleotides used according to the second aspect of the present invention, or a set of primer pairs as described above and
(ib) optionally at least one of the means selected from the group consisting of: at least one blood cell sample of a subject (e.g. human or animal), at least one sample of total RNA extracted from said blood cell sample of a patient (e.g. human or animal), and means to extract RNA from a blood cell sample for determining an expression profile of a set comprising, essentially consisting of, or consisting of at least two miRNAs representative for psoriasis in a blood cell sample from a patient (e.g. human or animal), wherein the nucleotide sequences of said miRNAs are selected from the group consisting of SEQ ID NO: 1 to 111, a fragment thereof, and a sequence having at least 80% sequence identity thereto, wherein the nucleotide sequence of a first of said at least two miRNAs is SEQ ID NO: 2.

It is more particularly preferred that the kit used comprises
(ia) a solid support, substrate, surface, platform or matrix (e.g a microarray of a set of beads) as described above comprising a set comprising, essentially consisting of, or consisting of at least two polynucleotides used according of the second aspect of the present invention, and
(ib) optionally at least one of the means selected from the group consisting of: at least one blood cell sample from a patient (e.g. human or animal), at least one sample of total RNA (or fractions thereof, e.g. miRNA) extracted from a blood cell sample from a patient (e.g. human or animal), means to extract total RNA (or fractions thereof, e.g. miRNA) from a blood cell sample, means for input/injection of a blood cell sample, positive controls for the hybridization experiment, means for holding the solid support, substrate, platform or matrix comprising the polynucleotides (probes), means for labelling the isolated miRNA (e.g. NTP/biotin-NTP), means for hybridization, means to carry out enzymatic reactions (e.g. exonuclease I and/or Klenow enzyme) means for washing steps, means for detecting the hybridization signal, and mean for analysing the detected hybridization signal,. for determining an expression profile of a set comprising, essentially consisting of, or consisting of at least two miRNAs representative for psoriasis in a blood cell sample from a patient (e.g. human or animal), wherein the nucleotide sequences of said miRNAs are selected from the group consisting of SEQ ID NO: 1 to 111, a fragment thereof, and a sequence having at least 80% sequence identity thereto, wherein the nucleotide sequence of a first of said at least two miRNAs is SEQ ID NO: 2.

Preferably, the above mentioned set comprising, essentially consisting of, or consisting of at least two polynucleotides are attached or immobilized to the solid support, substrate, surface, platform or matrix, e.g. to a microarray or to a set of beads.

Preferably, the above mentioned set comprising, essentially consisting of, or consisting of at least two polynucleotides is (are) attached or immobilized to microarray/biochip.

It is particularly preferred that the kit used comprises
(ia) at least two miRNA-specific primers for reverse transcription of miRNAs in miRNA-specific cDNAs for at least 2 miRNAs (e.g. human miRNAs or miRNAs from another mammal such as an animal (e.g. mouse or rat miRNAs)), preferably for at least 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40 or more, or 111 miRNAs (e.g. human miRNAs or miRNAs from another mammal such as an animal (e.g. mouse or rat miRNAs)), comprised in a set of miRNAs, wherein the nucleotide sequences of said miRNAs are selected from the group consisting of SEQ ID NO: 1 to 111, wherein the nucleotide sequence of a first of said at least 2 miRNAs is SEQ ID NO: 2, and
(ib) preferably, at least two primer sets comprising a forward primer which is specific for the single cDNA obtained from the miRNA and an universal reverse primer for amplifying the cDNA obtained from the miRNA via real time polymerase chain reaction (RT-PCR) such as real time quantitative polymerase chain reaction (RT qPCR) for at least 2, preferably for at least 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40 or more or 111 cDNAs obtained from the miRNAs comprised in the set of miRNAs, wherein preferably said cDNAs are complementary to the nucleotide sequences of the miRNAs selected from the group consisting of SEQ ID NO: 1 to 111, wherein the nucleotide sequence of a first of said at least 2 miRNAs is SEQ ID NO: 2, and
(ic) optionally at least one of the means selected from the group consisting of: at least one blood cell sample from a patient (e.g. human or animal), at least one sample of total RNA (or fractions thereof, e.g. miRNA) extracted from a blood cell sample form a patient (e.g. human or animal), means to extract total RNA (or fractions thereof, e.g. miRNA) from a blood cell sample, additional means to carry out the reverse transcriptase reaction (miRNA in cDNA) (e.g. reverse transcriptase (RT) enzyme, puffers, dNTPs, RNAse inhibitor), additional means to carry out real time polymerase chain reaction (RT-PCR) such as real time quantitative PCR (RT qPCR) (e.g. enzymes, puffers, water), means for labelling (e.g. fluorescent label and/or quencher), positive controls for reverse transcriptase reaction and real time PCR, and means for analysing the real time polymerase chain reaction (RT-PCR) result for determining an expression profile of a set comprising, essentially consisting of, or consisting of at least 2, preferably comprising, essentially consisting of, or consisting of at least 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50 or more , or 111 miRNAs representative for psoriasis in a blood cell sample from a patient (e.g. human or animal), wherein the nucleotide sequences of said miRNAs are selected from the group consisting of SEQ ID NO: 1 to 111, a fragment thereof, and a sequence having at least 80% sequence identity thereto, wherein the nucleotide sequence of a first of said at least 2 miRNAs is SEQ ID NO: 2.

The primer as defined above may also be an oligo-dT primer, e.g. if the miRNA comprises a polyA tail (e.g. as a result of a miRNA elongation, for example, subsequent to RNA extraction) or a miRNA specific looped RT primer (Please amend/adapted if required).

It is also preferred that the kit used comprises means for conducting next generation sequencing in order to determine an expression profile of a set comprising, essentially consisting of, or consisting of at least 2 miRNAs representative for psoriasis in a blood cell sample from a patient (e.g. human or animal), wherein the nucleotide sequences of said miRNAs are selected from the group consisting of SEQ ID NO: 1 to 111, a fragment thereof, and a sequence having at least 80% sequence identity thereto, wherein the nucleotide sequence of a first of said at least 2 miRNAs is SEQ ID NO: 2. Preferably, the kit used further comprises means selected from the group consisting of: at least one blood cell sample from a patient (e.g. human or animal), at least one sample of total RNA (or fractions thereof, e.g. miRNA) extracted from the blood cell sample of a patient (e.g. human or animal), and means to extract total RNA (or fractions thereof, e.g. miRNA) from a blood cell sample.

The above mentioned kits further comprise at least one reference (ii). A comparison to said reference may allow for the diagnosis and/or prognosis of psoriasis. Said reference is generated from expression profiles of at least two control subjects of at least two clinical conditions from which at least one is psoriasis determined in the same type of blood sample as the subject to be diagnosed and/or prognosed.

Also described is a reference which is a reference expression profile (data) of at least one subject (e.g. human or animal), preferably the reference is an average expression profile (data) of at least 2 to 200 subjects, more preferably at least 10 to 150 subjects, and most preferably at least 20 to 100 subjects, with one known clinical condition which is psoriasis or a specific form of psoriasis, or which is not psoriasis or not a specific form of psoriasis (i.e. healthy/healthiness), wherein the reference expression profile of a set comprising at least two miRNAs that have nucleotide sequences that essentially correspond (are essentially identical), preferably that correspond (are identical), to the nucleotide sequences of the miRNAs which expression profile is determined by the means of (i).

It is also preferred that said reference are (average) reference expression profiles (data) of at least two subjects, preferably of at least 2 to 200 subjects, more preferably of at least 10 to 150 subjects, and most preferably of at least 20 to 100 subjects, with at least two known clinical conditions, preferably at least 2 to 5, more preferably at least 2 to 4 (i.e. at least 2, 3, 4, or 5) known clinical conditions, from which at least one is psoriasis), wherein the reference expression profiles are the profiles of a set comprising at least two miRNAs that have nucleotide sequences that essentially correspond (are essentially identical), preferably that correspond (are identical), to the nucleotide sequences of the miRNAs which expression profile is determined by the means of (i).

It is preferred that the reference is generated from expression profilies (data) obtained from 2 clinical conditions, which are psoriasis and healthy control.

Preferably, (i) the (average) reference expression profile (data), which is provided with the kit, is determined in the same type of blood cell sample and is obtained from (control) subject(s) of the same species, gender and/or of similar age/stage of life, or (ii) the (average) reference expression profiles (data), which are provided with the kit, are determined in the same type of blood cell sample and are obtained from (control) subject(s) of the same species, gender and/or of similar age/stage of life.

Said reference, preferably said (average) reference expression profile(s) (data) may be comprised in an information leaflet (e.g. for comparing tested single reference miRNA biomarkers with the expression profile data of a patient to be diagnosed) or saved on a data carrier (e.g. for comparing tested sets of miRNA biomarkers with the expression profile data of a patient to be diagnosed). Said reference, preferably said (average) reference expression profile(s) (data) may also be comprised in a computer program which is saved on a data carrier. The kit may alternatively comprise an access code which allows the access to a database, e.g. an internet database, a centralized or a decentralized database, where said reference, preferably said (average) reference expression profile(s) (data) is (are) comprised.

It is particularly preferred that the reference is an algorithm or mathematical function.

Also described is an algorithm or a mathematical function which is obtained from a reference expression profile (data) of at least one subject, preferably the algorithm or mathematical function is obtained from an average reference expression profile (data) of at least 2 to 200 subjects, more preferably of at least 10 to 150 subjects, and most preferably of at least 20 to 100 subjects, i.e. of at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 subjects, with one known clinical condition which is psoriasis or a specific form of psoriasis, or which is not psoriasis or a specific form of psoriasis (i.e. healthy/healthiness), wherein the reference expression profile is the profile of a single miRNA that has a nucleotide sequence that essentially corresponds (is essentially identical), preferably that corresponds (is identical), to the nucleotide sequence of the miRNA which expression profile is determined by the means of (i), or is the profile of a set comprising at least two miRNAs that have nucleotide sequences that essentially correspond (are essentially identical), preferably that correspond (are identical), to the nucleotide sequences of the miRNAs which expression profile is determined by the means of (i).

It is also preferred that the algorithm or mathematical function is obtained from (average) reference expression profiles (data) of at least two subjects, preferably of at least 2 to 200 subjects, more preferably of at least 10 to 150 subjects, and most preferably of at least 20 to 100 subjects, i.e. of at least 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 subjects, with at least two known clinical conditions, preferably at least 2 to 5, more preferably at least 2 to 4 (i.e. at least 2, 3, 4, or 5) known clinical conditions, from which at least one is psoriasis, wherein the reference expression profiles are the profiles of a single miRNA that has a nucleotide sequence that essentially corresponds (is essentially identical), preferably that corresponds (is identical), to the nucleotide sequence of the miRNA which expression profile is determined by the means of (i) or are the profiles of a set comprising at least two miRNAs that have nucleotide sequences that essentially correspond (are essentially identical), preferably that correspond (are identical), to the nucleotide sequences of the miRNAs which expression profile is determined by the means of (i).

It is preferred that the algorithm or mathematical function is obtained using a machine learning approach (see first aspect of the present invention).

Preferably, the algorithm or mathematical function is saved on a data carrier comprised in the kit or the computer program, wherein the algorithm or mathematical function is comprised, is saved on a data carrier comprised in the kit. Said kit may alternatively comprise an access code which allows the access to an internet page, where the algorithm or mathematical function is saved or where the computer program, wherein the algorithm or mathematical function is comprised, can be downloaded.

Preferably, the algorithm or mathematical function is saved on a data carrier or the algorithm or mathematical function is comprised in a computer program which is saved on a data carrier. Said kit may alternatively comprise an access code which allows the access to a database or an internet page, where the algorithm or mathematical function is comprised, or where a computer program comprising the algorithm or mathematical function can be downloaded.

More than one reference may be comprised in the kit, e.g. 2, 3, 4, 5, or more references. For example, the kit may comprise reference data, preferably (average) reference expression profile(s) (data), which may be comprised in an information leaflet or saved on a data carrier. In addition, the kit may comprise more than one algorithm or mathematical function, e.g. two algorithms or mathematical functions, e.g. one trained to discriminate between a healthy condition and psoriasis and one trained to discriminate between specific forms of psoriasis, e.g. comprised in a computer program, preferably stored on a data carrier.

In a fourth aspect, the invention relates to the *in vitro* use of a set of miRNAs isolated from a blood sample from a subject for diagnosing and/or prognosing of psoriasis, wherein the miRNAs are selected from the group consisting of SEQ ID 1 to 111, wherein the nucleotide sequence of a first of said at least two miRNAs is SEQ ID NO: 2 and wherein the blood sample is a blood cell sample.

Preferably, the blood cell sample is a leukocyte- , erythrocyte- and/or platelet-containing sample..

It is further preferred that the blood sample has been collected under conditions where the RNA-fraction is guarded against degradation, preferably the blood sample is collected in a PAXgene (RNA) Tube.

It is preferred that the subject is a mammal including both a human and another mammal, e.g. an animal such as a mouse, a rat, a rabbit, or a monkey. It is particularly preferred that the subject is a human.

Preferably, the set comprising at least two miRNAs comprises at least one up-regulated miRNA listed in Figure 4a or comprises at least one down-regulated miRNAs listed in Figure 5a. More preferably, the set comprising at least two miRNAs comprises at least one up-regulated miRNA listed in Figure 4b or comprises at least one down-regulated miRNAs listed in Figure 5b. It is further preferred, that the set comprising at least two miRNAs comprises at least one up-regulated miRNA listed in Figure 4a and further comprises at least one down-regulated miRNAs listed in Figure 5a.

Preferably, the predetermined set comprising at least two miRNAs that are differentially regulated in blood cell samples from psoriasis patients as compared to healthy controls is selected from the set of miRNAs listed in Figure 2, which comprises SEQ ID NO: 2.

It is preferred that the predetermined set comprising at least two miRNAs that are differentially regulated in blood cell samples from psoriasis patients as compared to healthy controls comprises at least one set of miRNAs listed in Figure 2, which comprises SEQ ID NO: 2.

Also described herein but not encompassed by the present invention is a method for determining the status and/or the response of the immune system in a subject having or suspected of having psoriasis, comprising the steps of :
(i.) determining an expression profile of a set comprising at least two miRNAs representative for the status and/or the response of the immune system in a body fluid sample from a subject, and
(ii) comparing said expression profile to a reference, wherein the comparison of said expression profile to said reference allows for determining the status and/or the response of the immune system in said subject

It is preferred that the body fluid sample is a blood sample, particularly preferred it is a whole blood, PBMC, serum, plasma or leukocyte sample, more particularly preferred it is a blood cell sample, preferably a leukocyte- , erythrocyte and/or platelet-containing sample.

It is further preferred that the body fluid sample is a blood sample that has been collected under conditions where the RNA-fraction is guarded against degradation, preferably the blood sample is collected in a PAXgene (RNA) Tube.

It is preferred that the subject is a mammal including both a human and another mammal, e.g. an animal such as a mouse, a rat, a rabbit, or a monkey. It is particularly preferred that the subject is a human.

Preferably, the set comprising at least two miRNAs is from the group consisting of SEQ ID NO: 1 to 283

It is preferred that the set comprising at least two miRNAs is selected from the set of miRNAs listed in Figure 2.

It is preferred that the set comprising at least two miRNAs comprises at least one set of miRNAs listed in Figure 2.

Preferably, the set comprising at least two miRNAs in the method for determining the status and/or the response of the immune system in a subject having or suspected of having psoriasis comprises at least one up-regulated miRNA listed in Figure 4a or comprises at least one down-regulated miRNAs listed in Figure 4a. More preferably, the set comprising at least two miRNAs comprises at least one up-regulated miRNA listed in Figure 4b or comprises at least one down-regulated miRNAs listed in Figure 5b. It is further preferred, that the set comprising at least two miRNAs comprises at least one up-regulated miRNA listed in Figure 4a and further comprises at least one down-regulated miRNAs listed in Figure 5a.

It is preferred that the determining the expression profile of a set comprising at least two miRNAs selected from the group consisting of SEQ ID NO: 1 to 283 is for or is representative for determining the status and/or the response of the immune system in a body fluid sample, preferably in a blood sample, of the subject having of suspected of having psoriasis.

It is further preferred that the determination of the status and/or the response of the immune system in a body fluid sample or blood sample, allows for a diagnosis in the subject having or suspected of having psoriasis.

Preferably, the determination of the status and/or the response of the immune system in a body fluid sample or blood sample, allows for a treatment decision in said subject.

Also described herein but not encompassed by the present invention is a method for diagnosing and/or prognosing of psoriasis comprising the steps of:
(i) providing a set comprising at least two polynucleotides used according to the second aspect of the present invention for detecting a set comprising at least two miRNAs representative for psoriasis in a body fluid sample (e.g. blood sample) from a patient (e.g. human or animal),
   wherein the nucleotide sequence of said miRNA or the nucleotide sequences of said miRNAs is (are) preferably selected from the group consisting of SEQ ID NO: 1 to 111 or SEQ ID NO: 1 to 283, a fragment thereof, and a sequence having at least 80% sequence identity thereto,
(ii) using the polynucleotide(s) provided in (i) for determining an miRNA expression profile in a body fluid sample (e.g. blood sample) from a patient (e.g. human or animal) with an unknown clinical condition,
(iii) comparing said expression profile to a reference,
(iv) diagnosing or prognosing the clinical condition of the patient (e.g. human or animal) on the basis of said comparison.

The term "patient with an unknown clinical condition" refers to a patient (e.g. human or animal) which may suffer from psoriasis (i.e. diseased patient) or may not suffer from psoriasis (i.e. healthy patient). The patient (e.g. human or animal) to be diagnosed may further suffer from a specific type of psoriasis. It is also possible to determine, whether the patient (e.g. human or animal) to be diagnosed will develop the above mentioned disease as the inventors of the present invention surprisingly found that miRNAs representative for psoriasis are already present in the body fluid sample, e.g. blood sample, before psoriasis occurs or during the early stage of psoriasis. It should be noted that a patient that is diagnosed as being healthy, i.e. not suffering from psoriasis, may possibly suffer from another disease not tested/known.

### BRIEF DESCRIPTION OF THE DRAWINGS

Figure 1: MiRNAs for diagnosis or prognosis of psoriasis. Experimental data obtained for analysis of miRNAs according to SEQ ID NO: 1 to 111. Experimental details: SEQ ID NO: sequence identification number, miRNA: identifier of the miRNA according to miRBase, median g1: median intensity obtained from microarray analysis for healthy controls, median g2: median intensity obtained from microarray analysis for individuals with psoriasis, qmedian: ratio of median g1/median g2, logqmedian: log of qmedian, ttest_rawp: p-value obtained when applying t-test, ttest_adjp: adjusted p-value in order to reduce false discovery rate by Benjamini-Hochberg adjustment, AUC: Area under the curve, limma_rawp: p-value obtained when applying limma-test, limma_adjp: adjusted p-value in order to reduce false discovery rate by Benjamini-Hochberg adjustment.
Figure 2: Sets of miRNAs (miRNA-signatures SNP-1 to SNP-1008) that allow for effective diagnosis and/or prognosis of psoriasis when differentiating psoriasis and healthy controls. Experimental details: SEQ ID NO: sequence identification number, miRNA: identifier of the miRNA according to miRBase, Acc = accuracy, Spec = specificity, Sens = sensitivity.

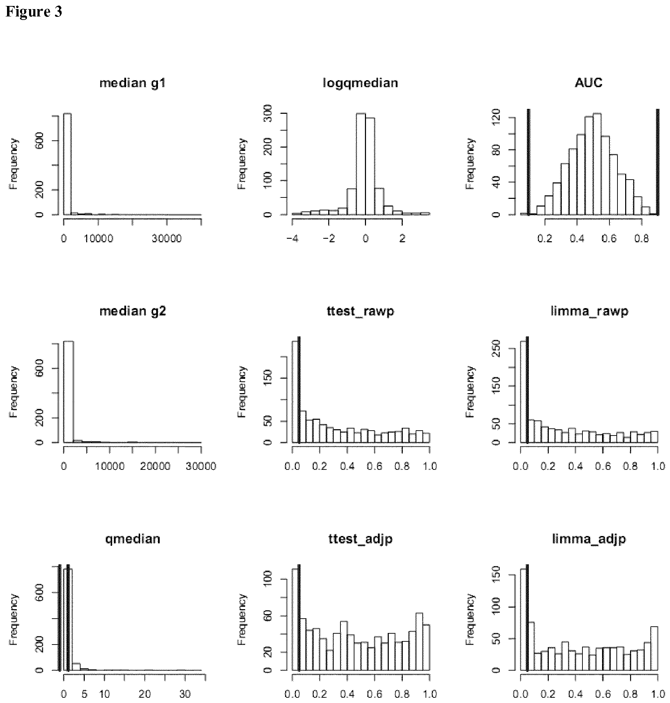

Figure 3: Graphical representation of the experimental data on miRNAs for diagnosis or prognosis of psoriasis. The histograms show the distribution curve obtained when 862 miRNAs biomarkers were analysed on microarrays. The thick lines separate the high informative miRNA biomarkers for diagnosis or prognosis of psoriasis in comparison to healthy controls from the non-informative ones. Experimental details: median g1: median intensity obtained from microarray analysis for healthy controls, median g2: median intensity obtained from microarray analysis for individuals with psoriasis, qmedian: ratio of median g1/median g2, logqmedian: log of qmedian, ttest_rawp: p-value obtained when applying t-test, ttest_adjp: adjusted p-value in order to reduce false discovery rate by Benjamini-Hochberg adjustment, AUC: Area under the curve, limma_rawp: p-value obtained when applying limma-test, limma_adjp: adjusted p-value in order to reduce false discovery rate by Benjamini-Hochberg adjustment.
Figure 4a: miRNAs that are up-regulated in psoriasis compared to healthy controls that allow for effective diagnosis and/or prognosis of psoriasis. Figure 4b: miRNAs that are strong up-regulated in psoriasis (Fold Change > 1.5) compared to healthy controls that allow for effective diagnosis and/or prognosis of psoriasis
Figure 5a : miRNAs that are down-regulated in psoriasis compared to healthy controls that allow for effective diagnosis and/or prognosis of psoriasis when differentiating psoriasis and healthy controls. Figure 5b : miRNAs that are strongly down-regulated (Fold Change > 1.5) in psoriasis compared to healthy controls that allow for effective diagnosis and/or prognosis of psoriasis when differentiating psoriasis and healthy controls.
Figure 6: MiRNAs for diagnosis or prognosis of psoriasis. Experimental data obtained for analysis of miRNAs. Experimental details: SEQ ID NO: sequence identification number, miRNA: identifier of the miRNA according to miRBase, median g1: median intensity obtained from microarray analysis for healthy controls, median g2: median intensity obtained from microarray analysis for individuals with psoriasis, qmedian: ratio of median g1/median g2, ttest_rawp: p-value obtained when applying t-test, ttest_adjp: adjusted p-value in order to reduce false discovery rate by Benjamini-Hochberg adjustment, AUC: Area under the curve, limma_rawp: p-value obtained when applying limma-test, limma_adjp: adjusted p-value in order to reduce false discovery rate by Benjamini-Hochberg adjustment.

### EXAMPLES

The Examples are designed in order to further illustrate the present invention and serve a better understanding. They are not to be construed as limiting the scope of the invention in any way.

### Materials and Methods

### Samples

All blood donors participating in this study have given their written informed consent. The patient samples have been prepared at the Institute for Human Genetics at University Saarland (Homburg,/Saar, Germany). Besides the samples of diseased patients, also control samples were provided.

### miRNA extraction and microarray screening

Blood of patients has been extracted as previously described [1]. In brief, 2.5 to 5 ml blood was extracted in PAXgene Blood RNA tubes (BD, Franklin Lakes, New Jersey USA) and centrifuged at 5000 x g for 10 min at room temperature. The miRNeasy kit (Qiagen GmbH, Hilden) was used to isolate total RNA including miRNA from the resuspended pellet according to manufacturer's instructions. The eluted RNA was stored at -70°C.

All samples were shipped overnight on dry ice and analyzed with the Geniom RT Analyzer (febit biomed GmbH, Heidelberg, Germany) at the in-house genomic service department using the Geniom Biochip miRNA Homo sapiens. Each array contains 7 replicates of about 863 miRNAs and miRNA star sequences as annotated in the Sanger miRBase releases 14.0. On-chip sample labeling with biotin was carried out by micro fluidic-based primer extension labeling of miRNAs (MPEA [2]). Following hybridization for 16 hours at 42°C, the biochip was washed and a program for signal enhancement was carried out. All steps from sample loading to miRNA detection were processed without any manual intervention and inside the machine. The detection pictures were evaluated using the Geniom Wizard Software. For each feature, the median signal intensity was calculated. Following a background correction step, the median of the 7 replicates of each miRNA was computed. To normalize the data across different arrays, quantile normalization [3] was applied and all further analyses were carried out using the normalized and background subtracted intensity values.

### Statistical analysis

To estimate the value of single miRNAs, t-tests (unpaired, two-tailed) were carried out. The resulting p-values have been adjusted for multiple testing by Benjamini-Hochberg adjustment [4, 5]. In addition to this single biomarker analysis, we performed supervised classification of samples by using Support Vector Machines (SVM [6]) as implemented in the R e1071 package [7]. As parameters, we evaluated different kernel methods including linear, polynomial (degree 2 to 5), sigmoid and radial basis function kernels. The cost parameter was sampled from 0.01 to 10 in decimal powers. As subset selection technique, a filter approach based on t-test was carried out. In each iteration, the *s* miRNAs with lowest p-values were computed on the training set in each fold of a standard 10-fold cross validation, where *s* was sampled in regular intervals between 2 and 300. The respective subset was used to train the SVM and to carry out the prediction of the test samples in the cross validation. To compute probabilities for classes instead of class labels, a regression approach based on the output of the support vectors has been applied. To test for overtraining, non-parametric permutation tests have been applied. All computations were carried out using R [7], a freely available language for statistical tasks.

### REFERENCES

1. Keller A, Leidinger P, Borries A, Wendschlag A, Wucherpfennig F, Scheffler M, Huwer H, Lenhof HP, Meese E: miRNAs in lung cancer - studying complex fingerprints in patient's blood cells by microarray experiments. BMC Cancer 2009, 9:353.
2. Vorwerk S, Ganter K, Cheng Y, Hoheisel J, Stahler PF, Beier M: Microfluidic-based enzymatic on-chip labeling of miRNAs. N Biotechnol 2008, 25(2-3):142-149.
3. Bolstad BM, Irizarry RA, Astrand M, Speed TP: A comparison of normalization methods for high density oligonucleotide array data based on variance and bias. Bioinformatics 2003, 19(2):185-193.
4. Benjamini Y, Drai D, Elmer G, Kafkafi N, Golani I: Controlling the false discovery rate in behavior genetics research. Behav Brain Res 2001, 125(1-2):279-284.
5. Hochberg Y: A sharper bonferroni procedure for multiple tests of significance. Biometrica 1988, 75:185-193.
6. Vapnik V: The nature of statistical learning theory., 2nd edition edn. New York: Spinger; 2000.
7. Team R: R: A Language and Environment for Statistical Computing. In. Vienna: R Foundation for Statistical Computing; 2008.

## Claims

1. A method for diagnosing and/or prognosing of psoriasis comprising the steps of:
(i) determining an expression profile of a set comprising at least two miRNAs representative for psoriasis in a blood sample from a subject, and
(ii) comparing said expression profile to a reference, wherein the comparison of said expression profile to said reference allows for the diagnosis and/or prognosis of psoriasis,
wherein the nucleotide sequences of the miRNAs comprised in the set are selected from the group consisting of SEQ ID NO: 1 to 111, a fragment thereof, and a sequence having at least 80% sequence identity thereto, and wherein the nucleotide sequence of a first of said at least two miRNAs is SEQ ID NO: 2, and wherein the blood sample is a blood cell sample.

2. The method of claim 1, wherein the blood cell sample is selected from blood cellular fractions including erythrocytes, leukocytes, platelets.

3. The method of claims 1 or 2, wherein the expression of said first miRNA with SEQ ID NO: 2 is downregulated when compared to the reference.

4. The method of any of the claims 1 to 3, wherein the set of miRNAs comprises at least one of the sets of miRNAs listed in Figure 2, which comprise SEQ ID NO:2.

5. Use of a set comprising polynucleotides for detecting a set comprising at least two miRNAs for diagnosing and/or prognosing of psoriasis in a blood sample from a subject, wherein the nucleotide sequences of the miRNAs comprised in the set are selected from the group consisting of SEQ ID NO: 1 to 111, and wherein the nucleotide sequence of a first of said at least two miRNAs is SEQ ID NO: 2, and wherein the blood sample is a blood cell sample.

6. The use according to claim 5, wherein the blood cell sample is selected from blood cellular fractions including erythrocytes, leukocytes, platelets.

7. The use according to claims 5 to 6, wherein the set comprising at least two miRNAs is selected from the sets of miRNAs listed in Figure 2, which comprise SEQ ID NO:2.

8. The use according to claims 5 to 7, wherein
(i) the polynucleotides comprised in the set are complementary to the miRNAs comprised in the set according to claims 5 to 7,
(ii) the polynucleotides comprised in the set are fragments of the polynucleotides comprised in the set according to (i), or
(iii) the polynucleotides comprised in the set have at least 80% sequence identity to the polynucleotide sequences of the polynucleotides comprised in the set according to (i) or polynucleotide fragments comprised in the set according to (ii).

9. Use of a kit for diagnosing and/or prognosing of psoriasis comprising
(i) means for determining an expression profile of a set comprising at least two miRNAs representative for psoriasis in a blood sample from a subject, and
(ii) at least one reference.
wherein the nucleotide sequences of said miRNAs are selected from the group consisting of SEQ ID NO: 1 to 111, a fragment thereof, and a sequence having at least 80% sequence identity thereto, and
wherein the nucleotide sequence of a first of said at least two miRNAs is SEQ ID NO: 2, and
wherein the blood sample is a blood cell sample, and
wherein the reference is generated from reference expression profiles of at least two control subjects of at least two clinical conditions from which at least one is psoriasis determined in the same type of blood sample as the subject to be diagnosed and/or prognosed.

10. The use of a kit of claim 9, wherein said means comprise
(i.) a set of at least two polynucleotides according to any of the claims 6 to 10 and
(ii.) a biochip, a RT-PCR system, a PCR-system, a flow cytometer or a next generation sequencing system

11. In vitro use of a set of at least two miRNAs isolated from a blood sample from a subject for diagnosing and/or prognosing of psoriasis, wherein the miRNAs are selected from the group consisting of SEQ ID NO: 1 to 111 and wherein the nucleotide sequence of a first of said at least two miRNAs is SEQ ID NO: 2, and wherein the blood sample is a blood cell sample.

12. The in vitro use according to claim 11, wherein the blood sample is selected from blood cellular fractions including erythrocytes, leukocytes, platelets.

13. The in vitro use according to claim 11 or 12, wherein the set comprising at least two miRNAs is selected from the sets of miRNAs listed in Figure 2, which comprise SEQ ID NO:2.

## Patentansprüche

1. Verfahren zur Diagnose und/oder Prognose von Psoriasis umfassend die Schritte:
(i) Ermitteln eines Expressionsprofiles eines Sets umfassend mindestens zwei miRNAs, die für Psoriasis repräsentativ sind, in einer Blutprobe eines Subjekts, und
(ii) Vergleichen des Expressionsprofiles mit einer Referenz, wobei der Vergleich des Expressionsprofils mit der Referenz die Diagnose und/oder Prognose von Psoriasis ermöglicht,
wobei die Nukleotidsequenzen der miRNAs, die von dem Set umfasst sind, aus der Gruppe bestehend aus SEQ ID NO: 1 bis SEQ ID NO: 111, einem Fragment davon, und einer Sequenz, die mindestens 80% Sequenzidentität dazu aufweist, ausgewählt sind, und
wobei die Nukleotidsequenz einer ersten der mindestens zwei miRNAs SEQ ID NO: 2 ist, und
wobei die Blutprobe eine Blutzellprobe ist.

2. Verfahren nach Anspruch 1, wobei die Blutzellprobe aus Blutzellfraktionen, die Erythrozyten, Leukozyten und Blutplättchen umfassen, ausgewählt ist.

3. Verfahren nach Anspruch 1 oder 2, wobei die Expression der ersten miRNA mit SEQ ID NO: 2 im Vergleich zu der Referenz herunterreguliert ist.

4. Verfahren nach einem der Ansprüche 1 bis 3, wobei das Set von miRNAs mindestens eines der Sets von miRNAs, die in Figur 2 aufgeführt sind und SEQ ID NO: 2 umfassen, umfasst.

5. Verwendung eines Sets umfassend Polynukleotide zum Nachweis eines Sets umfassend mindestens zwei miRNAs zur Diagnose und/oder Prognose von Psoriasis in einer Blutprobe eines Subjekts,
wobei die Nukleotidsequenzen der vom Set umfassten miRNAs aus der Gruppe bestehend aus SEQ ID NO: 1 bis SEQ ID NO: 111 ausgewählt sind, und
wobei die Nukleotidsequenz einer ersten der mindestens zwei miRNAs SEQ ID NO: 2 ist, und
wobei die Blutprobe eine Blutzellprobe ist.

6. Verwendung nach Anspruch 5, wobei die Blutzellprobe aus Blutzellfraktionen, die Erythrozyten, Leukozyten und Blutplättchen umfassen, ausgewählt ist.

7. Verwendung nach Anspruch 5 oder 6, wobei das Set, welches mindestens zwei miRNAs umfasst, aus den Sets von miRNAs ausgewählt ist, die in Figur 2 aufgeführt sind und SEQ ID NO: 2 umfassen.

8. Verwendung nach Ansprüchen 5 bis 7, wobei
(i) die Polynukleotide, die von dem Set umfasst sind, zu den miRNAs, die von dem Set gemäß den Ansprüchen 5 bis 7 umfasst sind, komplementär sind,
(ii) die Polynukleotide, die von dem Set umfasst sind, Fragmente der Polynukleotide sind, die von dem Set gemäß (i) umfasst sind, oder
(iii) die Polynukleotide, die von dem Set umfasst sind, mindestens 80% Sequenzidentität zu den Polynukleotidsequenzen der Polynukleotide, die von dem Set gemäß (i) umfasst sind, oder der Polynukleotidfragmente, die von dem Set gemäß (ii) umfasst sind, aufweisen.

9. Verwendung eines Kits zur Diagnose und/oder Prognose von Psoriasis umfassend:
(i) Mittel zum Ermitteln eines Expressionsprofils eines Sets umfassend mindestens zwei miRNAs, die für Psoriasis repräsentativ sind, in einer Blutprobe eines Subjekts, und
(ii) mindestens eine Referenz,
wobei die Nukleotidsequenzen der miRNAs aus der Gruppe bestehend aus SEQ ID NO: 1 bis SEQ ID NO: 111, einem Fragment davon, und einer Sequenz, die mindestens 80% Sequenzidentität dazu aufweist, ausgewählt sind, und
wobei die Nukleotidsequenz einer ersten der mindestens zwei miRNAs SEQ ID NO: 2 ist, und
wobei die Blutprobe eine Blutzellprobe ist, und
wobei die Referenz von Referenzexpressionsprofilen von mindestens zwei Kontrollsubjekten mit mindestens zwei klinischen Zuständen generiert ist, wobei mindestens einer dieser Zustände Psoriasis ist, die im gleichen Typ Blutprobe wie das Subjekt, welches zu diagnostizieren und/oder zu prognostizieren ist, ermittelt wurden.

10. Verwendung eines Kits nach Anspruch 9, wobei die Mittel umfassen:
(i.) ein Set von mindestens zwei Polynukleotiden gemäß einem der Ansprüche 6 bis 10, und
(ii.) einen Biochip, ein RT-PCR System, ein PCR-System, ein Durchflusszytometer oder ein *Next Generation* Sequenzierungssystem.

11. *In vitro* Verwendung eines Sets von mindestens zwei miRNAs isoliert aus einer Blutprobe eines Subjekts zur Diagnose und/oder Prognose von Psoriasis,
wobei die miRNAs aus der Gruppe bestehend aus SEQ ID NO: 1 bis SEQ ID NO: 111 ausgewählt sind, und
wobei die Nukleotidsequenz einer ersten der mindestens zwei miRNAs SEQ ID NO: 2 ist, und
wobei die Blutprobe eine Blutzellprobe ist.

12. *In vitro* Verwendung nach Anspruch 11, wobei die Blutzellprobe aus Blutzellfraktionen, die Erythrozyten, Leukozyten und Blutplättchen umfassen, ausgewählt ist.

13. *In vitro* Verwendung nach Anspruch 11 oder 12, wobei das Set, welches mindestens zwei miRNAs umfasst, aus den Sets von miRNAs ausgewählt ist, die in Figur 2 aufgeführt sind und SEQ ID NO: 2 umfassen.

## Revendications

1. Procédé de diagnostic et/ou de pronostic du psoriasis comprenant les étapes de :
(i) détermination d'un profil d'expression d'un ensemble comprenant au moins deux ARNmi représentatifs du psoriasis dans un échantillon de sang provenant d'un sujet, et
(ii) comparaison dudit profil d'expression à une référence, ladite comparaison dudit profil d'expression à ladite référence permettant le diagnostic et/ou le pronostic du psoriasis,
les séquences nucléotidiques des ARNmi compris dans l'ensemble étant choisies dans le groupe constitué par les SEQ ID n° : 1 à 111, un fragment de celles-ci et une séquence présentant une identité de séquence d'au moins 80 % à celles-ci, et ladite séquence nucléotidique d'un premier desdits au moins deux ARNmi étant la SEQ ID n° : 2, et ledit échantillon de sang étant une échantillon de cellules sanguines.

2. Procédé selon la revendication 1, ledit échantillon de cellules sanguines étant choisi parmi des fractions de cellules sanguines comprenant des érythrocytes, des leucocytes, des plaquettes.

3. Procédé selon la revendication 1 ou 2, ladite expression dudit premier ARNmi avec la SEQ ID n° : 2 étant régulée à la baisse comparée à la référence.

4. Procédé selon l'une quelconque des revendications 1 à 3, ledit ensemble d'ARNmi comprenant au moins l'un des ensembles d'ARNmi énumérés dans la figure 2, qui comprennent la SEQ ID n° : 2.

5. Utilisation d'un ensemble comprenant des polynucléotides permettant de détecter un ensemble d'au moins deux ARNmi pour le diagnostic et/ou le pronostic du psoriasis dans un échantillon de sang provenant d'un sujet, lesdites séquences nucléotidiques des ARNmi compris dans l'ensemble étant choisies dans le groupe constitué par les SEQ ID n° : 1 à 111, et ladite séquence nucléotidique d'un premier desdits au moins deux ARNmi étant la SEQ ID n° : 2, et ledit échantillon de sang étant un échantillon de cellules sanguines.

6. Utilisation selon la revendication 5, ledit échantillon de cellules sanguines étant choisi parmi des fractions de cellules sanguines comprenant des érythrocytes, des leucocytes, des plaquettes.

7. Utilisation selon les revendications 5 à 6, ledit ensemble comprenant au moins deux ARNmi étant choisi parmi les ensembles d'ARNmi énumérés dans la figure 2, qui comprennent la SEQ ID n° : 2.

8. Utilisation selon les revendications 5 à 7,
(i) les polynucléotides compris dans l'ensemble étant complémentaires aux ARNmi compris dans l'ensemble selon les revendications 5 à 7,
(ii) les polynucléotides compris dans l'ensemble étant des fragments des polynucléotides compris dans l'ensemble selon (i), ou
(iii) les polynucléotides compris dans l'ensemble ayant une identité de séquence d'au moins 80 % avec les séquences polynucléotidiques des polynucléotides compris dans l'ensemble selon (i) ou les fragments de polynucléotides compris dans l'ensemble selon (ii).

9. Utilisation d'un kit pour le diagnostic et/ou le pronostic du psoriasis comprenant
(i) des moyens pour déterminer un profil d'expression d'un ensemble comprenant au moins deux ARNmi représentatifs du psoriasis dans un échantillon de sang provenant d'un sujet, et
(ii) au moins une référence,
lesdites séquences nucléotidiques desdits ARNmi étant choisies dans le groupe constitué par les SEQ ID n° : 1 à 111, un fragment de celles-ci et une séquence présentant une identité de séquence d'au moins 80 % avec celles-ci, et
ladite séquence nucléotidique d'un premier desdits au moins deux ARNmi étant la SEQ ID n° : 2, et
ledit échantillon de sang étant un échantillon de cellules sanguines, et
ladite référence étant générée à partir de profils d'expression de référence d'au moins deux sujets témoins présentant au moins deux états cliniques parmi lesquels au moins l'un est le psoriasis déterminé dans le même type d'échantillon de sang que le sujet devant subir un diagnostic et/ou un pronostic.

10. Utilisation d'un kit selon la revendication 9, lesdits moyens comprenant
(i.) un ensemble d'au moins deux polynucléotides selon l'une quelconque des revendications 6 à 10, et
(ii.) une puce à ADN, un système de RT-PCR, un système de PCR, un cytomètre à flux ou un système de séquençage nouvelle génération.

11. Utilisation *in vitro* d'un ensemble d'au moins deux ARNmi isolés à partir d'un échantillon de sang provenant d'un sujet pour le diagnostic et/ou le pronostic du psoriasis, lesdits ARNmi étant choisis dans le groupe constitué par les SEQ ID n° : 1 à 111, et ladite séquence nucléotidique d'un premier desdits au moins deux ARNmi étant la SEQ ID n° : 2, et ledit échantillon de sang étant un échantillon de cellules sanguines.

12. Utilisation *in vitro* selon la revendication 11, ledit échantillon de sang étant choisi parmi des fractions de cellules sanguines comprenant des érythrocytes, des leucocytes, des plaquettes.

13. Utilisation *in vitro* selon la revendication 11 ou 12, ledit ensemble comprenant au moins deux ARNmi étant choisi parmi les ensembles d'ARNmi énumérés dans la figure 2, qui comprennent la SEQ ID n° : 2.


### SEQUENCE LISTING

<110> febit holding GmbH
<120> Complex sets of miRNAs as non-invasive biomarkers for psoriasis
<130> FP-005
<160> 283
<170> PatentIn version 3.5
<210> 1
   <211> 22
   <212> RNA
   <213> Homo sapiens
<400> 1
   uagcaccauu ugaaaucggu ua 22
<210> 2
   <211> 23
   <212> RNA
   <213> Homo sapiens
<400> 2
   uagcaccauu ugaaaucagu guu 23
<210> 3
   <211> 21
   <212> RNA
   <213> Homo sapiens
<400> 3
   uaaagugcug acagugcaga u 21
<210> 4
   <211> 22
   <212> RNA
   <213> Homo sapiens
<400> 4
   ucuacaaagg aaagcgcuuu cu 22
<210> 5
   <211> 22
   <212> RNA
   <213> Homo sapiens
<400> 5
   uucuggaauu cugugugagg ga 22
<210> 6
   <211> 22
   <212> RNA
   <213> Homo sapiens
<400> 6
   uguaaacauc cuugacugga ag 22
<210> 7
   <211> 20
   <212> RNA
   <213> Homo sapiens
<400> 7
   gugcauugcu guugcauugc 20
<210> 8
   <211> 22
   <212> RNA
   <213> Homo sapiens
<400> 8
   ugcccugugg acucaguucu gg 22
<210> 9
   <211> 22
   <212> RNA
   <213> Homo sapiens
<400> 9
   aucgggaaug ucguguccgc cc 22
<210> 10
   <211> 22
   <212> RNA
   <213> Homo sapiens
<400> 10
   ucagugcacu acagaacuuu gu 22
<210> 11
   <211> 22
   <212> RNA
   <213> Homo sapiens
<400> 11
   caaccuggag gacuccaugc ug 22
<210> 12
   <211> 23
   <212> RNA
   <213> Homo sapiens
<400> 12
   cagugcaaua guauugucaa agc 23
<210> 13
   <211> 23
   <212> RNA
   <213> Homo sapiens
<400> 13
   gcagcagaga auaggacuac guc 23
<210> 14
   <211> 22
   <212> RNA
   <213> Homo sapiens
<400> 14
   aacacaccua uucaaggauu ca 22
<210> 15
   <211> 22
   <212> RNA
   <213> Homo sapiens
<400> 15
   cuccugacuc cagguccugu gu 22
<210> 16
   <211> 21
   <212> RNA
   <213> Homo sapiens
<400> 16
   aucauagagg aaaauccacg u 21
<210> 17
   <211> 22
   <212> RNA
   <213> Homo sapiens
<400> 17
   ugcuaugcca acauauugcc au 22
<210> 18
   <211> 22
   <212> RNA
   <213> Homo sapiens
<400> 18
   gagcuuauuc auaaaagugc ag 22
<210> 19
   <211> 21
   <212> RNA
   <213> Homo sapiens
<400> 19
   acagucugcu gagguuggag c 21
<210> 20
   <211> 22
   <212> RNA
   <213> Homo sapiens
<400> 20
   uuuucaacuc uaaugggaga ga 22
<210> 21
   <211> 22
   <212> RNA
   <213> Homo sapiens
<400> 21
   ugugacuggu ugaccagagg gg 22
<210> 22
   <211> 22
   <212> RNA
   <213> Homo sapiens
<400> 22
   ugucuuacuc ccucaggcac au 22
<210> 23
   <211> 22
   <212> RNA
   <213> Homo sapiens
<400> 23
   ugagaccucu ggguucugag cu 22
<210> 24
   <211> 23
   <212> RNA
   <213> Homo sapiens
<400> 24
   ugaggggcag agagcgagac uuu 23
<210> 25
   <211> 21
   <212> RNA
   <213> Homo sapiens
<400> 25
   uagcagcaca gaaauauugg c 21
<210> 26
   <211> 22
   <212> RNA
   <213> Homo sapiens
<400> 26
   uuuugcaaua uguuccugaa ua 22
<210> 27
   <211> 23
   <212> RNA
   <213> Homo sapiens
<400> 27
   aacauucaac gcugucggug agu 23
<210> 28
   <211> 22
   <212> RNA
   <213> Homo sapiens
<400> 28
   uggaguguga caaugguguu ug 22
<210> 29
   <211> 22
   <212> RNA
   <213> Homo sapiens
<400> 29
   cugccaauuc cauaggucac ag 22
<210> 30
   <211> 21
   <212> RNA
   <213> Homo sapiens
<400> 30
   aucacauugc cagggauuuc c 21
<210> 31
   <211> 21
   <212> RNA
   <213> Homo sapiens
<400> 31
   gaaggcgcuu cccuuuggag u 21
<210> 32
   <211> 22
   <212> RNA
   <213> Homo sapiens
<400> 32
   acagcaggca cagacaggca gu 22
<210> 33
   <211> 23
   <212> RNA
   <213> Homo sapiens
<400> 33
   caacggaauc ccaaaagcag cug 23
<210> 34
   <211> 22
   <212> RNA
   <213> Homo sapiens
<400> 34
   aucgugcauc cuuuuagagu gu 22
<210> 35
   <211> 22
   <212> RNA
   <213> Homo sapiens
<400> 35
   uucaacgggu auuuauugag ca 22
<210> 36
   <211> 22
   <212> RNA
   <213> Homo sapiens
<400> 36
   ugagguagua gguuguauag uu 22
<210> 37
   <211> 20
   <212> RNA
   <213> Homo sapiens
<400> 37
   auggagauag auauagaaau 20
<210> 38
   <211> 22
   <212> RNA
   <213> Homo sapiens
<400> 38
   caacuagacu gugagcuucu ag 22
<210> 39
   <211> 20
   <212> RNA
   <213> Homo sapiens
<400> 39
   ccauggaucu ccaggugggu 20
<210> 40
   <211> 24
   <212> RNA
   <213> Homo sapiens
<400> 40
   uucuccaaaa gaaagcacuu ucug 24
<210> 41
   <211> 24
   <212> RNA
   <213> Homo sapiens
<400> 41
   uuuggcaaug guagaacuca cacu 24
<210> 42
   <211> 22
   <212> RNA
   <213> Homo sapiens
<400> 42
   ugccuacuga gcugaaacac ag 22
<210> 43
   <211> 22
   <212> RNA
   <213> Homo sapiens
<400> 43
   ugcaacuuac cugagucauu ga 22
<210> 44
   <211> 22
   <212> RNA
   <213> Homo sapiens
<400> 44
   uuauaaagca augagacuga uu 22
<210> 45
   <211> 21
   <212> RNA
   <213> Homo sapiens
<400> 45
   caucccuugc augguggagg g 21
<210> 46
   <211> 22
   <212> RNA
   <213> Homo sapiens
<400> 46
   aaucacuaac cacacggcca gg 22
<210> 47
   <211> 21
   <212> RNA
   <213> Homo sapiens
<400> 47
   ucagugcaug acagaacuug g 21
<210> 48
   <211> 21
   <212> RNA
   <213> Homo sapiens
<400> 48
   augaccuaug aauugacaga c 21
<210> 49
   <211> 22
   <212> RNA
   <213> Homo sapiens
<400> 49
   cuauacggcc uccuagcuuu cc 22
<210> 50
   <211> 22
   <212> RNA
   <213> Homo sapiens
<400> 50
   aggcagugua uuguuagcug gc 22
<210> 51
   <211> 23
   <212> RNA
   <213> Homo sapiens
<400> 51
   aaaagugcuu acagugcagg uag 23
<210> 52
   <211> 22
   <212> RNA
   <213> Homo sapiens
<400> 52
   caauuuagug ugugugauau uu 22
<210> 53
   <211> 22
   <212> RNA
   <213> Homo sapiens
<400> 53
   ggcuacaaca caggacccgg gc 22
<210> 54
   <211> 21
   <212> RNA
   <213> Homo sapiens
<400> 54
   cggcggggac ggcgauuggu c 21
<210> 55
   <211> 22
   <212> RNA
   <213> Homo sapiens
<400> 55
   auccgcgcuc ugacucucug cc 22
<210> 56
   <211> 21
   <212> RNA
   <213> Homo sapiens
<400> 56
   uacaguacug ugauaacuga a 21
<210> 57
   <211> 23
   <212> RNA
   <213> Homo sapiens
<400> 57
   ucaagagcaa uaacgaaaaa ugu 23
<210> 58
   <211> 22
   <212> RNA
   <213> Homo sapiens
<400> 58
   gaaguuguuc gugguggauu cg 22
<210> 59
   <211> 22
   <212> RNA
   <213> Homo sapiens
<400> 59
   caaaaguaau uguggauuuu gu 22
<210> 60
   <211> 20
   <212> RNA
   <213> Homo sapiens
<400> 60
   aaaguagcug uaccauuugc 20
<210> 61
   <211> 21
   <212> RNA
   <213> Homo sapiens
<400> 61
   caaagcgcuu cccuuuggag c 21
<210> 62
   <211> 22
   <212> RNA
   <213> Homo sapiens
<400> 62
   uuauaauaca accugauaag ug 22
<210> 63
   <211> 19
   <212> RNA
   <213> Homo sapiens
<400> 63
   aguguggcuu ucuuagagc 19
<210> 64
   <211> 17
   <212> RNA
   <213> Homo sapiens
<400> 64
   ucccuguucg ggcgcca 17
<210> 65
   <211> 25
   <212> RNA
   <213> Homo sapiens
<400> 65
   gggcgacaaa gcaagacucu uucuu 25
<210> 66
   <211> 22
   <212> RNA
   <213> Homo sapiens
<400> 66
   uacccauugc auaucggagu ug 22
<210> 67
   <211> 24
   <212> RNA
   <213> Homo sapiens
<400> 67
   cugaagugau guguaacuga ucag 24
<210> 68
   <211> 22
   <212> RNA
   <213> Homo sapiens
<400> 68
   uguaacagca acuccaugug ga 22
<210> 69
   <211> 23
   <212> RNA
   <213> Homo sapiens
<400> 69
   ccucagggcu guagaacagg gcu 23
<210> 70
   <211> 24
   <212> RNA
   <213> Homo sapiens
<400> 70
   ucccugagac ccuuuaaccu guga 24
<210> 71
   <211> 22
   <212> RNA
   <213> Homo sapiens
<400> 71
   cucuagaggg aagcacuuuc ug 22
<210> 72
   <211> 22
   <212> RNA
   <213> Homo sapiens
<400> 72
   uagcaccauc ugaaaucggu ua 22
<210> 73
   <211> 22
   <212> RNA
   <213> Homo sapiens
<400> 73
   augcaccugg gcaaggauuc ug 22
<210> 74
   <211> 22
   <212> RNA
   <213> Homo sapiens
<400> 74
   uugagaauga ugaaucauua gg 22
<210> 75
   <211> 22
   <212> RNA
   <213> Homo sapiens
<400> 75
   aaagugcauc cuuuuagagu gu 22
<210> 76
   <211> 22
   <212> RNA
   <213> Homo sapiens
<400> 76
   uauacaaggg cagacucucu cu 22
<210> 77
   <211> 23
   <212> RNA
   <213> Homo sapiens
<400> 77
   aggaugagca aagaaaguag auu 23
<210> 78
   <211> 21
   <212> RNA
   <213> Homo sapiens
<400> 78
   uagauaaaau auugguaccu g 21
<210> 79
   <211> 22
   <212> RNA
   <213> Homo sapiens
<400> 79
   caucuuccag uacaguguug ga 22
<210> 80
   <211> 23
   <212> RNA
   <213> Homo sapiens
<400> 80
   acugcccuaa gugcuccuuc ugg 23
<210> 81
   <211> 22
   <212> RNA
   <213> Homo sapiens
<400> 81
   ugcuggauca gugguucgag uc 22
<210> 82
   <211> 22
   <212> RNA
   <213> Homo sapiens
<400> 82
   uucucaagga ggugucguuu au 22
<210> 83
   <211> 22
   <212> RNA
   <213> Homo sapiens
<400> 83
   aagaugugga aaaauuggaa uc 22
<210> 84
   <211> 22
   <212> RNA
   <213> Homo sapiens
<400> 84
   gaggguuggg uggaggcucu cc 22
<210> 85
   <211> 22
   <212> RNA
   <213> Homo sapiens
<400> 85
   acccuaucaa uauugucucu gc 22
<210> 86
   <211> 22
   <212> RNA
   <213> Homo sapiens
<400> 86
   uggugggccg cagaacaugu gc 22
<210> 87
   <211> 22
   <212> RNA
   <213> Homo sapiens
<400> 87
   cacacacugc aauuacuuuu gc 22
<210> 88
   <211> 23
   <212> RNA
   <213> Homo sapiens
<400> 88
   ucccuguccu ccaggagcuc acg 23
<210> 89
   <211> 21
   <212> RNA
   <213> Homo sapiens
<400> 89
   cauaaaguag aaagcacuac u 21
<210> 90
   <211> 22
   <212> RNA
   <213> Homo sapiens
<400> 90
   uaacagucua cagccauggu cg 22
<210> 91
   <211> 22
   <212> RNA
   <213> Homo sapiens
<400> 91
   cauugcacuu gucucggucu ga 22
<210> 92
   <211> 23
   <212> RNA
   <213> Homo sapiens
<400> 92
   uuauugcuua agaauacgcg uag 23
<210> 93
   <211> 22
   <212> RNA
   <213> Homo sapiens
<400> 93
   gugacaucac auauacggca gc 22
<210> 94
   <211> 22
   <212> RNA
   <213> Homo sapiens
<400> 94
   cagugccucg gcagugcagc cc 22
<210> 95
   <211> 20
   <212> RNA
   <213> Homo sapiens
<400> 95
   ucugcagggu uugcuuugag 20
<210> 96
   <211> 22
   <212> RNA
   <213> Homo sapiens
<400> 96
   guggguacgg cccagugggg gg 22
<210> 97
   <211> 23
   <212> RNA
   <213> Homo sapiens
<400> 97
   agcagcauug uacagggcua uga 23
<210> 98
   <211> 22
   <212> RNA
   <213> Homo sapiens
<400> 98
   caaaacuggc aauuacuuuu gc 22
<210> 99
   <211> 19
   <212> RNA
   <213> Homo sapiens
<400> 99
   agcugucuga aaaugucuu 19
<210> 100
   <211> 22
   <212> RNA
   <213> Homo sapiens
<400> 100
   caggccauau ugugcugccu ca 22
<210> 101
   <211> 24
   <212> RNA
   <213> Homo sapiens
<400> 101
   agcagaagca gggagguucu ccca 24
<210> 102
   <211> 22
   <212> RNA
   <213> Homo sapiens
<400> 102
   aagcugccag uugaagaacu gu 22
<210> 103
   <211> 23
   <212> RNA
   <213> Homo sapiens
<400> 103
   ugugcaaauc uaugcaaaac uga 23
<210> 104
   <211> 22
   <212> RNA
   <213> Homo sapiens
<400> 104
   uauaccucag uuuuaucagg ug 22
<210> 105
   <211> 21
   <212> RNA
   <213> Homo sapiens
<400> 105
   guguugaaac aaucucuacu g 21
<210> 106
   <211> 22
   <212> RNA
   <213> Homo sapiens
<400> 106
   ccucuagaug gaagcacugu cu 22
<210> 107
   <211> 22
   <212> RNA
   <213> Homo sapiens
<400> 107
   acaaagugcu ucccuuuaga gu 22
<210> 108
   <211> 23
   <212> RNA
   <213> Homo sapiens
<400> 108
   uuacaguugu ucaaccaguu acu 23
<210> 109
   <211> 22
   <212> RNA
   <213> Homo sapiens
<400> 109
   cucggcgcgg ggcgcgggcu cc 22
<210> 110
   <211> 21
   <212> RNA
   <213> Homo sapiens
<400> 110
   auguaugugu gcaugugcau g 21
<210> 111
   <211> 21
   <212> RNA
   <213> Homo sapiens
<400> 111
   ucgaggagcu cacagucuag u 21
<210> 112
   <211> 22
   <212> RNA
   <213> Homo sapiens
<400> 112
   auauaauaca accugcuaag ug 22
<210> 113
   <211> 22
   <212> RNA
   <213> Homo sapiens
<400> 113
   ggauaucauc auauacugua ag 22
<210> 114
   <211> 23
   <212> RNA
   <213> Homo sapiens
<400> 114
   uuuggcacua gcacauuuuu gcu 23
<210> 115
   <211> 21
   <212> RNA
   <213> Homo sapiens
<400> 115
   cugaccuaug aauugacagc c 21
<210> 116
   <211> 22
   <212> RNA
   <213> Homo sapiens
<400> 116
   uagcuuauca gacugauguu ga 22
<210> 117
   <211> 22
   <212> RNA
   <213> Homo sapiens
<400> 117
   aauugcacgg uauccaucug ua 22
<210> 118
   <211> 21
   <212> RNA
   <213> Homo sapiens
<400> 118
   cuagacugaa gcuccuugag g 21
<210> 119
   <211> 21
   <212> RNA
   <213> Homo sapiens
<400> 119
   aucacauugc cagggauuac c 21
<210> 120
   <211> 21
   <212> RNA
   <213> Homo sapiens
<400> 120
   aggggugcua ucugugauug a 21
<210> 121
   <211> 22
   <212> RNA
   <213> Homo sapiens
<400> 121
   ugagaacuga auuccauggg uu 22
<210> 122
   <211> 23
   <212> RNA
   <213> Homo sapiens
<400> 122
   uaaagugcuu auagugcagg uag 23
<210> 123
   <211> 21
   <212> RNA
   <213> Homo sapiens
<400> 123
   uucaaguaau ucaggauagg u 21
<210> 124
   <211> 21
   <212> RNA
   <213> Homo sapiens
<400> 124
   caagucacua gugguuccgu u 21
<210> 125
   <211> 22
   <212> RNA
   <213> Homo sapiens
<400> 125
   aucaugaugg gcuccucggu gu 22
<210> 126
   <211> 21
   <212> RNA
   <213> Homo sapiens
<400> 126
   gaagugugcc gugguguguc u 21
<210> 127
   <211> 22
   <212> RNA
   <213> Homo sapiens
<400> 127
   ugagguagua guuugugcug uu 22
<210> 128
   <211> 21
   <212> RNA
   <213> Homo sapiens
<400> 128
   uucacagugg cuaaguuccg c 21
<210> 129
   <211> 21
   <212> RNA
   <213> Homo sapiens
<400> 129
   gccccugggc cuauccuaga a 21
<210> 130
   <211> 22
   <212> RNA
   <213> Homo sapiens
<400> 130
   uucaaguaau ccaggauagg cu 22
<210> 131
   <211> 21
   <212> RNA
   <213> Homo sapiens
<400> 131
   acuggacuug gagucagaag g 21
<210> 132
   <211> 23
   <212> RNA
   <213> Homo sapiens
<400> 132
   uauucauuua uccccagccu aca 23
<210> 133
   <211> 21
   <212> RNA
   <213> Homo sapiens
<400> 133
   uucacagugg cuaaguucug c 21
<210> 134
   <211> 22
   <212> RNA
   <213> Homo sapiens
<400> 134
   caacaaauca cagucugcca ua 22
<210> 135
   <211> 22
   <212> RNA
   <213> Homo sapiens
<400> 135
   uguaaacauc cucgacugga ag 22
<210> 136
   <211> 20
   <212> RNA
   <213> Homo sapiens
<400> 136
   uacaguauag augauguacu 20
<210> 137
   <211> 22
   <212> RNA
   <213> Homo sapiens
<400> 137
   accguggcuu ucgauuguua cu 22
<210> 138
   <211> 22
   <212> RNA
   <213> Homo sapiens
<400> 138
   ucguaccgug aguaauaaug cg 22
<210> 139
   <211> 22
   <212> RNA
   <213> Homo sapiens
<400> 139
   agagguagua gguugcauag uu 22
<210> 140
   <211> 23
   <212> RNA
   <213> Homo sapiens
<400> 140
   ugagugugug ugugugagug ugu 23
<210> 141
   <211> 22
   <212> RNA
   <213> Homo sapiens
<400> 141
   aacauucaac cugucgguga gu 22
<210> 142
   <211> 23
   <212> RNA
   <213> Homo sapiens
<400> 142
   uaaggugcau cuagugcaga uag 23
<210> 143
   <211> 23
   <212> RNA
   <213> Homo sapiens
<400> 143
   caaagugcug uucgugcagg uag 23
<210> 144
   <211> 22
   <212> RNA
   <213> Homo sapiens
<400> 144
   uguaaacauc cccgacugga ag 22
<210> 145
   <211> 22
   <212> RNA
   <213> Homo sapiens
<400> 145
   ucagugcauc acagaacuuu gu 22
<210> 146
   <211> 21
   <212> RNA
   <213> Homo sapiens
<400> 146
   agcuacaucu ggcuacuggg u 21
<210> 147
   <211> 23
   <212> RNA
   <213> Homo sapiens
<400> 147
   cugggaucuc cggggucuug guu 23
<210> 148
   <211> 22
   <212> RNA
   <213> Homo sapiens
<400> 148
   uauggcacug guagaauuca cu 22
<210> 149
   <211> 22
   <212> RNA
   <213> Homo sapiens
<400> 149
   ugagguagga gguuguauag uu 22
<210> 150
   <211> 22
   <212> RNA
   <213> Homo sapiens
<400> 150
   ugagguagua guuuguacag uu 22
<210> 151
   <211> 24
   <212> RNA
   <213> Homo sapiens
<400> 151
   aauccuugga accuaggugu gagu 24
<210> 152
   <211> 22
   <212> RNA
   <213> Homo sapiens
<400> 152
   cacccguaga accgaccuug cg 22
<210> 153
   <211> 22
   <212> RNA
   <213> Homo sapiens
<400> 153
   uacgucaucg uugucaucgu ca 22
<210> 154
   <211> 23
   <212> RNA
   <213> Homo sapiens
<400> 154
   caaagugcuc auagugcagg uag 23
<210> 155
   <211> 22
   <212> RNA
   <213> Homo sapiens
<400> 155
   ucacaaguca ggcucuuggg ac 22
<210> 156
   <211> 20
   <212> RNA
   <213> Homo sapiens
<400> 156
   acugccccag gugcugcugg 20
<210> 157
   <211> 22
   <212> RNA
   <213> Homo sapiens
<400> 157
   ugagguagua gauuguauag uu 22
<210> 158
   <211> 22
   <212> RNA
   <213> Homo sapiens
<400> 158
   aacgcacuuc ccuuuagagu gu 22
<210> 159
   <211> 22
   <212> RNA
   <213> Homo sapiens
<400> 159
   aagcccuuac cccaaaaagc au 22
<210> 160
   <211> 23
   <212> RNA
   <213> Homo sapiens
<400> 160
   ugaguaccgc caugucuguu ggg 23
<210> 161
   <211> 22
   <212> RNA
   <213> Homo sapiens
<400> 161
   ugagaacuga auuccauagg cu 22
<210> 162
   <211> 22
   <212> RNA
   <213> Homo sapiens
<400> 162
   aaacaaacau ggugcacuuc uu 22
<210> 163
   <211> 23
   <212> RNA
   <213> Homo sapiens
<400> 163
   caagucuuau uugagcaccu guu 23
<210> 164
   <211> 22
   <212> RNA
   <213> Homo sapiens
<400> 164
   acaggugagg uucuugggag cc 22
<210> 165
   <211> 23
   <212> RNA
   <213> Homo sapiens
<400> 165
   uaagugcuuc cauguuugag ugu 23
<210> 166
   <211> 21
   <212> RNA
   <213> Homo sapiens
<400> 166
   aauggcgcca cuaggguugu g 21
<210> 167
   <211> 22
   <212> RNA
   <213> Homo sapiens
<400> 167
   uguaaacauc cuacacucag cu 22
<210> 168
   <211> 21
   <212> RNA
   <213> Homo sapiens
<400> 168
   gaaagcgcuu cucuuuagag g 21
<210> 169
   <211> 23
   <212> RNA
   <213> Homo sapiens
<400> 169
   gaagugcuuc gauuuugggg ugu 23
<210> 170
   <211> 21
   <212> RNA
   <213> Homo sapiens
<400> 170
   cuauacaauc uacugucuuu c 21
<210> 171
   <211> 23
   <212> RNA
   <213> Homo sapiens
<400> 171
   uuaaugcuaa ucgugauagg ggu 23
<210> 172
   <211> 22
   <212> RNA
   <213> Homo sapiens
<400> 172
   uauugcacuc gucccggccu cc 22
<210> 173
   <211> 23
   <212> RNA
   <213> Homo sapiens
<400> 173
   cgcauccccu agggcauugg ugu 23
<210> 174
   <211> 22
   <212> RNA
   <213> Homo sapiens
<400> 174
   gggagccagg aaguauugau gu 22
<210> 175
   <211> 19
   <212> RNA
   <213> Homo sapiens
<400> 175
   aaaagcuggg uugagagga 19
<210> 176
   <211> 22
   <212> RNA
   <213> Homo sapiens
<400> 176
   ugagguagua gguuguaugg uu 22
<210> 177
   <211> 21
   <212> RNA
   <213> Homo sapiens
<400> 177
   ugagaugaag cacuguagcu c 21
<210> 178
   <211> 22
   <212> RNA
   <213> Homo sapiens
<400> 178
   aguucuucag uggcaagcuu ua 22
<210> 179
   <211> 21
   <212> RNA
   <213> Homo sapiens
<400> 179
   uccgguucuc agggcuccac c 21
<210> 180
   <211> 22
   <212> RNA
   <213> Homo sapiens
<400> 180
   aacccguaga uccgaucuug ug 22
<210> 181
   <211> 22
   <212> RNA
   <213> Homo sapiens
<400> 181
   cggguggauc acgaugcaau uu 22
<210> 182
   <211> 23
   <212> RNA
   <213> Homo sapiens
<400> 182
   uauggcuuuu cauuccuaug uga 23
<210> 183
   <211> 22
   <212> RNA
   <213> Homo sapiens
<400> 183
   uucccuuugu cauccuaugc cu 22
<210> 184
   <211> 23
   <212> RNA
   <213> Homo sapiens
<400> 184
   uagugcaaua uugcuuauag ggu 23
<210> 185
   <211> 22
   <212> RNA
   <213> Homo sapiens
<400> 185
   ucucccaacc cuuguaccag ug 22
<210> 186
   <211> 22
   <212> RNA
   <213> Homo sapiens
<400> 186
   agaauugugg cuggacaucu gu 22
<210> 187
   <211> 22
   <212> RNA
   <213> Homo sapiens
<400> 187
   cugguuucac augguggcuu ag 22
<210> 188
   <211> 22
   <212> RNA
   <213> Homo sapiens
<400> 188
   caugccuuga guguaggacc gu 22
<210> 189
   <211> 20
   <212> RNA
   <213> Homo sapiens
<400> 189
   guccgcucgg cgguggccca 20
<210> 190
   <211> 23
   <212> RNA
   <213> Homo sapiens
<400> 190
   cccaguguuc agacuaccug uuc 23
<210> 191
   <211> 21
   <212> RNA
   <213> Homo sapiens
<400> 191
   ucuaguaaga guggcagucg a 21
<210> 192
   <211> 21
   <212> RNA
   <213> Homo sapiens
<400> 192
   uuuccauagg ugaugaguca c 21
<210> 193
   <211> 22
   <212> RNA
   <213> Homo sapiens
<400> 193
   uggucuagga uuguuggagg ag 22
<210> 194
   <211> 22
   <212> RNA
   <213> Homo sapiens
<400> 194
   ucaccagccc uguguucccu ag 22
<210> 195
   <211> 23
   <212> RNA
   <213> Homo sapiens
<400> 195
   uauggcuuuu uauuccuaug uga 23
<210> 196
   <211> 22
   <212> RNA
   <213> Homo sapiens
<400> 196
   uagcagcaca uaaugguuug ug 22
<210> 197
   <211> 22
   <212> RNA
   <213> Homo sapiens
<400> 197
   cucuagaggg aagcgcuuuc ug 22
<210> 198
   <211> 20
   <212> RNA
   <213> Homo sapiens
<400> 198
   ugagcccugu ccucccgcag 20
<210> 199
   <211> 21
   <212> RNA
   <213> Homo sapiens
<400> 199
   ugagugccgg ugccugcccu g 21
<210> 200
   <211> 23
   <212> RNA
   <213> Homo sapiens
<400> 200
   ucucacacag aaaucgcacc cgu 23
<210> 201
   <211> 22
   <212> RNA
   <213> Homo sapiens
<400> 201
   cuauacaguc uacugucuuu cc 22
<210> 202
   <211> 22
   <212> RNA
   <213> Homo sapiens
<400> 202
   uaaugccccu aaaaauccuu au 22
<210> 203
   <211> 22
   <212> RNA
   <213> Homo sapiens
<400> 203
   ucucugggcc ugugucuuag gc 22
<210> 204
   <211> 22
   <212> RNA
   <213> Homo sapiens
<400> 204
   aaugcaccug ggcaaggauu ca 22
<210> 205
   <211> 22
   <212> RNA
   <213> Homo sapiens
<400> 205
   cugggagagg guuguuuacu cc 22
<210> 206
   <211> 23
   <212> RNA
   <213> Homo sapiens
<400> 206
   agcucggucu gaggccccuc agu 23
<210> 207
   <211> 22
   <212> RNA
   <213> Homo sapiens
<400> 207
   ucccugagac ccuaacuugu ga 22
<210> 208
   <211> 21
   <212> RNA
   <213> Homo sapiens
<400> 208
   uguucaugua gauguuuaag c 21
<210> 209
   <211> 21
   <212> RNA
   <213> Homo sapiens
<400> 209
   uugaaaggcu auuucuuggu c 21
<210> 210
   <211> 21
   <212> RNA
   <213> Homo sapiens
<400> 210
   caaagguauu ugugguuuuu g 21
<210> 211
   <211> 22
   <212> RNA
   <213> Homo sapiens
<400> 211
   aaggagcuca cagucuauug ag 22
<210> 212
   <211> 22
   <212> RNA
   <213> Homo sapiens
<400> 212
   aagugccucc uuuuagagug uu 22
<210> 213
   <211> 23
   <212> RNA
   <213> Homo sapiens
<400> 213
   ucccccaggu gugauucuga uuu 23
<210> 214
   <211> 23
   <212> RNA
   <213> Homo sapiens
<400> 214
   ugucugcccg caugccugcc ucu 23
<210> 215
   <211> 22
   <212> RNA
   <213> Homo sapiens
<400> 215
   aaugcacccg ggcaaggauu cu 22
<210> 216
   <211> 22
   <212> RNA
   <213> Homo sapiens
<400> 216
   aaagugcuuc cuuuuagagg gu 22
<210> 217
   <211> 22
   <212> RNA
   <213> Homo sapiens
<400> 217
   ucuauacaga cccuggcuuu uc 22
<210> 218
   <211> 20
   <212> RNA
   <213> Homo sapiens
<400> 218
   agagucuugu gaugucuugc 20
<210> 219
   <211> 22
   <212> RNA
   <213> Homo sapiens
<400> 219
   cacuagauug ugagcuccug ga 22
<210> 220
   <211> 22
   <212> RNA
   <213> Homo sapiens
<400> 220
   aggcggggcg ccgcgggacc gc 22
<210> 221
   <211> 23
   <212> RNA
   <213> Homo sapiens
<400> 221
   aucaacagac auuaauuggg cgc 23
<210> 222
   <211> 22
   <212> RNA
   <213> Homo sapiens
<400> 222
   uaguaccagu accuuguguu ca 22
<210> 223
   <211> 22
   <212> RNA
   <213> Homo sapiens
<400> 223
   uaacugguug aacaacugaa cc 22
<210> 224
   <211> 21
   <212> RNA
   <213> Homo sapiens
<400> 224
   acucuagcug ccaaaggcgc u 21
<210> 225
   <211> 20
   <212> RNA
   <213> Homo sapiens
<400> 225
   aaaagcuggg uugagagggu 20
<210> 226
   <211> 21
   <212> RNA
   <213> Homo sapiens
<400> 226
   aacaggugac ugguuagaca a 21
<210> 227
   <211> 21
   <212> RNA
   <213> Homo sapiens
<400> 227
   ccgucgccgc cacccgagcc g 21
<210> 228
   <211> 21
   <212> RNA
   <213> Homo sapiens
<400> 228
   uaauuuuaug uauaagcuag u 21
<210> 229
   <211> 22
   <212> RNA
   <213> Homo sapiens
<400> 229
   caaauucgua ucuaggggaa ua 22
<210> 230
   <211> 21
   <212> RNA
   <213> Homo sapiens
<400> 230
   ugcaggacca agaugagccc u 21
<210> 231
   <211> 22
   <212> RNA
   <213> Homo sapiens
<400> 231
   ccuauucuug auuacuuguu uc 22
<210> 232
   <211> 22
   <212> RNA
   <213> Homo sapiens
<400> 232
   aagugcuucc uuuuagaggg uu 22
<210> 233
   <211> 23
   <212> RNA
   <213> Homo sapiens
<400> 233
   uaauacugcc ggguaaugau gga 23
<210> 234
   <211> 23
   <212> RNA
   <213> Homo sapiens
<400> 234
   cucuugaggg aagcacuuuc ugu 23
<210> 235
   <211> 22
   <212> RNA
   <213> Homo sapiens
<400> 235
   ccucccacac ccaaggcuug ca 22
<210> 236
   <211> 21
   <212> RNA
   <213> Homo sapiens
<400> 236
   ucacagugaa ccggucucuu u 21
<210> 237
   <211> 21
   <212> RNA
   <213> Homo sapiens
<400> 237
   uaccacaggg uagaaccacg g 21
<210> 238
   <211> 22
   <212> RNA
   <213> Homo sapiens
<400> 238
   cgcaggggcc gggugcucac cg 22
<210> 239
   <211> 22
   <212> RNA
   <213> Homo sapiens
<400> 239
   ggagaaauua uccuuggugu gu 22
<210> 240
   <211> 22
   <212> RNA
   <213> Homo sapiens
<400> 240
   ugagguagua gguugugugg uu 22
<210> 241
   <211> 24
   <212> RNA
   <213> Homo sapiens
<400> 241
   acaaagugcu ucccuuuaga gugu 24
<210> 242
   <211> 22
   <212> RNA
   <213> Homo sapiens
<400> 242
   agacccuggu cugcacucua uc 22
<210> 243
   <211> 23
   <212> RNA
   <213> Homo sapiens
<400> 243
   cccaguguuu agacuaucug uuc 23
<210> 244
   <211> 22
   <212> RNA
   <213> Homo sapiens
<400> 244
   aaguucuguu auacacucag gc 22
<210> 245
   <211> 22
   <212> RNA
   <213> Homo sapiens
<400> 245
   ccucugaaau ucaguucuuc ag 22
<210> 246
   <211> 20
   <212> RNA
   <213> Homo sapiens
<400> 246
   gugucugggc ggacagcugc 20
<210> 247
   <211> 25
   <212> RNA
   <213> Homo sapiens
<400> 247
   agggguggug uugggacagc uccgu 25
<210> 248
   <211> 21
   <212> RNA
   <213> Homo sapiens
<400> 248
   gugggcgggg gcaggugugu g 21
<210> 249
   <211> 23
   <212> RNA
   <213> Homo sapiens
<400> 249
   cucucaccac ugcccuccca cag 23
<210> 250
   <211> 23
   <212> RNA
   <213> Homo sapiens
<400> 250
   cggcccgggc ugcugcuguu ccu 23
<210> 251
   <211> 22
   <212> RNA
   <213> Homo sapiens
<400> 251
   uauugcacau uacuaaguug ca 22
<210> 252
   <211> 20
   <212> RNA
   <213> Homo sapiens
<400> 252
   cuacaaaggg aagcccuuuc 20
<210> 253
   <211> 22
   <212> RNA
   <213> Homo sapiens
<400> 253
   aagacgggag gaaagaaggg ag 22
<210> 254
   <211> 22
   <212> RNA
   <213> Homo sapiens
<400> 254
   cagcagcaau ucauguuuug aa 22
<210> 255
   <211> 22
   <212> RNA
   <213> Homo sapiens
<400> 255
   uauagggauu ggagccgugg cg 22
<210> 256
   <211> 22
   <212> RNA
   <213> Homo sapiens
<400> 256
   uagguaguuu ccuguuguug gg 22
<210> 257
   <211> 22
   <212> RNA
   <213> Homo sapiens
<400> 257
   uuguacaugg uaggcuuuca uu 22
<210> 258
   <211> 22
   <212> RNA
   <213> Homo sapiens
<400> 258
   uuaugguuug ccugggacug ag 22
<210> 259
   <211> 22
   <212> RNA
   <213> Homo sapiens
<400> 259
   ugucuacuac uggagacacu gg 22
<210> 260
   <211> 22
   <212> RNA
   <213> Homo sapiens
<400> 260
   aagugcuguc auagcugagg uc 22
<210> 261
   <211> 23
   <212> RNA
   <213> Homo sapiens
<400> 261
   acuccauuug uuuugaugau gga 23
<210> 262
   <211> 22
   <212> RNA
   <213> Homo sapiens
<400> 262
   uagcagcaca ucaugguuua ca 22
<210> 263
   <211> 21
   <212> RNA
   <213> Homo sapiens
<400> 263
   cacauuacac ggucgaccuc u 21
<210> 264
   <211> 22
   <212> RNA
   <213> Homo sapiens
<400> 264
   ccuguucucc auuacuuggc uc 22
<210> 265
   <211> 21
   <212> RNA
   <213> Homo sapiens
<400> 265
   cacaagguau ugguauuacc u 21
<210> 266
   <211> 22
   <212> RNA
   <213> Homo sapiens
<400> 266
   auaagacgaa caaaagguuu gu 22
<210> 267
   <211> 22
   <212> RNA
   <213> Homo sapiens
<400> 267
   ugcccuuaaa ggugaaccca gu 22
<210> 268
   <211> 23
   <212> RNA
   <213> Homo sapiens
<400> 268
   uggugcggag agggcccaca gug 23
<210> 269
   <211> 19
   <212> RNA
   <213> Homo sapiens
<400> 269
   uggauuuuug gaucaggga 19
<210> 270
   <211> 22
   <212> RNA
   <213> Homo sapiens
<400> 270
   cacgcucaug cacacaccca ca 22
<210> 271
   <211> 22
   <212> RNA
   <213> Homo sapiens
<400> 271
   gaaaucaagc gugggugaga cc 22
<210> 272
   <211> 23
   <212> RNA
   <213> Homo sapiens
<400> 272
   aacauucauu gcugucggug ggu 23
<210> 273
   <211> 22
   <212> RNA
   <213> Homo sapiens
<400> 273
   ucggauccgu cugagcuugg cu 22
<210> 274
   <211> 22
   <212> RNA
   <213> Homo sapiens
<400> 274
   uccgagccug ggucucccuc uu 22
<210> 275
   <211> 22
   <212> RNA
   <213> Homo sapiens
<400> 275
   cagugcaaug uuaaaagggc au 22
<210> 276
   <211> 22
   <212> RNA
   <213> Homo sapiens
<400> 276
   cgaaucauua uuugcugcuc ua 22
<210> 277
   <211> 21
   <212> RNA
   <213> Homo sapiens
<400> 277
   cuuuuugcgg ucugggcuug c 21
<210> 278
   <211> 23
   <212> RNA
   <213> Homo sapiens
<400> 278
   uggaagacua gugauuuugu ugu 23
<210> 279
   <211> 21
   <212> RNA
   <213> Homo sapiens
<400> 279
   uaguagaccg uauagcguac g 21
<210> 280
   <211> 21
   <212> RNA
   <213> Homo sapiens
<400> 280
   caaaacguga ggcgcugcua u 21
<210> 281
   <211> 22
   <212> RNA
   <213> Homo sapiens
<400> 281
   gcuauuucac gacaccaggg uu 22
<210> 282
   <211> 22
   <212> RNA
   <213> Homo sapiens
<400> 282
   uucauucggc uguccagaug ua 22
<210> 283
   <211> 22
   <212> RNA
   <213> Homo sapiens
<400> 283
   cucuagaggg aagcacuuuc ug 22